(19) Europäisches Patentamt
European Patent Office
Office européen des brevets

(11) EP 1 242 389 B1

(12) FASCICULE DE BREVET EUROPEEN

(45) Date de publication et mention de la délivrance du brevet: **21.04.2004 Bulletin 2004/17**

(21) Numéro de dépôt: **00988916.3**

(22) Date de dépôt: **14.12.2000**

(51) Int Cl.[7]: **C07D 249/14**, A61K 31/4196, A61P 25/00

(86) Numéro de dépôt international: **PCT/FR2000/003536**

(87) Numéro de publication internationale: **WO 2001/044207 (21.06.2001 Gazette 2001/25)**

(54) **NOUVEAUX DERIVES AMINO SUBSTITUES RAMIFIES DU 3-AMINO-1-PHENYL-1H[1,2,4]TRIAZOLE, PROCEDES POUR LEUR PREPARATION ET COMPOSITIONS PHARMACEUTIQUES LES CONTENANT**

VERZWEIGTE SUBSTITUIERTE AMINODERIVATE VOM 3-AMINO-1-PHENYL-1H[1,2,4]TRIAZOL, VERFAHREN ZU DEREN HERSTELLUNG UND SIE ENTHALTENDE PHARMAZEUTISCHE ZUSAMMENSETZUNGEN

NOVEL BRANCHED SUBSTITUTED AMINO DERIVATIVES OF 3-AMINO-1-PHENYL-1H[1,2,4]TRIAZOL, METHODS FOR PRODUCING THEM AND PHARMACEUTICAL COMPOSITIONS CONTAINING THEM

(84) Etats contractants désignés:
**AT BE CH CY DE DK ES FI FR GB GR IE IT LI LU MC NL PT SE TR**
Etats d’extension désignés:
**AL LT LV MK RO SI**

(30) Priorité: **17.12.1999 FR 9915935**

(43) Date de publication de la demande: **25.09.2002 Bulletin 2002/39**

(73) Titulaire: **SANOFI-SYNTHELABO
75013 Paris (FR)**

(72) Inventeurs:
- **GESLIN, Michel
F-31270 Villeneuve Tolosane (FR)**
- **GULLY, Danielle
Saubens, 31600 Muret (FR)**
- **MAFFRAND, Jean-Pierre
F-31120 Portet sur Garonne (FR)**
- **ROGER, Pierre
F-78423 Montigny le Bretonneux (FR)**

(74) Mandataire: **Thouret-Lemaitre, Elisabeth et al
Sanofi-Synthélabo,
Département Brevets,
174, avenue de France
75013 Paris (FR)**

(56) Documents cités:
**WO-A-96/39400 WO-A-98/51686**

- **TRINKA, PETER ET AL: "Triazoles. XXXVI. The arylation of 5-amino-3-(methylthio)-1H-1,2,4-triazole with activated aryl chlorides" J. HETEROCYCL. CHEM. (1995), 32(4), 1359-71, XP002146823**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).

Printed by Jouve, 75001 PARIS (FR)

EP 1 242 389 B1

## Description

La présente invention a pour objet de nouveaux dérivés amino substitués ramifiés du 3-amino-1-phényl-1*H* [1,2,4]triazole, des procédés pour leur préparation et les compositions pharmaceutiques les contenant.

Ces nouveaux dérivés du triazole sont pourvus d'activité antagoniste du CRF (corticotropin releasing factor) et peuvent donc constituer des principes actifs de compositions pharmaceutiques.

Le facteur de libération de l'hormone corticotrope (CRF) est un peptide dont la séquence de 41 acides aminés a été caractérisée par Vale W. *et al*. en 1981 (Science, 1981, *213*, 1394-1397). Le CRF est le principal facteur endogène impliqué dans la régulation de l'axe hypothalamo-hypophyso-surrénalien (libération de l'hormone adrénocorticotrope : ACTH) et ses pathologies, ainsi que dans les syndromes dépressifs qui découlent de son dysfonctionnement. Le CRF provoque également la sécrétion de β-endorphine, de β-lipotropine et de corticostérone. Le CRF est donc le régulateur physiologique de la sécrétion de l'hormone adrénocorticotrope (ACTH) et du cortisol par l'effet de l'ACTH au niveau surrénalien et plus généralement des peptides dérivés de la propiomélanocortine (POMC). Outre sa localisation hypothalamique, le CRF est largement distribué dans le système nerveux central, mais également dans des tissus extra-neuronaux tels que les glandes surrénales et les testicules. La présence de CRF a été également mise en évidence au cours de processus inflammatoires.

De nombreuses expérimentations animales ont montré que l'administration centrale de CRF provoque des effets anxiogènes variés tels que la modification du comportement en général : par exemple néophobie, réduction de la réceptivité sexuelle, diminution de la consommation alimentaire et du sommeil lent chez le rat. L'injection intracérébroventriculaire de CRF augmente également l'excitation des neurones noradrénergiques du locus coeruleus qui est souvent associée chez l'animal, à un état d'anxiété. Chez le rat, l'administration centrale ou périphérique du CRF ou des peptides apparentés (par exemple urocortine, sauvagine) induit outre des effets centraux tels que l'augmentation de l'éveil et de la réactivité émotionnelle face à l'environnement, des modifications de la vidange gastrique, de la sécrétion acide, du transit intestinal et de l'excrétion fécale ainsi que des effets tensionnels. Le CRF est impliqué également dans la régulation complexe des réponses inflammatoires, d'une part avec un rôle pro-inflammatoire dans certains modèles animaux (dégranulation des mastocytes induisant le relarguage de molécules inflammatoires telles que l'histamine, les prostaglandines, ... ) et d'autre part en tant qu'inhibiteur des effets induits par l'augmentation de la perméabilité vasculaire consécutive de l'inflammation.

L'utilisation d'un antagoniste peptidique, l'alpha-hélical CRF(9-41) (αH-CRF) ou d'anticorps spécifiques (Rivier J. *et al*., Science, 1984, 224, 889-891) a permis de confirmer le rôle de ce peptide dans l'ensemble de ces effets. Ces expériences ont également confirmé le rôle important du CRF chez l'homme dans l'intégration des réponses complexes observées lors d'un stress physiologique, psychologique ou immunologique tant sur le plan neuroendocrinien, viscéral que comportemental (Morley J.E. *et al*, Endocrine Review, 1987, *8*, 3, 256-287 ; Smith M.A. *et al*., Horm. Res., 1989, 31, 66-71). En outre, des données cliniques militent en faveur de l'implication effective du CRF dans les nombreux désordres découlant d'un état de stress (Gulley L.R. *et al*., J. Clin. Psychiatry, 1993, 54, 1, (suppl.), 16-19) par exemple :

- l'existence du test au CRF (administration i.v.) chez l'homme a permis de démontrer la modification de la réponse en ACTH chez les patients dépressifs (Breier A. *et al*., Am. J. Psychiatry, 1987, *144*, 1419-1425).
- la découverte d'une hypersécrétion de CRF endogène dans certaines pathologies, par exemple d'un taux de CRF élevé dans le liquide céphalorachidien chez les patients non médiqués, déprimés ou atteints de démence type maladie d'Alzheimer (Nemeroff C.B. *et al*., Science, 1984, 226, 4680, 1342-1343 ; Regul. Pept., 1989, 25, 123-130), ou d'une densité de récepteurs au CRF diminuée dans le cortex de victimes de suicide (Nemeroff C.B. *et al.,* Arch. Gen. Psychiatry, 1988, *45*, 577-579).
- le dysfonctionnement des neurones CRF-dépendants est même suggéré dans les pathologies sévères que sont les maladies d'Alzheimer, de Parkinson, la chorée de Huntington et la sclérose latérale amyotrophique (De Souza E.B.,Hospital Practice, 1988, *23*, 59).

L'administration centrale de CRF dans de nombreuses espèces animales, produit des effets comportementaux semblables à ceux obtenus chez l'homme dans les situations de stress. Lorsqu'ils sont répétés dans le temps, ces effets peuvent entraîner des pathologies diverses telles que : fatigue, hypertension, troubles cardiaques et tensionnels, modification de la vidange gastrique, de l'excrétion fécale (colite, colon irritable), modification de la sécrétion acide, hyperglycémie, croissance retardée, anorexie, néophobie, migraines, troubles de la reproduction, immunosuppression (processus inflammatoires, infections multiples et cancers) et désordres neuropsychiatriques variés (dépression, anorexie ou boulimie nerveuse et anxiété).

L'injection par voie intracérébroventriculaire de l'antagoniste peptidique de référence, l'αH-CRF (9-41) prévient les effets obtenus soit par l'administration de CRF exogène, soit par l'utilisation d'agents stressants (éther, contrainte, bruit, choc électrique, sevrage éthanolique, chirurgie) capables par eux-mêmes d'induire une augmentation du

taux de CRF endogène. Ces résultats sont confirmés par l'étude de nombreuses molécules peptidiques antagonistes structuralement apparentées au CRF et qui possèdent une durée d'action prolongée par rapport à l'αH-CRF (9-41) (Rivier J. *et al.*, J. Med. Chem., 1993, *36*, 2851-2859 ; Menzaghi F. *et al.*, J. Pharmacol. Exp. Ther., 1994, *269*, 2, 564-572 ; Hernandez J.F. *et al.*, J. Med. Chem., 1993, *36*, 2860-2867).

De tels composés peptidiques antagonistes du CRF sont décrits par exemple dans les brevets US 5,109,111, US 5,132,111, US 5,245,009 et dans les demandes de brevet WO 92/22576 et WO 96/19499.

En outre, des études préliminaires ont montré que des antidépresseurs tricycliques pouvaient moduler le taux de CRF ainsi que le nombre de récepteurs au CRF dans le cerveau (Grigoriadis D.E. *et al.,* Neuropsychopharmacoloy, 1989, *2*, 53-60). De même des anxiolytiques benzodiazépiniques sont capables d'inhiber l'effet du CRF (Britton K.T. *et al.*, Psychopharmacology, 1988, *94*, 306), sans que le mécanisme d'action de ces substances soit totalement élucidé. Ces résultats confortent si nécessaire le besoin grandissant de molécules antagonistes non-peptidiques des récepteurs du CRF.

Il est important de signaler également, trois conséquences possibles des états de stress chronique que sont l'immunodépression, les troubles de la fertilité, ainsi que l'installation du diabète.

Le CRF exerce de tels effets en interagissant avec des récepteurs membranaires spécifiques qui ont été caractérisés dans l'hypophyse et le cerveau de nombreuses espèces (souris, rat et homme) ainsi que dans le coeur, le muscle squelettique (rat, souris) et dans le myomètre et le placenta au cours de la grossesse.

Les composés 3-amino-1-phényl-1*H*[1,2,4]triazoles sont peu représentés. On peut citer surtout des composés portant en position 5 un phényle comme décrit par Ebenreth A. *et al* (Pharmazie, 1992, *47*(7), 556-7) et par Bozo E., Szilagil G. et Janaky J. (Arch. Pharm., 1989, *322*(10), 583-7, brevets n° HU44522, HU195791, 1986) qui revendiquent des propriétés anti-inflammatoires-antirhumatismales. Dans deux brevets japonais (JP 02091061 et JP 2729810, 1988), Inamori *et al* revendiquent la préparation de 3-amino-1-phényl-1*H*[1,2,4]triazoles comme insecticides.

Dans la demande de brevet de Neurocrine publiée sous le numéro WO 96/39400, des composés 3-amino-5-phényl-1*H*[1,2,4]triazoles sont décrits comme antagonistes des récepteurs du CRF.

Il a maintenant été trouvé selon la présente invention que certains dérivés du 3-amino-1-phényl-1*H*[1,2,4] triazole, objet de la présente invention, possèdent une excellente affinité *vis à vis* des récepteurs du CRF. De plus, compte tenu de leur structure, ces molécules possèdent une bonne dispersibilité et/ou solubilité dans des solvants ou solutions couramment utilisés en thérapeutique qui leur confère une activité pharmacologique et permettent aussi la préparation aisée de formes galéniques orales et parentérales.

La présente invention a pour objet, les composés sous forme racémique ou énantiomère de formule :

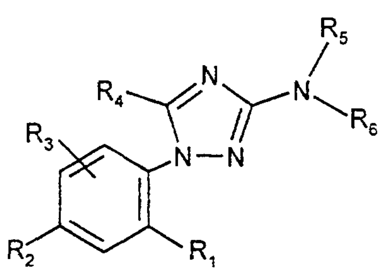

(I)

dans laquelle :

- $R_1$ et $R_2$ représentent, chacun indépendamment l'un de l'autre, un atome d'halogène ; un $(C_1\text{-}C_5)$alkyle ; un $(C_1\text{-}C_5)$alcoxy ; un groupe nitro, trifluorométhyle ou cyano ; un groupe amino $NR_aR_b$ dans lequel $R_a$ et $R_b$ représentent chacun indépendamment l'un de l'autre un hydrogène, un $(C_1\text{-}C_3)$alkyle , un $CO(C_1\text{-}C_3)$alkyle, ou bien dans lequel $R_a$ et $R_b$ constituent avec l'atome d'azote auquel ils sont liés un hétérocycle de 5 à 7 chaînons ; un groupe S-R dans lequel R représente un atome d'hydrogène ou un $(C_1\text{-}C_5)$alkyle, l'atome de soufre pouvant être monooxydé ou dioxydé ;
- $R_3$ représente l'hydrogène ou est tel que défini ci-dessus pour $R_1$ ;
- $R_4$ représente l'hydrogène ; un halogène ; un $(C_1\text{-}C_5)$alkyle ; un $(C_3\text{-}C_5)$cycloalkyle ; un $(C_3\text{-}C_5)$cycloalkyl$(C_1\text{-}C_2)$alkyle ; un groupe $R_c$-X-$(C_1\text{-}C_2)$alkyle dans lequel $R_c$ représente l'hydrogène ou un $(C_1\text{-}C_3)$alkyle et X représente O, S, SO, $SO_2$ ;
- $R_5$ représente un $(C_1\text{-}C_5)$alkyle, un alcynyle de 3 à 5 atomes de carbone, un alcényle de 3 à 5 atomes de carbone ; un $(C_3\text{-}C_5)$cycloalkyl$(C_1\text{-}C_3)$alkyle ; un $(C_1\text{-}C_3)$alkyl-X-$(C_0\text{-}C_3)$alkyle dans lequel X représente O, S, SO, $SO_2$ ;
- $R_6$ représente un groupe phényle substitué par un ou plusieurs radicaux Z dont l'un au moins est en position 2 et Z représente un halogène, un groupe nitro, trifluorométhyle ou cyano ; un $(C_1\text{-}C_5)$alkyle ; un $(C_1\text{-}C_5)$alkyl-X- ou

$(C_1-C_3)$alkyl-X-$(C_1-C_2)$alkyle dans lesquels X représente O, S, SO ou $SO_2$ ; un hydroxy$(C_1-C_3)$alkyle ; un $COR_d$ ou $COOR_d$ où $R_d$ représente un $(C_1-C_3)$alkyle ou un $(C_3-C_5)$cycloalkyle ;

- ou bien $R_6$ représente $-CHR_7R_8$ dans lequel
- $R_7$ représente un $(C_3-C_5)$cycloalkyle ; un groupe phényle pouvant être substitué en position 3, 4 et 5 par un ou plusieurs radicaux Z', avec Z' représentant un halogène, un groupe nitro, trifluorométhyle ou cyano ; un $(C_1-C_5)$ alkyle ; un $(C_1-C_5)$alkyl-X- ou $(C_1-C_3)$alkyl-X-$(C_1-C_2)$alkyle où X représente O, S, SO ou $SO_2$ ; un hydroxy$(C_1-C_3)$ alkyle ; un $COR_d$ ou $COOR_d$ dans lesquels $R_d$ est tel que défini ci-dessus ; un méthylènedioxy, un éthylènedioxy ; ou bien un groupe pyridyle éventuellement substitué par un groupe amino $NR_aR_b$ tel que défini précédemment ou par un radical Z' tel que défini ci-dessus ;
- $R_8$ représente un $(C_1-C_6)$alkyle ; $(C_3-C_5)$cycloalkyle ; un $(C_3-C_5)$cycloalkyl$(C_1-C_3)$alkyle ; $(C_1-C_3)$alkyl-X-$(C_1-C_3)$ alkyle où X représente O, S, SO ou $SO_2$ ; un $(C_3-C_5)$cycloalkyl$(C_1-C_2)$alkyl-X-$(C_1-C_3)$alkyle où X représente O, S, SO, $SO_2$ ;

ainsi que leurs sels d'addition pharmaceutiquement acceptables, leurs hydrates et/ou solvats.

Dans la présente description, on entend par halogène un atome de fluor, chlore, brome ou iode. Les groupes alkyles ou les groupes alcoxy sont linéaires ou ramifiés.

Selon un autre de ses aspects l'invention concerne les composés de formule (I) sous forme racémique ou énantiomère dans lesquels :

- $R_1$ et $R_2$ représentent, chacun indépendamment l'un de l'autre, un atome d'halogène ; un $(C_1-C_5)$alkyle ; un $(C_1-C_5)$ alcoxy ; un trifluorométhyle ou un groupe S-R dans lequel R représente un $(C_1-C_5)$alkyle ;
- $R_3$ représente l'hydrogène ou un $(C_1-C_5)$alkyle ;
- $R_4$ représente un $(C_1-C_5)$alkyle ; un $(C_3-C_5)$cycloalkyl ou un groupe $R_a$-X-$(C_1-C_2)$alkyle dans lequel $R_a$ représente un $(C_1-C_3)$alkyle et X représente O ;
- $R_5$ représente un $(C_1-C_5)$alkyle ou un alcynyle de 3 à 5 atomes de carbone ;
- $R_6$ représente $-CHR_7R_8$ dans lequel
- $R_7$ représente un groupe phényle pouvant être substitué en position 3, 4 et 5 par un ou plusieurs radicaux Z', avec Z' représentant un halogène ; un $(C_1-C_5)$alkyle ; un $(C_1-C_5)$alkyl-X- ou $(C_1-C_3)$alkyl-X-$(C_1-C_2)$alkyle où X représente O ; ou un groupe méthylènedioxy ;
- $R_8$ représente un $(C_1-C_6)$alkyle ; un $(C_3-C_5)$cycloalkyl$(C_1-C_3)$alkyle ; $(C_1-C_3)$alkyl-X-$(C_1-C_3)$alkyle où X représente O ;

ainsi que leurs sels d'addition pharmaceutiquement acceptables, leurs hydrates et/ou solvats.

Selon un autre de ses aspects l'invention concerne les composés de formule (I) sous forme racémique ou énantiomère dans lesquels $R_5$ représente un groupe propyle ou propargyle ainsi que leurs sels d'addition pharmaceutiquement acceptables, leurs hydrates et/ou solvats.

L'invention concerne plus particulièrement les composés ci-dessus sous forme d'énantiomère.

Selon un autre de ses aspects l'invention concerne les composés suivants :

- ■ Chlorhydrate de 5-cyclopropyl-*N*-[2-cyclopropyl-1-(4-fluorophényl)éthyl]-1-(2,4-dichlorophényl)-*N*-propyl-1*H*-1,2,4-triazol-3-amine (exemple 7)
- ■ Chlorhydrate de *N*-[2-cyclopropyl-1-(4-fluorophényl)éthyl]-1-(2,4-dichlorophényl)-5-(méthoxyméthyl)-*N*-propyl-1*H*-1,2,4-triazol-3-amine (exemple 9)
- ■ Chlorhydrate de *N*-[2-cyclopropyl-1-(4-fluorophényl)éthyl]-1-[2,6-dichloro-4-(trifluorométhyl)phényl]-5-méthyl-*N*-propyl-1*H*-1,2,4-triazol-3-amine (exemple 10)
- ■ Chlorhydrate de 1-(2-chloro-4-méthoxy-5-méthylphényl)-*N*-[2-cyclopropyl-1-(4-fluorophényl)éthyl]-5-méthyl-*N*-(2-propynyl)-1*H*-1,2,4-triazol-3-amine (exemple 13)
- ■ Chlorhydrate de 1-(2-chloro-4-méthoxy-5-méthylphényl)-5-cyclopropyl-*N*-[2-cyclopropyl-1-(4-fluorophényl)éthyl]-*N*-propyl-1*H*-1,2,4-triazol-3-amine (exemple 14)
- ■ Chlorhydrate de 1-(2-chloro-4-méthoxy-5-méthylphényl)-5-cyclopropyl-*N*-[2-cyclopropyl-1-(4-fluorophényl)éthyl]-*N*-(2-propynyl)-1*H*-1,2,4-triazol-3-amine (exemple 15)
- ■ Bromhydrate de 5-cyclopropyl-*N*-[2-cyclopropyl-1-(4-fluorophényl)éthyl]-1-[2,6-dichloro-4-(trifluorométhyl)phényl]-*N*-propyl-1*H*-1,2,4-triazol-3-amine (exemple 18)
- ■ Chlorhydrate de 5-cyclopropyl-*N*-(2-cyclopropyl-1-phényléthyl)-1-(2,4-dichlorophényl)-*N*-propyl-1*H*-1,2,4-triazol-3-amine (exemple 19)
- ■ Chlorhydrate de 1-(2-chloro-4-méthoxy-5-méthylphényl)-5-cyclopropyl-*N*-(2-cyclopropyl-1-phényléthyl)-*N*-propyl-1*H*-1,2,4-triazol-3-amine (exemple 20)
- ■ Chlorhydrate de 1-(2-chloro-4-méthoxy-5-méthylphényl)-5-cyclopropyl-*N*-[(1*S*)-2-cyclopropyl-1-(3-fluoro-4-méthylphényl)éthyl]-*N*-propyl-1*H*-1,2,4-triazol-3-amine (exemple 22)

- Chlorhydrate de 1-(2-chloro-4-méthoxy-5-méthylphényl)-5-cyclopropyl-*N*-(2-cyclopropyl-1-phényléthyl)-*N*-(2-propynyl)-1*H*-1,2,4-triazol-3-amine (exemple 23)
- Chlorhydrate de 1-(2-chloro-4-méthoxy-5-méthylphényl)-5-cyclopropyl-*N*-[(1*R*)-1-(4-fluorophényl)-2-méthoxyéthyl]-*N*-propyl-1*H*-1,2,4-triazol-3-amine (exemple 24)
- Bromhydrate de 1-(2-chloro-4-méthoxy-5-méthylphényl)-5-cyclopropyl-*N*-[(1*S*)-2-cyclopropyl-1-(3-fluoro-4-méthylphényl)éthyl]-*N*-(2-propynyl)-1*H*-1,2,4-triazol-3-amine (exemple 25)
- Chlorhydrate de 5-cyclopropyl-*N*-[2-cyclopropyl-1-(3-fluoro-4-méthylphényl)éthyl]-1-(2,4-dichlorophényl)-*N*-propyl-1*H*-1,2,4-triazol-3-amine (exemple 26)
- Chlorhydrate de 5-cyclopropyl-1-(2,4-dichlorophényl)-*N*-[(1*R*)-1-(4-fluorophényl)-2-méthoxyéthyl]-*N*-propyl-1*H*-1,2,4-triazol-3-amine (exemple 27)
- Chlorhydrate de 5-cyclopropyl-*N*-[(1*S*)-2-cyclopropyl-1-(3-fluoro-4-méthylphényl) éthyl]-1-(2,4-diméthylphényl)-*N*-propyl-1*H*-1,2,4-triazol-3-amine (exemple 28)
- Chlorhydrate de 1-(2,4-dichlorophényl)-5-éthyl-*N*-[(1*S*)-1-phénylbutyl]-*N*-propyl-1*H*-1,2,4-triazol-3-amine (exemple 29)
- Chlorhydrate de 1-(2-chloro-4-méthoxy-5-méthylphényl)-5-méthyl-N-[(1*S*)-1-phénylbutyl]-*N*-propyl-1*H*-1,2,4-triazol-3-amine (exemple 30)
- Chlorhydrate de 1-(2-chloro-4-méthoxy-5-méthylphényl)-*N*-[(1*S*)-2-cyclopropyl-1-(3-fluoro-4-méthylphényl)éthyl]-5-méthyl-*N*-propyl-1*H*-1,2,4-triazol-3-amine (exemple 31)
- Chlorhydrate de *N*-[(1*S*)-2-cyclopropyl-1-(4-fluorophényl)éthyl]-1-(2,4-dichlorophényl)-5-méthyl-*N*-propyl-1*H*-1,2,4-triazol-3-amine (exemple 32)
- Chlorhydrate de 1-(2-chloro-4-méthoxy-5-méthylphényl)-*N*-[(1*S*)-2-cyclopropyl-1-(3-fluoro-4-méthylphényl)éthyl]-5-éthyl-*N*-propyl-1*H*-1,2,4-triazol-3-amine (exemple 33)
- Chlorhydrate de *N*-[(1*S*)-2-cyclopropyl-1-(4-fluorophényl)éthyl]-1-(2,4-dichlorophényl)-5-éthyl-*N*-propyl-1*H*-1,2,4-triazol-3-amine (exemple 34)
- Chlorhydrate de 1-(2-chloro-4-méthoxy-5-méthylphényl)-*N*-[(1*S*)-2-cyclopropyl-1-(3-fluoro-4-méthylphényl)éthyl]-5-méthyl-*N*-(2-propynyl)-1*H*-1,2,4-triazol-3-amine (exemple 35)
- Chlorhydrate de 5-cyclopropyl-1-(2,4-dichlorophényl)-*N*-[(1*S*)-1-phénylbutyl]-*N*-propyl-1*H*-1,2,4-triazol-3-amine (exemple 36)
- Chlorhydrate de 1-(2-chloro-4-méthoxy-5-méthylphényl)-*N*-[(1*S*)-2-cyclopropyl-1-(4-fluorophényl)éthyl]-5-éthyl-*N*-propyl-1*H*-1,2,4-triazol-3-amine (exemple 37)
- Chlorhydrate de *N*-[(1*S*)-2-cyclopropyl-1-(3-fluoro-4-méthylphényl)éthyl]-1-(2,4-dichlorophényl)-5-méthyl-*N*-propyl-1*H*-1,2,4-triazol-3-amine (exemple 38)
- Chlorhydrate de 1-(2-chloro-4-méthoxy-5-méthylphényl)-*N*-[(1*S*)-2-cyclopropyl-1-(4-méthylphényl)éthyl]-5-méthyl-*N*-propyl-1*H*-1,2,4-triazol-3-amine (exemple 39)
- Chlorhydrate de *N*-[(1*S*)-2-cyclopropyl-1-(4-méthylphényl)éthyl]-1-(2,4-dichlorophényl)-5-méthyl-*N*-propyl-1*H*-1,2,4-triazol-3-amine (exemple 40)
- Chlorhydrate de 1-(2-chloro-4-méthoxy-5-méthylphényl)-5-cyclopropyl-*N*-[(1*S*)-1-phénylbutyl]-*N*-propyl-1*H*-1,2,4-triazol-3-amine (exemple 41)
- Chlorhydrate de 1-(2-chloro-4-méthoxy-5-méthylphényl)-*N*-[(1*S*)-2-cyclopropyl-1-(4-fluorophényl)éthyl]-5-méthyl-*N*-propyl-1*H*-1,2,4-triazol-3-amine (exemple 42)
- Chlorhydrate de 1-[2-chloro-4-(trifluorométhyl)phényl]-*N*-[(1*S*)-2-cyclopropyl-1-(3-fluoro-4-méthylphényl)éthyl]-5-méthyl-*N*-propyl-1*H*-1,2,4-triazol-3-amine (exemple 43)
- Chlorhydrate de *N*-[(1*S*)-1-(1,3-benzodioxol-5-yl)-2-cyclopropyléthyl]-1-(2-chloro-4-méthoxy-5-méthylphényl)-5-méthyl-*N*-propyl-1*H*-1,2,4-triazol-3-amine (exemple 44)
- Chlorhydrate de *N*-[(1*S*)-1-(1,3-benzodioxol-5-yl)-2-cyclopropyléthyl]-1-(2,4-dichlorophényl)-5-méthyl-*N*-propyl-1*H*-1,2,4-triazol-3-amine (exemple 45)
- Chlorhydrate de 1-(2-chloro-4-méthoxy-5-méthylphényl)-*N*-{(1*S*)-2-cyclopropyl-1-[(4-méthoxyméthyl)phényl}éthyl}-5-méthyl-*N*-propyl-1*H*-1,2,4-triazol-3-amine (exemple 46)
- Chlorhydrate de 1-(2-chloro-4-méthoxyphényl)-*N*-[(1*S*)-2-cyclopropyl-1-(3-fluoro-4-méthylphényl)éthyl]-5-méthyl-*N*-propyl-1*H*-1,2,4-triazol-3-amine (exemple 47)
- Chlorhydrate de 1-(2-chloro-4-méthoxy-5-méthylphényl)-*N*-[(1*S*)-2-cyclopropyl-1-phényl)éthyl]-5-méthyl-*N*-propyl-1*H*-1,2,4-triazol-3-amine (exemple 48)
- Chlorhydrate de *N*-[(1*S*)-2-cyclopropyl-1-phényl)éthyl]-1-(2,4-dichlorophényl)-5-méthyl-*N*-propyl-1*H*-1,2,4-triazol-3-amine (exemple 49)
- Chlorhydrate de 1-(2-chloro-4-méthoxy-5-méthylphényl)-5-cyclopropyl-*N*-[(1*S*)-1-(4-méthylphényl)butyl]-*N*-propyl-1*H*-1,2,4-triazol-3-amine (exemple 50)
- Chlorhydrate de 5-cyclopropyl-1-(2,4-dichlorophényl)-*N*-[(1*S*)-1-(4-méthylphényl) butyl]-*N*-propyl-1*H*-1,2,4-triazol-3-amine (exemple 51)

- Chlorhydrate de 1-[2-chloro-4-(méthylsulfanyl)phényl]-*N*-[2-cyclopropyl-1-(3-fluoro-4-méthylphényl)éthyl]-5-méthyl-*N*-propyl-1*H*-1,2,4-triazol-3-amine (exemple 52)
- Chlorhydrate de 1-(2-chloro-4-méthoxyphényl)-*N*-[(1*S*)-2-cyclopropyl-1-(4-fluorophényl)éthyl]-5-méthyl-*N*-propyl-1*H*-1,2,4-triazol-3-amine (exemple 53)

ainsi,que les bases correspondantes, les autres sels d'addition pharmaceutiquement acceptables, leurs solvats et/ou hydrates.

Les composés de l'invention sous forme libre présentent généralement des propriétés de base faible.

Les sels des composés de formule (I) avec des acides pharmaceutiquement acceptables sont les sels préférés, mais ceux qui peuvent permettre d'isoler les composés de formule (I) notamment de les purifier ou d'obtenir des énantiomères ou diastéréoisomères purs, sont aussi objet de l'invention.

Parmi les acides pharmaceutiquement acceptables pour la préparation des sels d'addition aux composés de formule (I), on peut citer les acides chlorhydrique, bromhydrique, phosphorique, fumarique, citrique, oxalique, sulfurique, ascorbique, tartrique, maléique, mandélique, méthanesulfonique, lactobionique, gluconique, glucarique, succinique, sulfonique, hydroxypropane sulfonique.

Les composés de l'invention peuvent être préparés selon deux voies de synthèse A et B.

Le SCHEMA 1 représente la voie de synthèse A.

**SCHEMA 1 : Voie A**

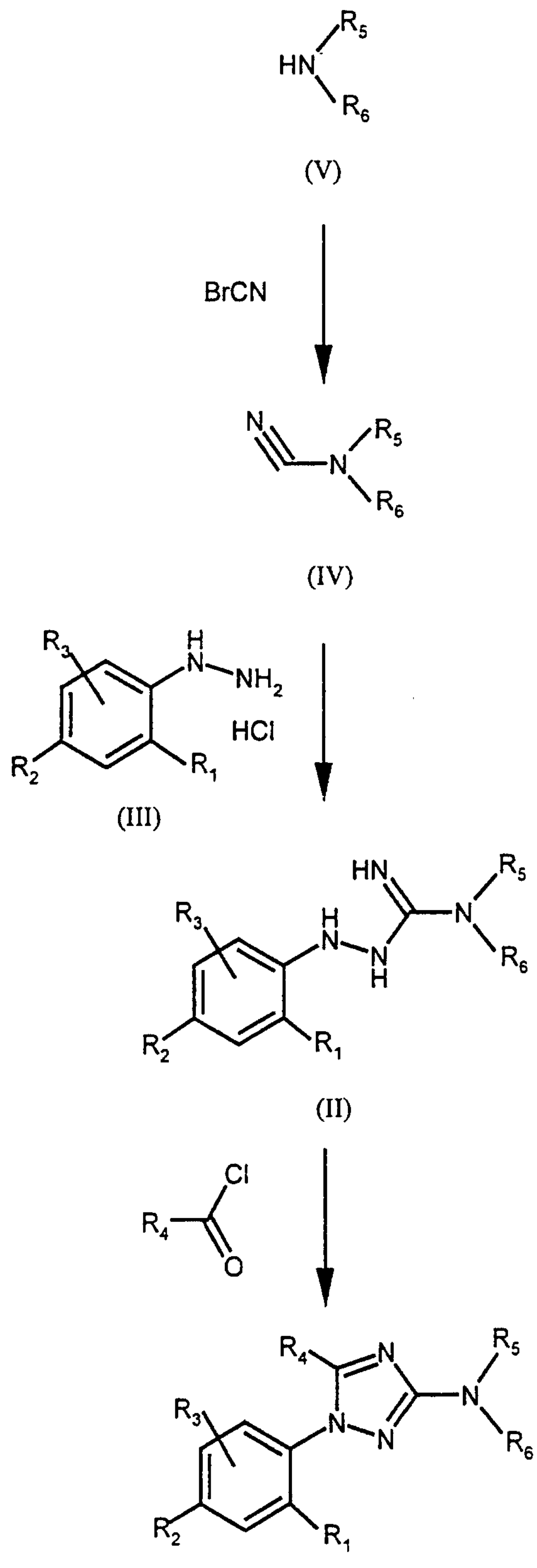


(V)

(IV)

(III)

(II)

(I)

Par la voie A, le composé (I) est préparé par condensation du chlorhydrate d'anilino-guanidine (II) avec un

halogénure d'acide de type $R_4$-CO-X ou un anhydride d'acide $R_4$-CO-O-CO-$R_4$ en présence de base organique (pyridine, triéthylamine...) dans un solvant organique de préférence la pyridine.

Dans le cas où $R_4$ représente l'hydrogène, l'halogénure d'acide est remplacé par de l'acide formique (Chem. Ber. 1965, 98, 1476-1486).

Le chlorhydrate d'anilino-guanidine (II) est obtenu par réaction d'un chlorhydrate de phénylhydrazine (III) avec une cyanamide (IV) dans un alcool à reflux, de préférence l'isopropanol, selon la méthode décrite par Erczi *et al.* (Eur. J. Med. Chem., 1993, *28*, 185-193).

Le chlorhydrate de phénylhydrazine (III) peut être commercial ou préparé à partir de l'aniline correspondante par diazotation puis réduction par le chlorure d'étain (II) ou par des sulfates alcalins.

Le cyanamide (IV) est préparée à partir de l'amine secondaire (V) par réaction avec le bromure de cyanogène dans un solvant organique comme l'éther diéthylique ou l'acétonitrile.

Cette voie de synthèse A est particulièrement efficace lorsque $R_5$ est un groupement de type alkyle.

Le SCHEMA 2 représente la voie de synthèse B.

Lorsque l'amine de départ est secondaire on utilise la variante B1 et lorsqu'elle est primaire, la variante B2.

Par la voie B1, l'aminotriazole (I) est préparé par condensation d'une phénylhydrazine (III) avec une *N*-acyl-*S*-méthylisothiourée (VI), par chauffage dans le toluène, le xylène, le diméthylformamide ou le diméthylsulfoxyde. Lorsque la phénylhydrazine (III) est salifiée par exemple sous forme chlorhydrate, une base comme la triéthylamine, la *N*,*N*-diéthylaniline ou le carbonate de césium est ajoutée.

La *N*-acyl-*S*-méthyl-isothiourée (VI) est obtenue à partir de la *N*-acyl-thiourée (VII) par S-méthylation par le iodométhane dans un solvant comme le dichlorométhane ou le tétrahydrofurane après action d'une base, de préférence l'hydrure de sodium, ou bien dans le diméthylformamide ou le tetrahydrofurane en présence de carbonate de césium.

La *N*-acyl-thiourée (VII) est obtenue par addition de l'amine secondaire (V) sur l'isothiocyanate d'acyle $R_4$CONCS. Les conditions opératoires, température et solvant, sont variables selon la réactivité des amines. Les solvants utilisés peuvent être par exemple de l'acétone, du dichlorométhane, du benzène, du toluène, du chloroforme.

## SCHEMA 2

Voie B1 | Voie B2

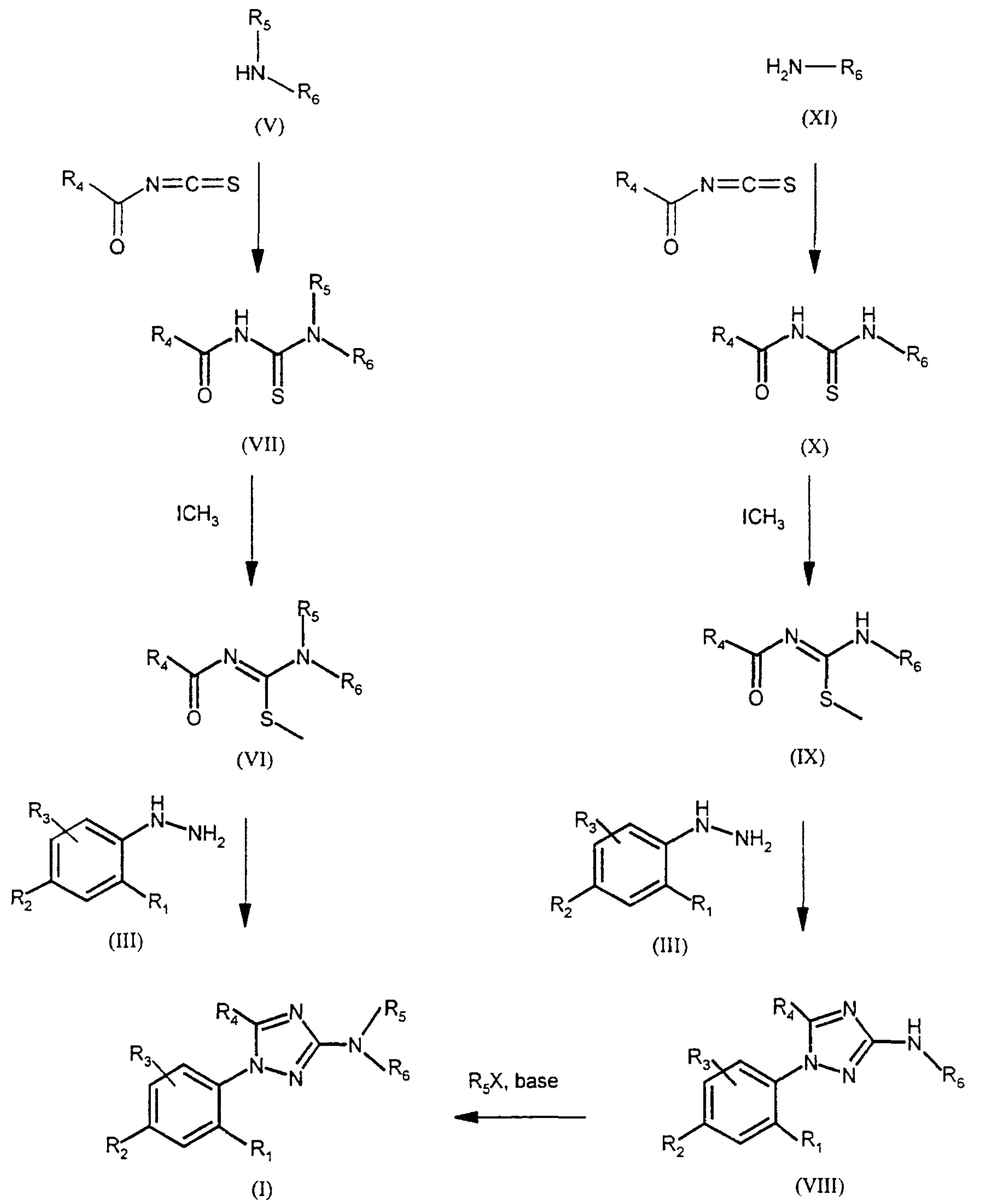


(V) (XI) (VII) (X) (VI) (IX) (III) (III) (I) (VIII)

L'isothiocyanate d'acyle $R_4CONCS$ est préparé préalablement par réaction entre un halogénure d'acyle $R_4COCl$ et un thiocyanate alcalin (thiocyanate d'ammonium par exemple) dans l'acétone en général.

Par la voie B2, l'aminotriazole (I) est préparé à partir d'un aminotriazole NH (VIII), par une réaction d'alkylation. Les conditions opératoires varient selon la réactivité de l'amine secondaire et de l'halogénure. Les systèmes utilisés peuvent être : hydrure de sodium/DMF, hydrure de potassium/benzène/éther couronne, hydrure de potassium/tetrahydrofurane/éther couronne, *tert*-butylate alcalin/DMSO.

Les conditions de synthèse pour la préparation de l'aminotriazole NH (VIII) à partir de l'amine primaire (XI) par les intermédiaires *N*-alcylthiourée (X) puis *N*-acyl-*S*-méthyl-isothiourée (IX) sont semblables à celles décrites pour la voie B1.

Les composés de formule (I) sont salifiés en général sous forme de chlorhydrate. Ces chlohydrates peuvent être sous forme de solides cristallins ou amorphes, obtenus par précipitation dans un solvant (ou un mélange de solvant) comme le pentane, l'éther diisopropylique, l'éther diéthylique.

Les amines primaires (XI) et secondaires (V) utilisées, lorsqu'elles présentent un carbone asymétrique ($R_6 = CH(R_8)R_7$) peuvent être sous forme racémique ou énantiomère.

Parmi lés méthodes habituelles de synthèse d'amines primaires, deux méthodes ont été particulièrement utilisées, l'une à partir d'amino-acide, l'autre à partir de phénylcétone substituée (1). Dans ce dernier cas, on procède selon le SCHEMA 3 ci-après. La phénylcétone substituée (1) est transformée en oxime (2) puis en benzyloxime (3). La benzyloxime (3) est réduite soit par de l'hydrure d'aluminium lithium pour obtenir une amine racémique, soit par un complexe chiral comme un complexe oxazaborolidine-borane chiral pour obtenir une amine sous la forme d'un énantiomère.

## SCHEMA 3

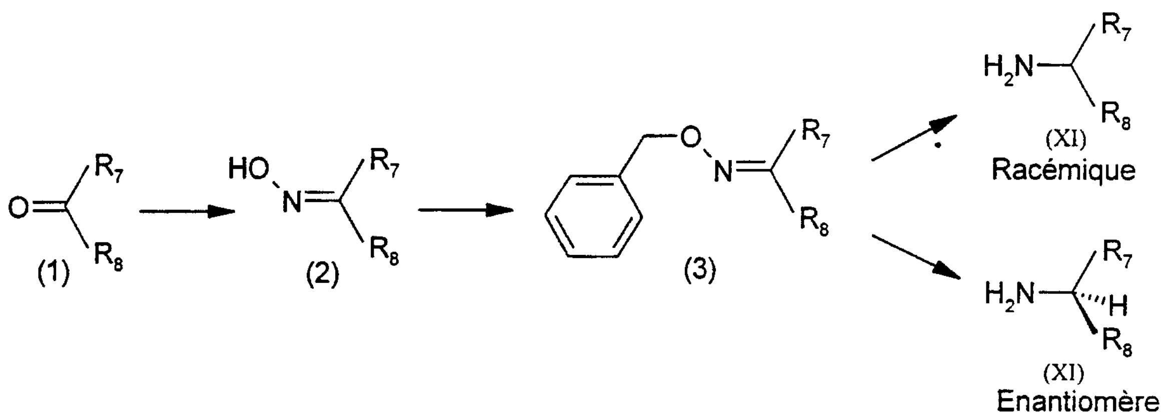
(1) (2) (3) (XI) Racémique (XI) Enantiomère

Les composés de formule (I), ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans les travaux de recherche, de métabolisme ou de pharmacocinétique, ou encore dans des essais biochimiques en tant que ligands de récepteur.

Les composés de la présente invention ont fait l'objet d'études biochimiques et pharmacologiques. Ils possèdent des propriétés pharmacologiques fort intéressantes. Les composés de l'invention déplacent, à des concentrations inférieures à 10 μM, la liaison du CRF ou de peptides apparentés iodés (urotensine, sauvagine) par exemple $^{125}$I-Tyr CRF, aux récepteurs présents sur membranes de cerveau ou sur des cellules en culture, selon la méthode décrite par E.B. De Souza (J. Neurosci., 1987, 7, 1 , 88-100).

L'activité antagoniste des composés selon l'invention a été démontrée par leur capacité à inhiber certaines activités associées au CRF. En particulier, les composés de formule (I) sont capables d'inhiber la sécrétion de corticotropine (ACTH) induite par le CRF. L'étude sur la sécrétion d'ACTH induite par le CRF a été réalisée, *in vivo* chez des rats éveillés, selon une méthode adaptée de C. Rivier *et al*., Endocrinology, 1982, *110*(1), 272-278.

Le CRF est un neuropeptide qui contrôle l'activité de l'axe hypothalamo-hypophyso-surrénalien. Ce facteur est responsable des réponses endocrines et comportementales liées au stress.

En effet, il a été démontré que le CRF peut moduler le comportement comme aussi certaines fonctions du système nerveux autonome (G.F. Koob, F.E. Bloom, Fed. Proc., 1985, 44, 259 ; M.R. Brown, L.A. Fisher, Fed. Proc., 1985, 44, 243). Plus particulièrement, le CRF induit la sécrétion de la corticotropine (ACTH), des β-endorphines et autres peptides dérivés de la pro-opiomélanocortine (A. Tazi *et al*., Régul. Peptides, 1987, *18*, 37 ; M.R. Brown *et al*, Regul. Peptides, 1986, *16*, 321 ; C.L. Williams *et al*., Am. J. Physiol., 1987, G 582, 253).

Les composés de l'invention peuvent donc être utiles à la régulation de la sécrétion de ces substances endogènes. Ils trouvent plus spécialement leurs applications en tant que principes actifs de médicaments pour diminuer la réponse au stress (comportement, états émotionnels, troubles gastro-intestinaux et cardiovasculaires, désordres du système immunitaire) et plus généralement dans les pathologies impliquant le CRF, par exemple les désordres

psychiatriques, l'anxiété, la dépression, l'anorexie et la boulimie nerveuse, l'épilepsie, les troubles de l'activité sexuelle et de la fertilité, la maladie d'Alzheimer ou autres.

Les composés de l'invention sont très stables et sont donc ainsi particulièrement appropriés pour constituer le principe actif de médicaments.

L'invention s'étend aussi aux compositions pharmaceutiques contenant comme principe actif, un composé de formule (I) ou l'un de ses sels pharmaceutiquement acceptables, éventuellement en association avec un ou plusieurs excipients inertes et appropriés.

Dans chaque unité de dosage le principe actif de formule (I) est présent dans les quantités adaptées aux doses journalières envisagées. Chaque unité de dosage est convenablement ajustée selon le dosage et le type d'administration prévu, par exemple comprimés, gélules et similaires, sachets, ampoules, sirops et similaires, gouttes, patch transdermique ou transmucosal de façon à ce qu'une telle unité de dosage contienne 0,5 à 800 mg de principe actif, de préférence 0,5 à 200 mg devant être administrés chaque jour.

Les composés selon l'invention peuvent également être utilisés en association avec un autre principe actif utile pour la thérapeutique souhaitée tels que par exemple des anxiolytiques, des antidépresseurs ou des anorexigènes.

Les composés de formule (I) sont peu toxiques ; leur toxicité est compatible avec leur utilisation comme médicament pour le traitement des troubles et des maladies ci-dessus.

Les composés de formule (I), peuvent être formulés dans des compositions pharmaceutiques pour l'administration aux mammifères, en particulier l'homme, pour le traitement des maladies susdites.

Les compositions pharmaceutiques ainsi obtenues sont présentées avantageusement sous des formes diverses, telles que par exemple, des solutions injectables ou buvables, dragées, comprimés ou gélules. Les compositions pharmaceutiques contenant comme principe actif au moins un composé de formule (I) ou l'un de ses sels, sont notamment utiles pour le traitement à titre préventif ou curatif, des maladies liées au stress et plus généralement dans le traitement de toutes les pathologies impliquant le CRF, tels que par exemple : la maladie de Cushing, les désordres neuropsychiatriques comme la dépression, l'anxiété, les attaques de panique, le stress post-traumatique, les désordres compulsifs obsessionnels, les troubles de l'humeur, les troubles du comportement, l'agressivité, l'anorexie, la boulimie, l'hyperglycémie, le travail prématuré, les grossesses à risque, la croissance retardée, les troubles du sommeil, l'épilepsie, et les dépressions de tout types ; les désordres dégénératifs : la maladie d'Alzheimer, de Parkinson, chorée de Huntington et sclérose latérale amyotrophique ; les troubles vasculaires, cardiaques et cérébraux ; les troubles de l'activité sexuelle et de la fertilité ; le travail prémature, l'immunodépression, l'immunosuppression, les processus inflammatoires, les infections multiples, la cystite interstitielle, l'arthrite rhumatoïde, l'ostéo-arthrite, les uvéites, le psoriasis ainsi que le diabète ; les cancers ; les troubles gastro-intestinaux fonctionnels et les inflammations qui en découlent (colon irritable et inflammatoire, diarrhées) ; les troubles de perception de la douleur, les fibromyalgies liées ou non aux troubles du sommeil, la fatigue, la migraine ; les symptômes liés à la dépendance (alcoolique) et au sevrage de drogues.

La posologie peut largement varier en fonction de l'âge, du poids et de l'état de santé du patient, de la nature et de la sévérité de l'affection, ainsi que de la voie d'administration. Cette posologie comprend l'administration d'une ou plusieurs doses d'environ 0,5 mg à 800 mg de principe actif, de préférence 0,5 à 200 mg chaque jour.

Dans les compositions pharmaceutiques de la présente invention pour l'administration orale, sublinguale, sous-cutanée, intramusculaire, intraveineuse, transdermique, transmucosale, locale ou rectale, le principe actif peut être administré sous formes unitaires d'administration, en mélange avec des supports pharmaceutiques classiques, aux animaux et aux êtres humains. Les formes unitaires d'administration appropriées comprennent les formes destinées à la voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Les EXEMPLES suivants illustrent l'invention.

Dans les PREPARATIONS sont décrites les méthodes de synthèse des différents intermédiaires permettant d'obtenir les composés de l'invention. Ces intermédiaires sont tous obtenus selon des méthodes bien connues de l'homme de l'art.

Les points de fusion ont été mesurés selon la technique Micro-Këfler et sont exprimés en degré Celsius.

Les spectres de résonance magnétique nucléaire du proton ($^1H$ RMN) ont été effectués dans le chloroforme deutérié ($CDCl_3$) sauf mention contraire, à 200 MHz ou à 300 MHz. Les déplacements chimiques sont exprimés en p.p.m. et les constantes de couplage en Hertz.

Les excès énantiomériques (ee) sont évalués à partir des chromatogrammes obtenus soit par chromatographie HPLC sur phase chirale, soit par chromatographie par fluide supercritique (SFC) chirale.

Les pouvoirs rotatoires des produits optiquement actifs sont caractérisés par leur $[\alpha]^{t°}_D$ (les concentrations c des solutions analysées sont exprimées en grammes pour 100 ml)

Les abréviations utilisées ci-après sont les suivantes : s = singulet ; m = multiplet ; d = doublet ; t = triplet ; q

= quadruplet ; c-Pr = radical cyclopropyle ; Ph = radical phényle.

Les composés de l'invention présentent une analyse centésimale conforme à la théorie.

Les composés de l'invention décrits dans les TABLEAUX 1 et 6 ont également des spectres RMN et des spectres de masse conformes à leur structure.

**PREPARATION DES SYNTHONS INITIAUX**

**1) <u>Préparation des phénylhydrazines de formule III</u>**

**• Chlorhydrate de 2-chloro-4-méthoxy-5-méthylphénylhydrazine *Composé III.1*.**

Une solution de 8,6 g (50 mmol) de 2-chloro-4-méthoxy-5-méthylaniline dans 75 ml d'acide chlorhydrique 5N est agitée à -5°C et additionnée de 3,52 g (51 mmol) de nitrite de sodium en solution dans 12,5 ml d'eau. Le mélange est agité pendant une heure à 0°C puis additionné d'une solution de 22,56 g (100 mmol) de chlorure d'étain (II) dihydraté dans 20 ml d'acide chlorhydrique à 35 %. Le mélange est agité pendant deux heures avec retour progressif à température ambiante. Le précipité formé est filtré et lavé à l'acide chlorhydrique normal, l'éthanol puis l'éther diéthylique. Après séchage au dessiccateur, on obtient 7,8 g de *composé III.1*. F = 140°C. Rendement 70 %.
$^1$H RMN (DMSO-$D_6$, $\delta$ ppm) : 2,09(s, 3H, $CH_3$) ; 3,73(s, 3H, $OCH_3$) ; 7,0(s, 1H, Ph) ; 7,05(s, 1H, Ph) ; 7,58(s, 1H, NH) ; 10,13(s, 3H, $NH_3^+$)

**2) <u>Préparation des amines secondaires de formule V</u>**

**• [2-Cyclopropyl-1-(4-fluorophényl)éthyl]propylamine *Composé V.1***

Une solution de 8,9 g (50 mmol) de 2-cyclopropyl-1-(4-fluorophényl)éthanone dans 100 ml de dichlorométhane avec 16,4 ml (200 mmol) de propylamine est agitée à 0°C et additionnée lentement de 30 ml d'une solution normale de tétrachlorure de titane dans le dichlorométhane. Le mélange est agité pendant quinze heures à température ambiante puis refroidi à 0°C et additionné de 100 ml de méthanol. 2,1 g (55 mmol) de borohydrure de sodium sont ajoutés par portions et le mélange est agité pendant deux heures à température ambiante. Le mélange est concentré sous pression réduite à environ 100 ml puis additionné de 100 ml d'eau. La suspension est filtrée et le filtrat, repris par le dichlorométhane, est lavé à l'eau puis à l'eau saturée en chlorure de sodium, séché sur sulfate de sodium puis les solvants sont évaporés sous pression réduite. On obtient 9,43 g de produit huileux. Rendement 85 %.
$^1$H RMN ($CDCl_3$, $\delta$ ppm) : -0,08-0,12(m, 2H , c-Pr) ; 0,28-0,50(m, 2H, c-Pr) ; 0,50-0,62(m, 1H, c-Pr) ; 0,81-0,88(m, 3H, $CH_3$) ; 1,35-1,70(m, 5H, $CH_2$, $CH_2$-c-Pr et NH) ; 2,30-2,45(m, 2H, N-$CH_2$) ; 3,62-3,68(m, 1H, CH) ; 6,90-7,05(m, 2H, Ph) ; 7,21-7,31(m, 2H, Ph).

**3) <u>Préparation des amines primaires de formule XI</u>**

**<u>Première méthode : préparation des amines primaires à partir d'aminoacides</u>**

**a) • (1*R*)-2-amino-2-(4-fluorophényl)éthanol**

240 ml (240 mmol) d'une solution 1M d'hydrure d'aluminium-lithium dans le tétrahydrofurane sont agités à reflux puis additionnés par portions de 20 g (118 mmol) de (R)-(4-fluorophényl)glycine Après agitation à reflux pendant six heures trente minutes, le mélange réactionnel est agité à 0°C puis additionné lentement de 9,5 ml d'eau, 9,5 ml d'une solution d'hydroxyde de sodium à 15 % puis 28,5 ml d'eau. La suspension obtenue est filtrée sur célite. Le filtrat est concentré et repris par 1 litre de dichlorométhane. La solution est lavée avec une solution saturée en chlorure de sodium, séchée sur du sulfate de sodium anhydre puis les solvants sont évaporés sous pression réduite. Une cristallisation dans l'éther isopropylique permet d'obtenir 13,22 g (85,2 mmol) de produit cristallisé. Rendement 72 %. F = 95°C.
$^1$H RMN (DMSO-$D_6$) : 1,82(s, 2H, $NH_2$); 3,35-3,45(m, 2H, $CH_2O$); 3,84(m,1H, CH); 4,73(s, 1H, OH); 7,01-7,13(m, 2H, Ph); 7,30-7,41(m, 2H, Ph).

**b) • (1*R*)-1-(4-fluorophényl)-2-méthoxyéthylamine *Composé XI.1***

3,64 g (91 mmol) d'hydrure de potassium obtenus par lavage au pentane de 8,1 g d'une suspension huileuse, sont mis en suspension dans 70 ml de tétrahydrofurane et agités à 10°C. Une solution de 13,22 g (85 mmol) de (1 R)-2-amino-2-(4-fluorophényl)éthanol dans 175 ml de tétrahydrofurane est additionnée lentement. Après agitation pendant seize heures à température ambiante, une solution de 5,2 ml (83,5. mmol) d'iodométhane dans 105 ml de tétrahydrofurane est additionnée en deux heures. Le mélange réactionnel est agité pendant trois heures à température ambiante puis est versé sur 1 litre d'eau glacée salée. Le mélange est extrait par 1 litre de *tert*-butylméthyloxyde. La phase organique est lavée à l'eau, puis avec une solution saturée en chlorure de sodium, séchée sur sulfate de sodium anhydre puis les solvants sont évaporés sous pression réduite. On obtient 11,87 g (70 mmol) d'amine huileuse. Rendement 82 %.
$^1$H RMN ($CDCl_3$): 1,66(s, 2H, $NH_2$); 3,29(d, 1H, $CH_2$); 3,36(s, 3H, $OCH_3$); 3,45(dd, 1H, $CH_2$); 4,16(m, 1H, CH); 6,93-7,05 (m, 2H, Ph); 7,24-7,38(m, 2H, Ph)

**Deuxième méthode : préparation des amines primaires à partir de phénylcétones**

**a) Synthèse de phénylcétones substituées *Composé 1***

**♦ Mode opératoire A**

**• 2-Cyclopropyl-1-(3-fluoro-4-méthylphényt)éthan-1-one *Composé 1.1***

Une solution de 61 g (323 mmol) de 4-bromo-3-fluoro-toluène dans 280 ml d'éther diéthylique est additionnée lentement à 7,8 g (323 mmol) de tournure de magnésium de manière à créer un léger reflux. Le mélange est ensuite chauffé à reflux pendant deux heures puis refroidi et filtré sur laine de verre. Le filtrat est agité à 0°C et additionné de 25 g (308 mmol) de cyclopropylacétonitrile dilué dans 20 ml d'éther diéthylique. Le mélange réactionnel est agité pendant trois heures à température ambiante puis est refroidi à 0°C et additionné lentement par une solution d'acide chlorhydrique 1 N jusqu'à l'obtention d'un pH égal à 1. Le mélange est extrait trois fois par l'acétate d'éthyle et les phases organiques réunies sont lavées à l'eau puis à l'eau saturée en chlorure de sodium, séchées sur sulfate de sodium et les solvants sont évaporés sous pression réduite. On obtient 53 g de produit brut utilisé tel quel dans l'étape suivante. Rendement : environ 85 %.
$^1$H RMN ($CDCl_3$, $\delta$ ppm) : 0,15-0,21(m, 2H, c-Pr); 0,55-0,65(m, 2H, c-Pr); 1,07-1,20(m, 1H, c-Pr); 2,31 (d, J=1,9Hz, 3H, $CH_3$); 2,82(d, J=6,7Hz, 2H, $CH_2$-c-Pr); 7,22-7,30(m, 1H, Ph); 7,54-7,64(m, 2H, Ph).
Par le même procédé la cétone suivante a été synthétisée :

**• 2-Cyclopropyl-1-(4-méthylphényl)éthan-1-one *Composé 1.2***

**♦ Mode opératoire B**

**• 2-Cyclopropyl-1-(4-méthoxyméthylphényl)éthan-1-one *Composé 1.3***

Une solution de 32,5 g (162 mmol) de 1-bromo-4-méthoxyméthylphényle dans 300 ml de tétrahydrofurane est agitée à -60°C et additionnée lentement de 112 ml (179 mmol) d'une solution 1,6 M de butyl-lithium. Le mélange réactionnel est agité pendant 30 minutes à -60°C, puis additionné lentement d'une solution de 27,6 g (192 mmol) de 2-cyclopropyl-*N*-méthoxy-*N*-méthylacétamide. Le mélange réactionnel est agité en laissant la température remonter progressivement à l'ambiante. Après 4 heures sous agitation, il est refroidi à 0°C et additionné lentement de 5 ml d'éthanol. Le mélange est extrait par l'acétate d'éthyle et la phase organique est lavée à l'eau puis avec une solution saturée en chlorure de sodium, séchée sur sulfate de sodium anhydre puis les solvants sont évaporés sous pression réduite. Le résidu obtenu est purifié par chromatographie sur une colonne de gel de silice (solvant: cyclohexane puis cyclohexane/acétate d'éthyle 20/1 (v/v)). On obtient 21.8 g de cétone. Rendement 66%.
$^1$H RMN ($CDCl_3$, $\delta$ ppm): 0,13-0,21(m, 2H, c-Pr); 0,53-0,62(m, 2H, c-Pr); 0,84-0,93(m, 1H, c-Pr); 2,85(d, J=6,6 Hz, 2H, $CH_2$-c-Pr); 3,40(s, 3H, $OCH_3$); 4,68(s, 2H, $OCH_2$); 7,57(d, J=7,5 Hz, 2H, Ph); 7,92(d, J=7,5 Hz, 2H, Ph)
Par le même procédé la cétone suivante a été synthétisée :

**• 2-Cyclopropyl-1-(3,4-méthylènedioxyphényl)éthan-1-one *Composé 1.4***

**b) Synthèse d'oximes substituées *Composé 2***

**• 2-Cyclopropyl-1-(3-fluoro-4-méthylphényl)éthan-1-one oxime (*E*) *Composé 2.1***

Une solution de 53 g (275 mmol) de *composé 1.1* dans 200 ml de pyridine est agitée à 0°C et additionnée lentement de 28,5 g (410 mmol) de chlorhydrate d'hydroxylamine. Le mélange est agité pendant 12 heures à température ambiante puis est concentré sous pression réduite. Le résidu est repris par l'acétate d'éthyle et la phase organique est lavée trois fois à l'eau puis à l'eau saturée en chlorure de sodium, séchée sur sulfate de sodium puis les solvants sont évaporés sous pression réduite. Le résidu est purifié par chromatographie sur une colonne de gel de silice (éluant :cyclohexane/acétate d'éthyle 20/1 (v/v)). On obtient 30 g d'oxime. Rendement 52 %.
$^1$H RMN (DMSO-$D_6$, $\delta$ ppm) : 0,10-0,20(m, 2H, c-Pr) ; 0,28-0,40(m, 2H, c-Pr) ; 0,78-0,95(m, 1H, c-Pr) ; 2,21(d, J=1,7Hz, 3H, $CH_3$) ; 2,63(d, J=6,8Hz, 2H, $CH_2$-c-Pr) ; 7,20-7,56(m, 3H, Ph) ; 11,16(s, 1H, OH).

Par le même procédé les oximes suivantes ont été synthétisées :

- **2-cyclopropyl-1-phényléthan-1-one oxime (*E*) *Composé 2.2***
- **2-cyclopropyl-1-(4-fluorophényl)éthan-1-one oxime (*E*) *Composé 2.3***
- **2-cyclopropyl-1-(4-méthylphényl)éthan-1-one oxime (*E*) *Composé 2.4***
- **2-cyclopropyl-1-(4-méthoxyméthylphényl)éthan-1-one oxime (*E*) *Composé 2.5***
- **2-cyclopropyl-1-(3,4-méthylènedioxyphényl)éthan-1-one oxime (*E*) *Composé 2.6***
- **1-phénylbutan-1-one oxime (*E*) *Composé 2.7***
- **1-(4-méthylphényl)butan-1-one oxime (*E*) *Composé 2.8***

**c) Synthèse de O-benzyloximes substituées *Composé 3***

- **2-Cyclopropyl-1-(3-fluoro-4-méthylphényl)éthan-1-one *O*-benzyloxime (*E*) *Composé 3.1***

Une solution de 30 g (144 mmol) de *composé 2.1* dans 140 ml de diméthylformamide est agitée à 0°C et additionnée par portions de 8,3 g (180 mmol) d'hydrure de sodium à 55 % dans l'huile. Le mélange est agité pendant trente minutes à 0°C puis 20,5 ml (172 mmol) de bromure de benzyle sont additionnés lentement. Le mélange réactionnel est agité pendant trois heures à température ambiante, puis est refroidi à 0°C et additionné de 10 ml d'éthanol puis 500 ml d'eau. Le mélange est extrait par l'acétate d'éthyle et la phase organique est lavée à l'eau puis à l'eau saturée en chlorure de sodium, séchée sur sulfate de sodium et les solvants sont évaporés sous pression réduite. Le résidu est purifié par chromatographie sur une colonne de gel de silice (éluant : cyclohexane/dichlorométhane 95/5(v/v)). On obtient 30,2 g de benzyloxime *E.* Rendement 70 %.
$^1$H RMN (DMSO-$D_6$, $\delta$ ppm) : 0,10-0,17(m, 2H, c-Pr) ; 0,28-0,40(m, 2H, c-Pr) ; 0,78-0,90(m, 1H, c-Pr) ; 2,22(d, J=1,8Hz, 3H, $CH_3$) ; 2,67(d, J=6,8Hz, 2H, $CH_2$-c-Pr) ; 5,16(s, 2H, O-$CH_2$-Ph) ; 7,20-7,43(m, 8H, Ph).

Par le même procédé les benzyloximes suivantes ont été synthétisées

- **2-Cyclopropyl-1-phényléthan-1-one *O*-benzyloxime (*E*) *Composé 3.2***
- **2-Cyclopropyl-1-(4-fluorophényl)éthan-1-one *O*-benzyloxime (*E*) *Composé 3.3***
- **2-Cyclopropyl-1-(4-méthylphényl)éthan-1-one *O*-benzyloxime (E) *Composé 3.4***
- **2-Cyclopropyl-1-(4-méthoxyméthylphényl)éthan-1-one *O*-benzyloxime (*E*) *Composé 3.5***
- **2-Cyclopropyl-1-(3,4-méthylènedioxyphényl)éthan-1-one *O*-benzyloxime (*E*) *Composé 3.6***
- **1-Phénylbutan-1-one *O*-benzyloxime (*E*) *Composé 3.7***
- **1-(4-Méthylphényl)butan-1-one *O*-benzyloxime (*E*) *Composé 3.8***

**d) Synthèse d'amines primaires racémiques de formule XI**

- **2-Cyclopropyl-1-(4-fluorophényl)éthylamine *Composé XI.2***

Une suspension de 10,6 g (280 mmol) d'hydrure d'aluminiumlithium dans 500 ml de tétrahydrofurane est agitée à température ambiante et additionnée lentement de 40 g (140 mmol) de la 2-cyclopropyl-1-(4-fluorophényl) éthan-1-one *O*-benzyloxime. Le mélange est agité au reflux pendant quatre heures puis est refroidi à 0°C et additionné

goutte à goutte de 10,6 ml d'eau, de 10,6 ml d'une solution d'hydroxyde de sodium à 15 %, puis de 32 ml d'eau. La suspension obtenue est filtrée sur célite et lavée par l'acétate d'éthyle. Les filtrats organiques réunis sont lavés à l'eau puis à l'eau saturée en chlorure de sodium, séchés sur sulfate de sodium et les solvants sont évaporés sous pression réduite. L'extrait brut est purifié par chromatographie sur une colonne de gel de silice (éluant : dichlorométhane/méthanol 95/5 (v/v)). On obtient 14,2 g d'amine sous forme huileuse. Rendement 57 %.

$^1$H RMN ($CDCl_3$, δ ppm) : - 0,05-0,18(m, 2H, c-Pr); 0,32-0,50(m, 2H, c-Pr); 0,50-0,70(m, 1H, c-Pr); 1,40-1,70(m, 2H, $CH_2$-c-Pr); 1,76(s, 2H, $NH_2$); 3,97-4,05(m, 1H, CH); 6,92-7,04(m, 2H, Ph); 7,24-7,34(m, 2H, Ph).

Par le même procédé, les amines racémiques suivantes ont été synthétisées :

- **2-Cyclopropyl-1-phényléthylamine *Composé XI.3***
- **2-Cyclopropyl-1-(3-fluoro-4-méthylphényl)éthylamine *Composé XI.4***

**e) <u>Synthèse d'amines primaires chirales de formule XI</u>**

- **(1*S*)-2-Cyclopropyl-1-(3-fluoro-4-méthylphényl)éthylamine *Composé XI.5***

Une solution de 37,17 g (145 mmol) de (*S*)-2-amino-3-méthyl-1,1-diphénylbutan-1-ol dans 180 ml de tétrahydrofurane est agitée à -40°C et additionnée lentement de 285 ml d'une solution 1M de complexe borane-tétrahydrofurane (285 mmol). Le mélange est agité pendant trois heures de -40°C à température ambiante, puis il est refroidi à -10°C et additionné d'une solution de 17 g (57 mmol) de composé *3.1*. Le mélange réactionnel est agité pendant vingt heures à température ambiante puis est refroidi à -10°C et additionné de 285ml d'acide chlorhydrique 2N. On laisse sous agitation pendant vingt heures puis le tétrahydrofurane est évaporé sous pression réduite. Il se forme un précipité de chlorhydrate de (*S*)-2-amino-3-méthyl-1,1-diphénylbutan-1-ol qui est filtré et rincé par l'acide chlorhydrique 1 N. Les filtrats acides réunis sont lavés par du *tert*-butylméthyléther puis refroidis à 0°C et alcalinisés lentement par une solution aqueuse d'hydroxyde de sodium à 35 %. Après extraction par trois fois par du dichlorométhane, les phases organiques réunies sont lavées à l'eau puis à l'eau saturée en chlorure de sodium, séchées sur sulfate de sodium et les solvants sont évaporés sous pression réduite. Le résidu est purifié par chromatographie sur une colonne de gel de silice (éluant : dichlorométhane/méthanol 95/5 (v/v)). On obtient 7,2 g d'amine sous forme huileuse. Rendement 47 %. ee > 98%

$[\alpha]_D^{22}$ = + 5,4 (c = 1,16, dichlorométhane)

$^1$H RMN ($CDCl_3$, δ ppm) : -0,05-0,15(m, 2H, c-Pr); 0,35-0,50(m, 2H, c-Pr); 0,55-0,70(m, 1H, c-Pr); 1,43-1,70(m, 2H, $CH_2$-c-Pr); 2,0(s, 2H, $NH_2$); 2,25(d, J=1,7Hz, 3H, $CH_3$); 3,99-4,04(m, 1H, CH); 6,99-7,17(m, 3H, Ph).

Par le même procédé, les amines chirales suivantes ont été synthétisées :

- **(1*S*)-2-cyclopropyl-1-phényléthylamine** *ee* = 96% ***Composé XI.6***
- **(1*S*)-2-cyclopropyl-1-(4-fluorophényl)éthylamine** *ee* = 98 % ***Composé XI.7***
- **(1*S*)-2-cyclopropyl-1-(4-méthylphényl)éthylamine** *ee* = 97,2 % ***Composé XI.8***
- **(1*S*)-2-cyclopropyl-1-(4-méthoxyméthylphényl)éthylamine** *ee* = 97,8 % ***Composé XI.9***
- **(1*S*)- 2-cyclopropyl-1-(3,4-méthylènedioxyphényl)éthylamine** *ee* = 96,6 % ***Composé XI.10***
- **(1*S*)-1-phényl butylamine** *ee* > 99 % ***Composé XI.11***
- **(1*S*)-1-(4-méthylphényl)butylamine** *ee* = 97,9 % ***Composé XI.12***

Les excès énantiomériques (ee) de ces composés ont été évalués par chromatographie par fluide supercritique chirale de leur dérivé acétamide ou thiourée. Seul l'excès énantiomérique du *composé XI.11* a été évalué directement par HPLC en phase chirale. Pour ce composé une salification puis une recristallisation avec la *N*-acétyl-L-leucine (Yamamoto Y et al , Bull. Chem. Soc. Jpn. 1976, *49*(11), 3247-3249) a permis d'améliorer l'excès énantiomérique.

**PREPARATION PAR LA VOIE A**

**1) Préparation des cyanamides de formule IV**

• **(1-Phénylbutyl)propylcyanamide *Composé IV.1***

Une suspension de 1,4 g (16,4 mmol) de carbonate de magnésium dans 20 ml d'un mélange d'éther diéthylique/eau 9/1 (v/v) est agitée à 0°C et additionnée de 5 g (47 mmol) de bromure de cyanogène. A ce mélange, agité à 0°C, sont ajoutés lentement 9 g (47 mmol) de (1-phénylbutyl)propylamine. Après agitation pendant une heure à température ambiante, le mélange réactionnel est additionné de 50 ml d'eau puis 100 ml d'éther diéthylique. La phase éthérée est lavée à l'eau puis avec une solution saturée en chlorure de sodium. Elle est séchée sur sulfate de sodium puis les solvants sont évaporés sous pression réduite. Le résidu huileux obtenu est distillé au four à boule à 150°C sous ≅ 0,3 mm de Hg. 7,6 g d'huile incolore sont obtenus. Rendement 74 %.
$^1$H RMN ($CDCl_3$, δ ppm) : 0,84-0,95(m, 6H, $2CH_3$); 1,28-2,10(m, 6H, $3CH_2$); 2,65-2,90(m, 2H, $NCH_2$); 3,77(t, J=7,5Hz, 1H, CH); 7,25-7,39(m, 5H, Ph).

Par la même méthode le composé suivant a été synthétisé:

• **[2-Cyclopropyl-1-(4-fluorophényl)éthyl]propylcyanamide *Composé IV.2***

$^1$H RMN ($CDCl_3$, δ ppm) : 0-0,2(m, 2H, c-Pr); 0,37-0,55(m, 2H, c-Pr); 0,60-0,75(m, 1H, c-Pr); 0,85-0,93(m, 3H, $CH_3$); 1,55-1,75(m, 3H, $CH_2$ et HC<u>H</u>-c-Pr); 1,95-2,10(m, 1H, HC<u>H</u>-c-Pr); 2,72-2,90(m, 2H, $NCH_2$); 3,87(t, J=7,5Hz, 1H, CH); 6,98-7,09(m, 2H, Ph); 7,25-7,35(m, 2H, Ph).

**2) Préparation des anilino-guanidines de formule II**

• **Chlorhydrate de *N*-(2,4-dichlorophénylamino)-*N'*-(1-phénylbutyl)-*N'*-propylguanidine *Composé II.1***

Un mélange constitué de 5,13 g (24 mmol) de chlorhydrate de 2,4-dichlorophénylhydrazine, 6,5 g (30 mmol) du *composé IV.1* et 10 ml de *n*-propanol anhydre est agité à 130°C pendant vingt-quatre heures. Après avoir été refroidi, le mélange réactionnel est mis en suspension dans 50 ml d'éther diéthylique. Le précipité est filtré puis repris par 50 ml d'acétone. La suspension obtenue est agitée pendant trente minutes à température ambiante puis le précipité est filtré et lavé à l'acétone. Il est ensuite dissout à chaud dans 10 ml de méthanol puis additionné de 50 ml d'éther diéthylique. Il se forme un précipité blanc qui est filtré, lavé à l'éther et séché. On obtient 6 g (14 mmol) de poudre blanche. Rendement 58 %.
F = 225°C

Par la même méthode le composé suivant a été synthétisé:

• **Chlorhydrate de *N*-[2-cyclopropyl-1-(4-fluorophényl)éthyl]-*N'*-(2,4-dichlorophényl-amino)-*N*-propyl-guanidine *Composé II.2***

$^1$H RMN (DMSO $D_6$, δ ppm) : 0,1-0,7(m, 5H, c-Pr); 0,78(m, 3H, $CH_3$); 1,4-1,75(m, 2H, $CH_2$); 1,85-2,15(m, 2H, $CH_2$); 3,0-3,4(m, 2H, $NCH_2$); 5,41 (t, 7,3Hz, 1H, CH); 7,73(d, 1H, Ph); 7,1-7,6(m, 6H, Ph); 8,1(s, 2H, $NH_2$); 8,23 (s, 1H, NH); 9,98(s, 1H, NH)

**3) Préparation des aminotriazoles de formule I par la voie A**

**EXEMPLE 1 • [1-(2,4-Dichlorophényl)-5-méthyl-*N*-(1-phénylbutyl)-*N*-propyl-1*H*-1,2,4-triazol-3-amine**

Une suspension de 1,29 g (3 mmol) de *composé II.1* dans 12 ml de pyridine est agitée à 0°C et additionnée lentement de 1,07 ml (15 mmol) de chlorure d'acétyle. Le mélange réactionnel est agité pendant vingt heures à température ambiante puis est versé sur 100 ml d'eau glacée. Après acidification à pH 1 par de l'acide chlorhydrique 1 N, le mélange est extrait à l'acétate d'éthyle. La phase organique est ensuite lavée par une solution saturée d'hydrogénocarbonate de sodium, par de l'eau, puis par de l'eau saturée en chlorure de sodium. Elle est séchée par du sulfate de sodium anhydre puis évaporée. Le résidu brut est purifié par chromatographie sur une colonne de gel de silice (éluant : cyclohexane/acétate d'éthyle 9/1 (v/v)). On obtient 645 mg (1,54 mmol) de produit gommeux incolore. Rendement 52 %.
$^1$H RMN ($CDCl_3$, δ ppm) : 0,71(m, 3H, $CH_3$); 0,94(m, 3H, $CH_3$); 1,35-1,55(m, 4H, $2CH_2$); 1,96-2,02(m, 2H, $CH_2$); 2,23 (s, 3H, $CH_3$); 2,97-3,07(m, 2H, $NCH_2$); 5,41(t, J=7,6Hz, 1H, CH); 7,18-7,40(m, 7H, Ph); 7,53(d, J=1,9Hz, 1H, Ph).

Ce produit est salifié sous forme de chlorhydrate ; F = 142°C (HCl)

Par la même méthode sont synthétisés les EXEMPLES 2 à 9 du TABLEAU 1 suivant :

## TABLEAU 1 : Composés de formule I synthétisés par la voie A

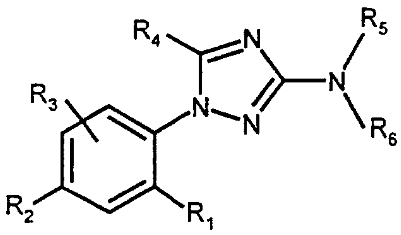

(I)

| Exemples N° | $R_1,R_2,R_3$ | $R_4$ | $R_5$ | $R_6$ | Sel ; F (°C) |
|---|---|---|---|---|---|
| **2** | 2-Cl<br>4-Cl<br>H | 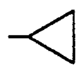 | $-(CH_2)_2CH_3$ | 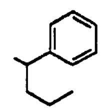 | HCl ; 114 |
| **3** | 2-Cl<br>4-Cl<br>H | $-CH_3$ | $-(CH_2)_2CH_3$ | 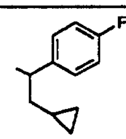 | HCl ; 142 |
| **4** | 2-Cl<br>4-Cl<br>H | $-CH_2CH_3$ | $-(CH_2)_2CH_3$ | 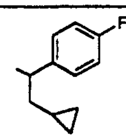 | HCl ; 153 |
| **5** | 2-Cl<br>4-Cl<br>H | $-CH_2CH_2CH_3$ | $-(CH_2)_2CH_3$ | 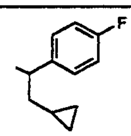 | HCl ; 141 |

| Exemples N° | $R_1,R_2,R_3$ | $R_4$ | $R_5$ | $R_6$ | Sel ; F (°C) |
|---|---|---|---|---|---|
| **6** | 2-Cl<br>4-Cl<br>H | 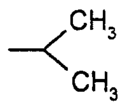 | $-(CH_2)_2CH_3$ | 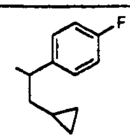 | HCl ; 153 |
| **7** | 2-Cl<br>4-Cl<br>H | 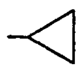 | $-(CH_2)_2CH_3$ | 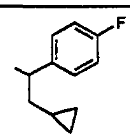 | HCl ; 132 |
| **8** | 2-Cl<br>4-Cl<br>H | 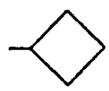 | $-(CH_2)_2CH_3$ | 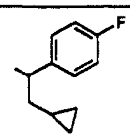 | HCl ; 144 |
| **9** | 2-Cl<br>4-Cl<br>H | $-CH_2OCH_3$ | $-(CH_2)_2CH_3$ | 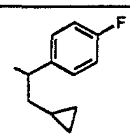 | HCl ; 130 |

**PREPARATION PAR LA VOIE B1**

**1) <u>Préparation des *N*-acyl-thiourées de formule VII</u>**

**•*N*-acétyl-*N'*-[2-cyclopropyl-1-(4-fluorophényl)éthyl]-*N'*-propylthiourée *Composé VII.1***

Une solution de 2 g (26,4 mmol) de thiocyanate d'ammonium dans 27 ml d'acétone est agitée à température ambiante et additionnée lentement de 1,82 ml (24,2 mmol) de chlorure d'acétyle. Après dix minutes sous agitation, une solution de 4,87 g (22 mmol) de *composé V.I* dans 44 ml de dichlorométhane est additionnée lentement. Après agitation pendant trente minutes à température ambiante, le mélange réactionnel est additionné de 100 ml de dichlorométhane et 100 ml d'eau. La phase organique est lavée à l'eau puis à l'eau saturée en chlorure de sodium, séchée sur sulfate de sodium et les solvants sont évaporés sous pression réduite. Le résidu brut. est purifié par chromatographie sur une colonne de gel de silice (éluant : cyclohexane/acétate d'éthyle 4/1 (v/v). On obtient 5,5 g (17 mmol) de *composé VII.1*. Rendement 77 %.
$^1H$ RMN (DMSO-$D_6$, δ ppm) : 0,1-0,2(m, 2H, c-Pr); 0,35-0,50(m, 2H, c-Pr); 0,50-0,70(m, 3H, $CH_3$); 0,75-0,95(m, 1H, c-Pr); 1,25-1,75(m, 2H, $CH_2$); 1,80-2,10(m, 5H, $CH_2$-c-Pr et $COCH_3$); 3,10-3,60(m, 2H, $NCH_2$); 5,32(m, 1H, CH); 7,15-7,24(m, 2H, Ph); 7,55-7,62(m, 2H, Ph); 10,33(s, 1H, NH).

**2) <u>Préparation de *N*-acyl-*S*-méthyl-isothiourées *de* formule VI</u>**

**•*N*-acétyl-*N'*-[2-cyclopropyl-1-(4-fluorophényl)éthyl]-*S*-méthyl-*N'*-propylisothiourée *Composé VI.1***

Une solution de 5,5 g (17 mmol) de *composé VII.1* dans 170 ml de dichlorométhane est agitée à 0°C et additionnée de 740 mg (18,5 mmol) d'hydrure de sodium à 60 % dans l'huile. Après agitation pendant 10 minutes à 0°C, le mélange réactionnel est additionné de 2,1 ml (34 mmol) d'iodure de méthyle. Le mélange réactionnel est agité pendant trois heures à température ambiante puis est refroidi à 0°C et additionné de 10 ml d'éthanol puis de 10 ml d'eau. La phase organique est lavée à l'eau puis à l'eau saturée en chlorure de sodium, séchée sur sulfate de sodium puis et les solvants sont évaporés sous pression réduite. Le résidu brut, huileux et incolore est utilisé tel quel dans l'étape suivante (rendement quantitatif).
$^1H$ RMN ($CDCl_3$, δ ppm) : 0,1-0,16(m, 2H, c-Pr); 0,45-0,52(m, 2H, c-Pr); 0,62-0,70(m, 3H, $CH_3$); 0,80-0,95(m, 1H, CH, c-Pr); 1,0-1,2 et 1,45-1,65(2m, 2H, $CH_2$); 1,8-2,05(m, 2H, $CH_2$-c-Pr), 2,18(s, 3H, $CH_3CO$); 2,41(s, 3H, $SCH_3$); 3,04-3,20 (m, 2H, $NCH_2$); 5,80(t, 7,6Hz, 1H, CH); 6,96-7,08(m, 2H, Ph); 7,30-7,38(m, 2H, Ph).

3) Préparation des aminotriazoles de formule (I) par la voie B1

**EXEMPLE 10**

**• *N*-[2-Cyclopropyl-1-(4-fluorophényl)éthyl]-1-[2,6-dichioro-4-(trifluorométhyl)phényl]-5-méthyl-*N*-propyl-1*H*-1,2,4-triazol-3-amine**

Un mélange constitué de 1 g (3 mmol) de *composé VI.1*, 1 g (4 mmol) de 2,6-dichloro-4-trifluorométhylphénylhydrazine et 8 ml de diméthylsulfoxyde anhydre est agité et chauffé progressivement de 100 à 200°C en trente heures jusqu'à disparition de l'isothiourée de départ. Le mélange réactionnel préalablement refroidi est versé dans de l'eau glacée et acidifié par de l'acide chlorhydrique normal. Le mélange est extrait par l'acétate d'éthyle et l'extrait organique est lavé avec une solution aqueuse saturée en hydrogénocarbonate de sodium puis avec de l'eau et de l'eau saturée en chlorure de sodium. Il est séché sur sulfate de sodium anhydre puis et les solvants sont évaporés sous pression réduite. Le résidu brut est purifié sur une colonne de gel de silice (éluant : cyclohexane/acétate d'éthyle 9/1 (v/v)). On obtient 690 mg de produit gommeux. Rendement 44 %.
$^1$H RMN ($CDCl_3$, δ ppm) : 0,08-0,18(m, 2H, c-Pr); 0,37-0,41(m, 2H, c-Pr); 0,67-0,75(m, 3H, $CH_3$ et 1H, c-Pr); 1,30-1,45 et 1,50-1,65(2m, 2H, $CH_2$); 1,84-2,03(m, 2H, $CH_2$ -c-Pr); 2,21 (s, 3H, $CH_3$); 2,94-3,02 et 3,10-3,18(2m, 2H, $NCH_2$); 5,43(t, 7,5Hz, 1H, CH); 6,93-7,0(m, 2H, Ph); 7,36-7,41(m, 2H, Ph); 7,71(s, 2H, Ph)
Le composé est salifié sous forme de chlorhydrate ; F = 135°C (HCl).

**PREPARATION PAR LA VOIE B2**

1) Préparation des *N*-acyl-thiourées de formule X

**• *N*-acétyl-*N'*-[2-cyclopropyl-1-(4-fluorophényl)éthyl]thiourée *Composé X.1***

Une solution de 3,38 g (44,4 mmol) de thiocyanate d'ammonium dans 44 ml d'acétone est agitée à température ambiante et additionnée de 3,07 ml (40,7 mmol) de chlorure d'acétyle. Après agitation pendant cinq minutes, 88 ml de benzène sont ajoutés et le mélange réactionnel est chauffé à 60°C. Une solution de 6,62 g (37 mmol) de *composé . XI* 2 dans 27 ml de benzène est alors additionnée. La température est maintenue à 60°C pendant cinq minutes puis le mélange est refroidi à température ambiante et dilué par 100 ml d'acétate d'éthyle. Le mélange est lavé par de l'eau puis de l'eau saturée en chlorure de sodium, séché sur sulfate de sodium et les solvants sont évaporés sous pression réduite. Le résidu brut (11 g) est purifié par chromatographie sur une colonne de gel de silice (éluant : cyclohexane/ acétate d'éthyle 4/1 (v/v)). On obtient 5,6 g de *composé X 1*. Rendement 54 %.
$^1$H RMN ($CDCl_3$, δ ppm) : 0,10-0,25(m, 2H, c-Pr); 0,45-0,60(m, 2H, c-Pr); 0,60-0,75(m, 1H, c-Pr); 1,75-1,85(m, 2H, $CH_2$-c-Pr); 2,11(s, 3H, $CH_3CO$); 5,38-5,49(m, 1H, CH); 6;95-7,06(m, 2H, Ph); 7,22-7,32(m, 2H, Ph); 8,73(s, 1H, NH); 11,0(d, 1H, NH).
Par la même méthode sont synthétisés :

**•*N*-cyclopropylcarbonyl-*N'*-[2-cyclopropyl-1-(4-fluorophényl)éthyt]thiourée *Composé X.2***

$^1$H RMN ($CDCl_3$, δ ppm) : 0,05-0,15(m, 2H, c-Pr) ; 0,41-0,50(m, 2H, c-Pr) ; 0,50-0,70(m, 1H, c-Pr) ; 1,0-1,1 (m, 2H, c-Pr) ; 1,1-1,2(m, 2H, c-Pr) ; 1,4-1,65(m, 1H, c-Pr) ; 1,75-1,85(m, 2H, $CH_2$-c-Pr) ; 5,40-5,50(m, 1H, CH) ; 6,95-7,07(m, 2H, Ph) ; 7,23-7,31 (m, 2H, Ph) ; 8,89(s, 1H, NH) ; 11,05(d, 1H, NH)

**• *N*-acétyl-*N'*-(2-méthoxy-5-méthylphényl)thiourée *Composé X.3***

F = 152°C
Par la même méthode ou en remplaçant le benzène par du chloroforme sont également synthétisés les composés du TABLEAU 2 suivant :

**TABLEAU 2 : *N*-acyl-thiourées de formule X**

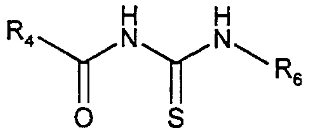

(X)

| *Composés* N° | $R_4$ | $R_6$ | $^1H$ RMN ($CDCl_3$, δ ppm) |
|---|---|---|---|
| *X.4* | 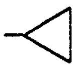 | 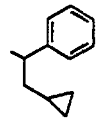 | 0,04-0,14(m, 2H, c-Pr); 0,39-0,45(m, 2H, c-Pr); 0,48-0,68(m, 1H, c-Pr); 0,90-1,08(m, 2H, c-Pr); 1,08-1,15(m, 2H, c-Pr); 1,40-1,55(m, 1H, c-Pr); 1,75-1,85(m, 2H, $CH_2$-c-Pr); 5,41-5,52(m, 1H, CH); 7,15-7,40(m, 5H, Ph); 8,98(s, 1H, NH); 11,08(d, 1H, NH). |

| *Composés* N° | $R_4$ | $R_6$ | $^1H$ RMN ($CDCl_3$, δ ppm) |
|---|---|---|---|
| *X.5* | 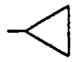 | 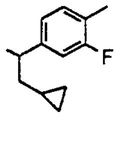 | 0,04-0,15(m, 2H, c-Pr); 0,39-0,50(m, 2H, c-Pr); 0,50-0,68(m, 1H, c-Pr); 0,90-1,02(m, 2H, c-Pr); 1,03-1,18(m, 2H, c-Pr); 1,40-1,52(m, 1H, c-Pr); 1,71-1,81(m, 2H, $CH_2$-c-Pr); 2,22(d, J=1,8Hz, 3H, $CH_3$); 5,34-5,49(m, 1H, CH); 6,90-7,00(m, 2H, Ph); 7,08-7,20(m, 1H, Ph); 9,00(s, 1H, NH); 11,03(d, 1H, NH). |
| *X.6* | 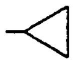 | 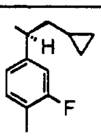 | 0,04-0,15(m, 2H, c-Pr); 0,39-0,50(m, 2H, c-Pr); 0,50-0,70(m, 1H, c-Pr); 0,90-1,03(m, 2H, c-Pr); 1,03-1,18(m, 2H, c-Pr); 1,40-1,52(m, 1H, c-Pr); 1,70-1,81(m, 2H, $CH_2$-c-Pr); 2,22(d, J=1,8Hz, 3H, $CH_3$); 5,35-5,47(m, 1H, CH); 6,90-7,00(m, 2H, Ph); 7,08-7,20(m, 1H, Ph); 8,97(s, 1H, NH); 11,03(d, 1H, NH). |
| *X.7* | 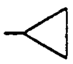 | 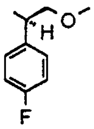 | 0,88-1,02(m, 2H, c-Pr); 1,08-1,18(m, 2H, c-Pr); 1,40-1,52(m, 1H, c-Pr); 3,35(s, 3H, $OCH_3$);3,62-3,75(m, 2H, $CH_2O$); 5,50-5,60(m, 1H, CH); 6,94-7,06(m, 2H, Ph); 7,26-7,35(m, 2H, Ph); 8,99(s, 1H, NH); 11,16(d, 1H, NH). |
| *X.8* | $-CH_2CH_3$ | 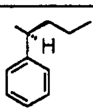 | 0,88-0,95(m, 3H, $CH_3$); 1,11-1,20(m, 3H, $CH_3$); 1,20-1,41(m, 2H, $CH_2$); 1,72-2,00(m, 2H, $CH_2$); 2,30-2,38(m, 2H, $CH_2$); 5,34-5,46(m, 1H, CH); 7,19-7,37(m, 5H, Ph); 8,73(s, 1H, NH); 10,95(d, 1H, NH). |
| *X.9* | $-CH_3$ | 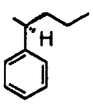 | 0,88-0,95(m, 3H, $CH_3$); 1,22-1,40(m, 2H, $CH_2$); 1,71-2,02(m, 2H, $CH_2$); 2,10(s, 3H, $CH_3$); 5,33-5,45(m, 1H, CH); 7,19-7,37(m, 5H, Ph); 8,89(s, 1H, NH); 10,92(d, 1H, NH). |
| *X.10* | $-CH_3$ | 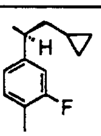 | 0,07-0,15(m, 2H, c-Pr); 0,39-0,49(m, 2H, c-Pr); 0,50-0,68(m, 1H, c-Pr); 1,74-1,85(m, 2H, $CH_2$-c-Pr); 2,10(s, 3H, $CH_3$); 2,22(d, J=1,8Hz, 3H, $CH_3$); 5,36-5,46(m, 1H, CH); 6,90-7,00(m, 2H, Ph); 7,08-7,16(m, 1H, Ph); 8,69(s, 1H, NH); 10,96(d, 1H, NH). |
| *X.11* | $-CH_3$ | 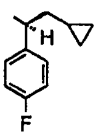 | 0,02-0,17(m, 2H, c-Pr); 0,40-0,47(m, 2H, c-Pr); 0,48-0,65(m, 1H, c-Pr); 1,74-1,82(m, 2H, $CH_2$-c-Pr); 2,10(s, 3H, $CH_3$); 5,38-5,49(m, 1H, CH); 6,94-7,06(m, 2H, Ph); 7,21-7,31(m, 2H, Ph); 8,72(s, 1H, NH); 10,98(d, 1H, NH). |
| *X.12* | $-CH_2CH_3$ | 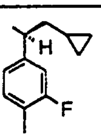 | 0,07-0,17(m, 2H, c-Pr); 0,39-0,49(m, 2H, c-Pr); 0,50-0,68(m, 1H, c-Pr); 1,12-1,23(m,3H, $CH_3$); 1,74-1,82(m, 2H, $CH_2$-c-Pr); 2,22(d, J=1,8Hz, 3H, $CH_3$); 2,27-2,39(m,2H, $CH_2$); 5,36-5,46(m, 1H, CH); 6,91-7,00(m, 2H, Ph); 7,08-7,17(m, 1H, Ph); 8,52(s, 1H, NH); 10,97(d, 1H, NH). |
| *X.13* | $-CH_2CH_3$ | 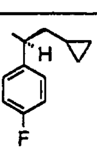 | 0,03-0,17(m, 2H, c-Pr); 0,41-0,49(m, 2H, c-Pr); 0,50-0,68(m, 1H, c-Pr); 1,12-1,22(m,3H, $CH_3$); 1,74-1,82(m, 2H, $CH_2$-c-Pr); 2,27-2,39(m,2H, $CH_2$); 5,39-5,49(m, 1H, CH); 6,95-7,06(m, 2H, Ph); 7,22-7,32(m, 2H, Ph); 8,77(s, 1H, NH); 10,98(d, 1H, NH). |

| *Composés* N° | $R_4$ | $R_6$ | $^1H$ RMN ($CDCl_3$, δ ppm) |
|---|---|---|---|
| *X.14* | 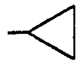 | 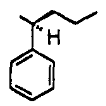 | 0,87-1,02(m, 2H, c-Pr et 3H, $CH_3$); 1,02-1,15(m, 2H, c-Pr); 1,18-1,52(m, 1H, c-Pr et 2H, $CH_2$); 1,71-2,02(m, 2H, $CH_2$); 5,33-5,44(m, 1H, CH); 7,19-7,37(m, 5H, Ph); 9,05(s, 1H, NH); 10,97(d, 1H, NH) . |
| *X.15* | $-CH_3$ | 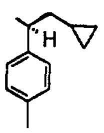 | 0,04-0,17(m, 2H, c-Pr); 0,35-0,47(m, 2H, c-Pr); 0,48-0,65(m, 1H, c-Pr); 1,75-1,82(m, 2H, $CH_2$-c-Pr); 2,09(s, 3H, $CH_3$); 2,31(s, 3H, $CH_3$); 5,38-5,49(m, 1H, CH); 7,10-7,23(m, 4H, Ph); 8,69(s, 1H, NH); 10,95(d, 1H, NH). |
| *X.16* | $-CH_3$ | 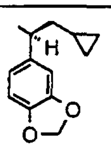 | 0,06-0,17(m, 2H, c-Pr); 0,40-0,50(m, 2H, c-Pr); 0,52-0,64(m, 1H, c-Pr); 1,75-1,82(m, 2H, $CH_2$-c-Pr); 2,14(s, 3H, $CH_3$); 5,38-5,45(m, 1H, CH); 5,97(s, 2H, $OCH_2O$); 6,77-6,83(m, 3H, Ph); 8,61(s, 1H, NH); 10,93(d, 1H, NH). |
| *X.17* | $-CH_3$ | 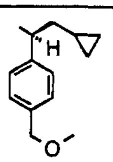 | 0,07-0,20(m, 2H, c-Pr); 0,43-0,50(m, 2H, c-Pr); 0,58-0,68(m, 1H, c-Pr); 1,80-1,90(m, 2H, $CH_2$-c-Pr); 2,14(s, 3H, $CH_3$); 3,40(s, 3H, $OCH_3$); 4,45(s, 2H, $CH_2$-O); 5,47-5,55(m, 1H, CH); 7,28-7,35(m, 4H, Ph); 8,71(s, 1H, NH); 11,03(d, 1H, NH). |
| *X.18* | $-CH_3$ | 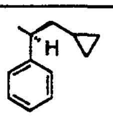 | 0,07-0,20(m, 2H, c-Pr); 0,43-0,50(m, 2H, c-Pr); 0,58-0,70(m, 1H, c-Pr); 1,80-1,90(m, 2H, $CH_2$-c-Pr); 2,14(s, 3H, $CH_3$); 5,48-5,56(m, 1H, CH); 7,26-7,40(m, 5H, Ph); 8,67(s, 1H, NH); 11,04(d, 1H, NH). |
| *X.19* | 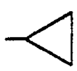 | 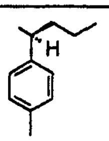 | 0,90-1,03(m, 2H, c-Pr et 3H, $CH_3$); 1,03-1,18(m, 2H, c-Pr); 1,25-1,50(m, 1H, c-Pr et 2H, $CH_2$); 1,75-2,0 (m, 2H, $CH_2$); 2,34(s, 3H, $CH_3$); 5,34-5,43(m, 1H, CH); 7,14-7,22(m, 4H, Ph); 8,79(s, 1H, NH); 10,92(d, 1H, NH). |

2) <u>Préparation des *N*-acyl-*S*-méthyl-isothiourées de formule IX</u>

**Première méthode**

• ***N*-acétyl-*N'*-[2-cyclopropyl-1-(4-fluorophényl)éthyl]-*S*-méthyl-isothiourée *Composé IX.1***

Une solution de 2,8 g (10 mmol) de *composé X.1* dans 50 ml de tétrahydrofurane est agitée à 0°C et additionnée par portions de 440 mg (11 mmol) d'hydrure de sodium à 60 % dans l'huile. Après agitation pendant vingt minutes à 0°C, 0,75 ml (12 mmol) d'iodure de méthyle sont ajoutés. Le mélange réactionnel est agité pendant quatre heures à température ambiante puis est refroidi à 0°C et additionné lentement de 5 ml d'éthanol puis 5 ml d'eau. Il est extrait par 150 ml d'acétate d'éthyle et la phase organique est lavée à l'eau puis à l'eau saturée en chlorure de sodium, séchée sur sulfate de sodium et les solvants sont évaporés sous pression réduite. Le résidu brut (3,5 g) est utilisé tel quel dans l'étape suivante (rendement quantitatif).
$^1H$ RMN ($CDCl_3$, δ ppm) : 0,05-0,15(m, 2H, c-Pr); 0,43-0,55(m, 2H, c-Pr); 0,55-0,75(m, 1H, c-Pr); 1,70-1,85(m, 2H, $CH_2$-c-Pr); 2,15(s, 3H, $CH_3CO$); 2,42(s, 3H, $SCH_3$); 4,62-4,71(m, 1H, CH); 6,92-7,06(m, 2H, Ph); 7,19-7,33(m, 2H, Ph); 11,57(s, 1H, NH).

Par la même méthode sont obtenus les produits du TABLEAU 3.

## TABLEAU 3: *N*-acyl-*S*-méthyl-isothiourées de formule IX

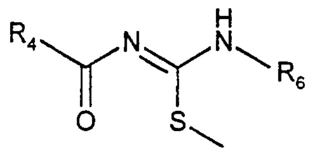

(IX)

| Composés N° | $R_4$ | $R_6$ | $^1H$ RMN ($CDCl_3$, δ ppm) |
|---|---|---|---|
| *IX.2* | 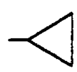 | 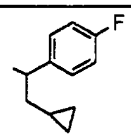 | 0,05-0,11(m, 2H, c-Pr); 0,43-0,55(m, 2H, c-Pr); 0,55-0,72(m, 1H, c-Pr); 0,78-0,92(m, 2H, c-Pr); 1,05-1,10(m, 2H, c-Pr); 1,55-1,65(m, 1H, c-Pr); 1,70-1,80(m, 2H, $CH_2$-c-Pr); 2,42(s, 3H, $SCH_3$); 4,63-4,73(m, 1H, CH); 6,95-7,05(m, 2H, Ph); 7,18-7,31(m, 2H, Ph); 11,49(1H, NH) |
| *IX.3* | $-CH_3$ | 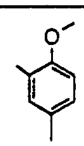 | 2,24 et 2,27(2s, 6H, $CH_3$ et $CH_3CO$); 2,40(s, 3H, $SCH_3$); 3,79(s, 3H, $OCH_3$); 6,80(d, J=8,3Hz, 1H, Ph); 7,02(d, J=8,3Hz, 1H, Ph); 7,18(s, 1H, Ph); 12,1(s, 1H, NH) |
| *IX.4* | 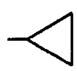 | 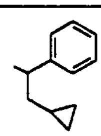 | 0,02-0,18(m, 2H, c-Pr); 0,38-0,50(m, 2H, c-Pr); 0,50-0,70(m, 1H, c-Pr); 0,75-0,95(m, 2H, c-Pr); 1,00-1,13(m, 2H, c-Pr); 1,50-1,65(m, 1H, c-Pr); 1,70-1,82(m, 2H, $CH_2$-c-Pr); 2,38(s, 3H, $SCH_3$); 4,62-4,75(m, 1H, CH); 7,15-7,40(m, 5H, Ph); 11,53(s, 1H, NH). |
| *IX.5* | 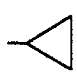 | 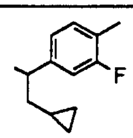 | 0,04-0,12(m, 2H, c-Pr); 0,40-0,50(m, 2H, c-Pr); 0,50-0,70(m, 1H, c-Pr); 0,78-0,90(m, 2H, c-Pr); 1,02-1,10(m, 2H, c-Pr); 1,52-1,82(m, 1H, c-Pr et 2H, $CH_2$-c-Pr); 2,23(d, J=1,8Hz, 3H, $CH_3$); 2,38(s, 3H, $SCH_3$) 4,60-4,70(m, 1H, CH); 6,86-6,97(m, 2H, Ph); 7,08-7,20(m, 1H, Ph); 11,46(s, 1H, NH). |
| *IX.6* | 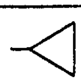 | 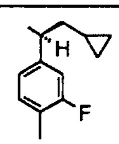 | 0,04-0,12(m, 2H, c-Pr); 0,40-0,50(m, 2H, c-Pr); 0,50-0,70(m, 1H, c-Pr); 0,78-0,90(m, 2H, c-Pr); 1,00-1,09(m, 2H, c-Pr); 1,52-1,82(m, 1H, c-Pr et 2H, $CH_2$-c-Pr); 2,23(d, J=1,8Hz, 3H, $CH_3$); 2,38(s, 3H, $SCH_3$); 4,60-4,70(m, 1H, CH); 6,86-6,97(m, 2H, Ph); 7,08-7,20(m, 1H, Ph); 11,47(s, 1H, NH). |

| *Composés* N° | $R_4$ | $R_6$ | $^1H$ RMN ($CDCl_3$, δ ppm) |
|---|---|---|---|
| *IX.7* | 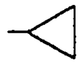 | 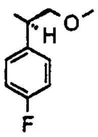 | 0,78-0,90(m, 2H, c-Pr); 1,03-1,11(m, 2H, c-Pr); 1,58-1,72(m, 1H, c-Pr); 2,37(s, 3H, $SCH_3$); 3,35(s, 3H, $OCH_3$); 3,52-3,65(m, 2H, $CH_2O$); 4,74-4,85(m, 1H, CH); 6,95-7,10(m, 2H, Ph); 7,15-7,30(m, 2H, Ph); 11,50(s, 1H, NH). |

**Deuxième méthode**

• ***S*-méthyl-*N*-[(1*S*)-1-phénylbutyl]-*N'*-propionyl-isothiourée *Composé IX.8***

Une solution de 6,4 g (24,2 mmol) de *composé X.8* dans 120 ml de diméthylformamide est agitée à température ambiante et additionnée de 7,9 g (24.2 mmol) de carbonate de césium. Ensuite 3 ml (24 mmol) de iodométhane sont additionnés lentement. Le mélange est agité à température ambiante pendant deux heures puis est additionné de glace et extrait par l'acétate d'éthyle. La phase organique est lavée à l'eau puis à l'eau saturée en chlorure de sodium, séchée sur sulfate de sodium et les solvants sont évaporés sous pression réduite. Le résidu brut est purifié par chromatographie sur une colonne de gel de silice (éluant : cyclohexane/acétate d'éthyle 9/1 (v/v)). On obtient 5,15 g de *composé IX.8*. Rendement 72 %.
$^1H$ RMN ($CDCl_3$, δ ppm): 0,86-0,94(m, 3H, $CH_3$); 1,09-1,17(m, 3H, $CH_3$); 1,22-1,45(m, 2H, $CH_2$); 1,72-1,86(m, 2H, $CH_2$); 2,38(s, 3H, $SCH_3$); 2,35-2,50(m, 2H, $CH_2$); 4,57-4,63(m, 1H, CH); 7,19-7,37(m, 5H, Ph); 11,52(s, 1H, NH).
Par la même méthode sont obtenus les produits du TABLEAU 4.

## TABLEAU 4: *N*-acyl-*S*-méthyl-isothiourées de formule IX

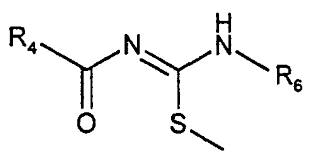

(IX)

| *Composés* N° | $R_4$ | $R_6$ | $^1H$ RMN ($CDCl_3$, δ ppm) |
|---|---|---|---|
| *IX.9* | $-CH_3$ | 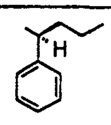 | 0,85-0,95(m, 3H, $CH_3$); 1,20-1,48(m, 2H, $CH_2$); 1,65-1,88(m, 2H, $CH_2$); 2,15(s, 3H, $CH_3$); 2,38(s, 3H, $SCH_3$); 4,55-4,65(m, 1H, CH); 7,18-7,37(m, 5H, Ph); 11,53(s, 1H, NH). |
| *IX.10* | $-CH_3$ | 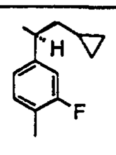 | 0,07-0,15(m, 2H, c-Pr); 0,42-0,51(m, 2H, c-Pr); 0,52-0,68(m, 1H, c-Pr); 1,70-1,90(m, 2H, $CH_2$-c-Pr); 2,16(s, 3H, $CH_3$); 2,23(d, J=1,8Hz, 3H, $CH_3$); 2,39(s, 3H, $SCH_3$); 4,62-4,70(m, 1H, CH); 6,86-6,95(m, 2H, Ph); 7,08-7,16(m, 1H, Ph); 11,56(s, 1H, NH). |

| *Composés* N° | $R_4$ | $R_6$ | $^1H$ RMN ($CDCl_3$, δ ppm) |
|---|---|---|---|
| *IX.11* | $-CH_3$ | 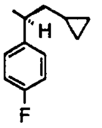 | 0,08-0,20(m, 2H, c-Pr); 0,42-0,55(m, 2H, c-Pr); 0,56-0,72(m, 1H, c-Pr); 1,68-1,88(m, 2H, $CH_2$-c-Pr); 2,20(s, 3H, $CH_3$); 2,48(s, 3H, $SCH_3$); 4,74-4,80(m, 1H, CH); 7,03-7,12(m, 2H, Ph); 7,25-7,31(m, 2H, Ph); 11,67(s, 1H, NH). |
| *IX.12* | $-CH_2CH_3$ | 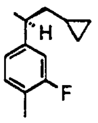 | 0,05-0,15(m, 2H, c-Pr); 0,40-0,50(m, 2H, c-Pr); 0,52-0,70(m, 1H, c-Pr); 1,10-1,20(m, 3H, $CH_3$); 1,60-1,80(m, 2H, $CH_2$-c-Pr); 2,22(d, J=1,8Hz, 3H, $CH_3$); 2,38(s, 3H, $SCH_3$); 2,35-2,50(m, 2H, $CH_2$); 4,60-4,70(m, 1H, CH); 6,87-6,95(m, 2H, Ph); 7,08-7,17(m, 1H, Ph); 11,52(s, 1H, NH). |
| *IX.13* | $-CH_2CH_3$ | 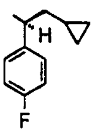 | 0,05-0,15(m, 2H, c-Pr); 0,40-0,50(m, 2H, c-Pr); 0,50-0,70(m, 1H, c-Pr); 1,10-1,20(m,3H, $CH_3$); 1,60-1,82(m, 2H, $CH_2$-c-Pr); 2,38(s, 3H, $SCH_3$); 2,35-2,53(m,2H, $CH_2$); 4,65-4,72(m, 1H, CH); 6,94-7,06(m, 2H, Ph); 7,17-7,27(m, 2H, Ph); 11,50(s, 1H, NH). |
| *IX.14* | 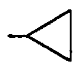 | 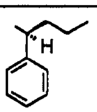 | 0,78-0,98(m, 2H, c-Pr et 3H, $CH_3$); 1,00-1,08(m, 2H, c-Pr); 1,25-1,45(m, 2H, $CH_2$); 1,65-1,88(m, 1H, c-Pr et 2H, $CH_2$); 2,37(s, 3H, $SCH_3$); 4,55-4,65(m, 1H, CH); 7,18-7,36(m, 5H, Ph); 11,45(s, 1H, NH). |
| *IX.15* | $-CH_3$ | 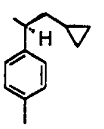 | 0,05-0,15(m, 2H, c-Pr); 0,40-0,52(m, 2H, c-Pr); 0,53-0,70(m, 1H, c-Pr); 1,60-1,82(m, 2H, $CH_2$-c-Pr); 2,15(s, 3H, $CH_3$); 2,31(s, 3H, $CH_3$); 2,37(s, 3H, $SCH_3$); 4,60-4,70(m, 1H, CH); 7,08-7,23(m, 4H, Ph); 11,59(s, 1H, NH). |
| *IX.16* | $-CH_3$ | 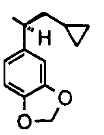 | 0,06-0,17(m, 2H, c-Pr); 0,42-0,55(m, 2H, c-Pr); 0,56-0,70(m, 1H, c-Pr); 1,65-1,80(m, 2H, $CH_2$-c-Pr); 2,19(s, 3H, $CH_3$); 2,43(s, 3H, $SCH_3$); 4,60-4,70(m, 1H, CH); 5,97(s, 2H, $OCH_2O$); 6,73-6,81(m, 3H, Ph); 11,58(s, 1H, NH). |
| *IX.17* | $-CH_3$ | 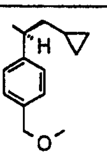 | 0,10-0,18(m, 2H, c-Pr); 0,45-0,52(m, 2H, c-Pr); 0,60-0,70(m, 1H, c-Pr); 1,70-1,85(m, 2H, $CH_2$-c-Pr); 2,20(s, 3H, $CH_3$); 2,42(s, 3H, $SCH_3$); 3,40(s, 3H, $OCH_3$); 4,45(s, 2H, $CH_2$-O); 4,70-4,80(m, 1H, CH); 7,25-7,35(m, 4H, Ph); 11,65(s, 1H, NH). |
| *IX.18* | $-CH_3$ | 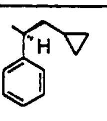 | 0,08-0,20(m, 2H, c-Pr); 0,43-0,55(m, 2H, c-Pr); 0,60-0,72(m, 1H, c-Pr); 1,63-1,88(m, 2H, $CH_2$-c-Pr); 2,20(s, 3H, $CH_3$); 2,43(s, 3H, $SCH_3$); 4,66-4,77(m, 1H, CH); 7,28-7,40(m, 5H, Ph); 11,66(s, 1H, NH). |
| *IX.19* | 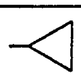 | 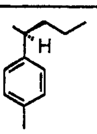 | 0,80-0,87(m, 2H, c-Pr); 0,87-0,97(m, 3H, $CH_3$); 1,02-1,10(m, 2H, c-Pr); 1,25-1,48(m, 1H, c-Pr et 2H, $CH_2$); 1,70-1,85(m, 2H, $CH_2$); 2,33(s, 3H, $CH_3$); 2,40(s, 3H, $SCH_3$); 4,52-4,62(m, 1H, CH); 7,10-7,20(m, 4H, Ph); 11,45(s, 1H, NH). |

### 3) Préparation des aminotriazoles NH de formule VIII

**•1-(2-Chloro-4-méthoxy-5-méthylphényl)-*N*-[2-cyclopropyl-1-(4-fluorophényl)éthyl]-5-méthyl-1*H*-1,2,4-triazol-3-amine *Composé VIII.1***

Une solution de 3,2 g (10 mmol) de *composé IX.1* dans 25 ml de toluène est additionnée de 3 g (11,5 mmol) de chlorhydrate de 2-chloro-4-méthoxy-5-méthylphénylhydrazine (*composé III.1*), puis de 3,5 ml (25 mmol) de triéthylamine et de tamis moléculaire 4Å. Le mélange réactionnel est agité une nuit à reflux modéré, puis est refroidi. Le tamis moléculaire est éliminé par filtration et le filtrat est dilué par 100 ml d'acétate d'éthyle. La phase organique est lavée par l'acide chlorhydrique 1 N, par de l'eau, par une solution aqueuse saturée en hydrogénocarbonate de sodium, par de l'eau, puis de l'eau saturée en chlorure de sodium. Elle est séchée sur sulfate de sodium puis les solvants sont évaporés sous pression réduite. Le résidu brut est purifié par chromatographie sur une colonne de gel de silice (éluant cyclohexane/acétate d'éthyle 3/1 puis 2/1 puis 1/1 (v/v)). On obtient 1,35 g de *composé VIII.1*. Rendement 32 %.
$^1$H RMN ($CDCl_3$, δ ppm) : 0,02-0,12(m, 2H, c-Pr); 0,35-0,45(m, 2H, c-Pr); 0,55-0,70(m, 1H, c-Pr); 1,60-1,82(m, 2H, $CH_2$-c-Pr); 2,17(s, 6H, $2CH_3$); 3,83(s, 3H, $OCH_3$); 4,60(d, J=8,2Hz, 1H, NH); 4,73-4,84(m, 1H, CH); 6,85(s, 1H, Ph); 6,90-7,00(m, 2H, Ph); 7,05(s, 1H, Ph); 7,25-7,37(m, 2H, Ph).

Les *composés VIII* du TABLEAU 5 ont été préparés par la même méthode. Le toluène peut être remplacé par le xylène, le diméthylformamide ou le diméthylsulfoxyde. Lorsque une base est nécessaire, la triéthylamine peut être remplacée par la *N*,*N*-diéthylaniline ou le carbonate de césium.

**TABLEAU 5 : aminotriazoles NH de formule VIII**

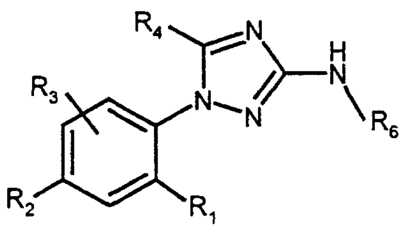

(VIII)

| *Composés* N° | $R_1$,$R_2$,$R_3$ | $R_4$ | $R_6$ | $^1$H RMN ($CDCl_3$, δ ppm) |
|---|---|---|---|---|
| *VIII.2* | 2-Cl<br>4-$OCH_3$<br>5-$CH_3$ | 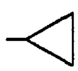 | 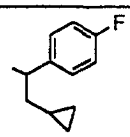 | 0,0-0,10(m, 2H, c-Pr); 0,35-0,45(m, 2H, c-Pr); 0,52-0,70(m, 1H, c-Pr); 0,83-0,97(m, 2H, c-Pr); 1,0-1,05(m, 2H, c-Pr); 1,40-1,52(m, 1H, c-Pr); 1,60-1,85(m, 2H, $CH_2$-c-Pr); 2,15(s, 3H, $CH_3$); 3,83(s, 3H, $OCH_3$); 4,49(d, J=8,2Hz, 1H, NH); 4,68-4,82(m, 1H, CH); 6,87(s, 1H, Ph); 6,90-6,99(m, 2H, Ph); 7,11(s, 1H, Ph); 7,20-7,34(m, 2H, Ph). |

| *Composés* N° | $R_1,R_2,R_3$ | $R_4$ | $R_6$ | $^1H$ RMN ($CDCl_3$, δ ppm) |
|---|---|---|---|---|
| *VIII.3* | 2-Cl<br>4-Cl<br>H | $-CH_3$ | 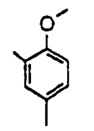 | 2,28(s, 6H, $2CH_3$); 3,83(s, 3H, $OCH_3$); 6,62(dd, $J_1$=8,1Hz, $J_2$=1,5Hz, 1H, Ph); 6,73(d, J=8,1Hz, 1H, Ph); 7,18(s, 1H, NH); 7,37-7,41(m, 2H, Ph); 7,57(d, J=1,8Hz, 1H, Ph); 7,89(d, J=1,9Hz, 1H, Ph). |
| *VIII.4* | 2-Cl<br>$4-CF_3$<br>6-Cl | 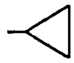 | 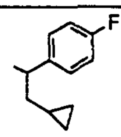 | 0,02-0,10(m, 2H, c-Pr); 0,35-0,45(m, 2H, c-Pr); 0,52-0,70(m, 1H, c-Pr); 0,83-1,04(m, 2H, c-Pr); 1,05-1,20(m, 2H, c-Pr); 1,25-1,42(m, 1H, c-Pr); 1,60-1,85(m, 2H, $CH_2$-c-Pr); 4,65-4,82(m, 2H, CH et NH); 6,90-7,01(m, 2H, Ph); 7,25-7,45(m, 2H, Ph); 7,63-7,70(m, 2H, Ph) |
| *VIII.5* | 2-Cl<br>4-Cl<br>H | 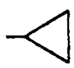 | 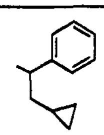 | 0,02-0,12(m, 2H, c-Pr); 0,35-0,47(m, 2H c-Pr); 0,52-0,70(m, 1H, c-Pr); 0,80-0,98(m, 2H, c-Pr); 1,0-1,10(m, 2H, c-Pr); 1,38-1,50(m, 1H, c-Pr); 1,60-1,85(m, 2H, $CH_2$-c-Pr); 4,58(d, J=8,2Hz, 1H, NH); 4,69-4,81(m, 1H, CH); 7,10-7,40(m, 7H, Ph); 7,50(s, 1H, Ph). |
| *VIII.6* | 2-Cl<br>$4-OCH_3$<br>$5-CH_3$ | 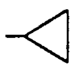 | 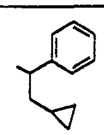 | 0,0-0,10(m, 2H, c-Pr); 0,32-0,42(m, 2H, c-Pr); 0,52-0,70(m, 1H, c-Pr); 0,83-0,97(m, 2H, c-Pr); 1,0-1,10(m, 2H, c-Pr); 1,40-1,54(m, 1H, c-Pr); 1,60-1,87(m, 2H, $CH_2$-c-Pr); 2,16(s, 3H, $CH_3$); 3,83(s, 3H, $OCH_3$); 4,55(d, J=8,5Hz, 1H, NH); 4,73-4,84(m, 1H, CH); 6,87(s, 1H, Ph); 7,13-7,38(m, 6H, Ph). |
| *VIII.7* | 2-Cl<br>$4-OCH_3$<br>$5-CH_3$ | 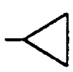 | 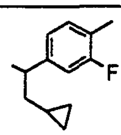 | 0,02-0,10(m, 2H, c-Pr); 0,35-0,45(m, 2H, c-Pr); 0,52-0,70(m, 1H, c-Pr); 0,85-0,97(m, 2H, c-Pr); 0,99-1,07(m, 2H, c-Pr); 1,40-1,52(m, 1H, c-Pr); 1,60-1,85(m, 2H, $CH_2$-c-Pr); 2,16(s, 3H, $CH_3$); 2,21(d, J=1,8 Hz, 3H, $CH_3$); 3,83(s, 3H, $OCH_3$); 4,48(d, J=8,2Hz, 1H, NH); 4,68-4,80(m, 1H, CH); 6,87(s, 1H, Ph); 6,96-7,15(m, 4H, Ph). |
| *VIII.8* | 2-Cl<br>$4-OCH_3$<br>$5-CH_3$ | 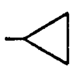 | 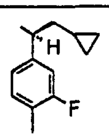 | 0,02-0,10(m, 2H, c-Pr); 0,35-0,45(m, 2H, c-Pr); 0,53-0,70(m, 1H, c-Pr); 0,85-0,97(m, 2H, c-Pr); 1,0-1,08(m, 2H, c-Pr); 1,40-1,52(m, 1H, c-Pr); 1,60-1,85(m, 2H, $CH_2$-c-Pr); 2,16(s, 3H, $CH_3$); 2,21(d, J=1,8 Hz, 3H, $CH_3$); 3,83(s, 3H, $OCH_3$); 4,49(d, J=8,2Hz, 1H, NH); 4,68-4,80(m, 1H, CH); 6,87(s, 1H, Ph); 6,97-7,15(m, 4H, Ph). |
| *VIII.9* | 2-Cl<br>$4-OCH_3$<br>$5-CH_3$ | 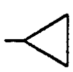 | 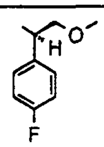 | 0,83-0,97(m, 2H, c-Pr); 1,0-1,10(m, 2H, c-Pr); 1,40-1,52(m, 1H, c-Pr); 2,15(s, 3H, $CH_3$); 3,32(s, 3H, $OCH_3$); 3,58-3,72(m, 2H, $CH_2O$); 3,83(s, 3H, $OCH_3$); 4,78-4,86(m, 2H, CH et NH); 6,85(s, 1H, Ph); 6,87-7,00(m, 2H, Ph); 7,12(s, 1H, Ph); 7,27-7,40(m, 2H, Ph). |
| *VIII.10* | 2-Cl<br>4-Cl<br>H | 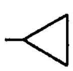 | 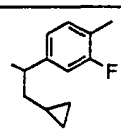 | (DMSO-$D_6$, δ ppm): -0,04-0,10(m, 2H, c-Pr); 0,25-0,40(m, 2H c-Pr); 0,55-0,70(m, 1H, c-Pr); 0,80-0,95(m, 4H, c-Pr); 1,35-1,78(m, 3H, c-Pr et $CH_2$-c-Pr); 2,14(d, J=1,5 Hz, 3H, $CH_3$); 4,39-4,51(m, 1H, CH); 6,57(d, J=9,1Hz, 1H, NH); 7,00-7,15(m, 3H, Ph); 7,55(s, 2H, Ph); 7,85(s, 1H, Ph). |

| Composés N° | $R_1$,$R_2$,$R_3$ | $R_4$ | $R_6$ | $^1H$ RMN ($CDCl_3$, δ ppm) |
|---|---|---|---|---|
| *VIII.11* | 2-Cl<br>4-Cl<br>H | 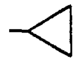 | 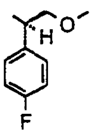 | 0,85-1,02(m, 2H, c-Pr); 1,02-1,12(m, 2H, c-Pr); 1,40-1,52(m, 1H, c-Pr); 3,32(s, 3H, $OCH_3$); 3,53-3,70(m, 2H, $CH_2O$); 4,73-4,82(m, 1H, CH); 4,91(d, J=6,7Hz, 1H, NH); 6,90-7,00(m, 2H, Ph); 7,26-7,40(m, 4H, Ph); 7,50(s, 1H, Ph). |
| *VIII.12* | 2-$CH_3$<br>4-$CH_3$<br>H | 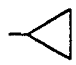 | 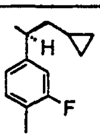 | 0,02-0,10(m, 2H, c-Pr); 0,35-0,45(m, 2H, c-Pr); 0,55-0,70(m, 1H, c-Pr); 0,83-0,97(m, 2H, c-Pr); 1,0-1,10(m, 2H, c-Pr); 1,42-1,55(m, 1H, c-Pr); 1,60-1,80(m, 2H, $CH_2$-c-Pr); 1,94(s, 3H, $CH_3$); 2,20(d, J=1,8 Hz, 3H, $CH_3$); 2,32(s, 3H, $CH_3$); 4,54(d, J=8,2Hz, 1H, NH); 4,64-4,75(m, 1H, CH); 6,97-7,15(m, 6H, Ph). |
| *VIII.13* | 2-Cl<br>4-Cl<br>H | $-CH_2CH_3$ | 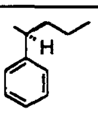 | 0,85-0,95(m, 3H, $CH_3$); 1,11-1,20(m, 3H, $CH_3$); 1,20-1,45(m, 2H, $CH_2$); 1,70-1,95(m, 2H, $CH_2$); 2,35-2,48(m, 2H, $CH_2$); 4,47(d, J=8,5Hz, 1H, NH); 4,60-4,71(m, 1H, CH); 7,14-7,37(m, 7H, Ph); 7,50(s, 1H, Ph). |
| *VIII.14* | 2-Cl<br>4-$OCH_3$<br>5-$CH_3$ | $-CH_3$ | 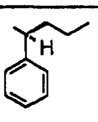 | 0,86-0,96(m, 3H, $CH_3$); 1,22-1,45(m, 2H, $CH_2$); 1,70-1,90(m, 2H, $CH_2$); 2,16(s, 6H, $CH_3$); 3,84(s, 3H, $OCH_3$); 4,39(d, J=8,8Hz, 1H, NH); 4,66-4,77(m, 1H, CH); 6,86(s, 1H, Ph); 7,09(s, 1H, Ph); 7,15-7,38(m, 5H, Ph). |
| *VIII.15* | 2-Cl<br>4-$OCH_3$<br>5-$CH_3$ | $-CH_3$ | 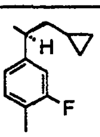 | 0,02-0,10(m, 2H, c-Pr); 0,36-0,46(m, 2H, c-Pr); 0,55-0,72(m, 1H, c-Pr); 1,60-1,85(m, 2H, $CH_2$-c-Pr); 2,15(s, 6H, $CH_3$); 2,21(d, J=1,8 Hz, 3H, $CH_3$); 3,83(s, 3H, $OCH_3$); 4,65-4,85(m, 2H, CH et NH); 6,87(s, 1H, Ph); 6,99-7,12(m, 4H, Ph). |
| *VIII.16* | 2-Cl<br>4-Cl<br>H | $-CH_3$ | 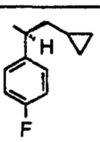 | 0,02-0,13(m, 2H, c-Pr); 0,35-0,46(m, 2H, c-Pr); 0,55-0,70(m, 1H, c-Pr); 1,61-1,85(m, 2H, $CH_2$-c-Pr); 2,17(s, 3H, $CH_3$); 4,68-4,80(m, 2H, CH et NH); 6,90-7,01(m, 2H, Ph); 7,21-7,36(m, 4H, Ph) 7,50(d, J=2Hz, 1H, Ph). |
| *VIII-17* | 2-Cl<br>4-$OCH_3$<br>5-$CH_3$ | $-CH_2CH_3$ | 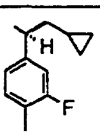 | 0,02-0,12(m, 2H, c-Pr); 0,36-0,46(m, 2H, c-Pr); 0,55-0,72(m, 1H, c-Pr); 1,19(t, J=7,6Hz, 3H, $CH_3$); 1,64-1;89(m, 2H, $CH_2$-c-Pr); 2,15(s, 3H, $CH_3$); 2,21(d, J=1,8 Hz, 3H, $CH_3$); 2,45(q, J=7,6Hz, 2H, $CH_2$); 3,83(s, 3H, $OCH_3$); 4,68-4,88(m, 2H, CH et NH); 6,86(s, 1H, Ph); 6,99-7,12(m, 4H, Ph). |
| *VIII.18* | 2-Cl<br>4-Cl<br>H | $-CH_2CH_3$ | 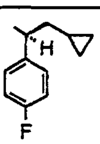 | 0,02-0,12(m, 2H, c-Pr); 0,36-0,52(m, 2H, c-Pr); 0,55-0,72(m, 1H, c-Pr); 1,14-1,24(m, 3H, $CH_3$); 1,60-1,85(m, 2H, $CH_2$-c-Pr); 2,36-2,48(m, 2H, $CH_2$); 4,68-4,80(m, 2H, CH et NH); 6,91-7,02(m, 2H, Ph); 7,20-7,36(m, 4H, Ph); 7,48(d, J=2Hz, 1H, Ph). |
| *VIII.19* | 2-Cl<br>4-Cl<br>H | 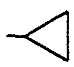 | 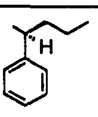 | 0,83-0,97(m, 3H, $CH_3$ et 2H, c-Pr); 1,0-1,10(m, 2H, c-Pr); 1,30-1,52(m, 1H, c-Pr et 2H, $CH_2$); 1,65-1,90(m, 2H, $CH_2$); 4,41(d, J=8,4Hz, 1H, NH); 4,55-4,68(m, 1H, CH); 7,14-7,37(m, 7H, Ph); 7,50(d, J=2Hz, 1H, Ph). |

| *Composés* N° | $R_1$,$R_2$,$R_3$ | $R_4$ | $R_6$ | $^1H$ RMN ($CDCl_3$, δ ppm) |
|---|---|---|---|---|
| *VIII.20* | 2-Cl<br>4-$OCH_3$<br>5-$CH_3$ | -$CH_2CH_3$ | 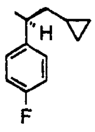 | 0,00-0,12(m, 2H, c-Pr); 0,35-0,50(m, 2H, c-Pr); 0,52-0,70(m, 1H, c-Pr); 1,17(t, J=7,6Hz, 3H, $CH_3$); 1,55-1,90(m, 2H, $CH_2$-c-Pr); 2,15(s, 3H, $CH_3$); 2,41(q, J=7,6Hz, 2H, $CH_2$); 3,83(s, 3H, $OCH_3$); 4,60(d, J=8,4Hz, 1H, NH); 4,66-4,83(m, 1H, CH); 6,86(s, 1H, Ph); 6,90-7,04(m, 2H, Ph); 7,10(s, 1H, Ph); 7,22-7,35(m, 2H, Ph). |
| *VIII.21* | 2-Cl<br>4-Cl<br>H | -$CH_3$ | 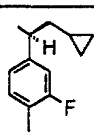 | 0,02-0,12(m, 2H, c-Pr); 0,36-0,46(m, 2H, c-Pr); 0,55-0,70(m, 1H, c-Pr); 1,60-1,85(m, 2H, $CH_2$-c-Pr); 2,15(s, 3H, $CH_3$); 2,21(d, J=1,8 Hz, 3H, $CH_3$); 4,62-4,80(m, 2H, CH et NH); 6,93-7,12(m, 3H, Ph); 7,23-7,36(m, 2H, Ph); 7,50(d, J=2Hz, 1H, Ph). |
| *VIII.22* | 2-Cl<br>4-$OCH_3$<br>5-$CH_3$ | -$CH_3$ | 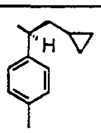 | 0,02-0,10(m, 2H, c-Pr); 0,36-0,44(m, 2H, c-Pr); 0,55-0,70(m, 1H, c-Pr); 1,60-1,90(m, 2H, $CH_2$-c-Pr); 2,15(s, 6H, $CH_3$); 2,30(s, 3H, $CH_3$); 3,83(s, 3H, $OCH_3$); 4,65-4,85(m, 2H, CH et NH); 6,87(s, 1H, Ph); 7,07-7,12(m, 3H, Ph); 7,23-7,27(m, 2H, Ph). |
| *VIII.23* | 2-Cl<br>4-Cl<br>H | -$CH_3$ | 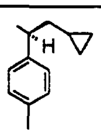 | 0,02-0,10(m, 2H, c-Pr); 0,36-0,48(m, 2H, c-Pr); 0,55-0,72(m, 1H, c-Pr); 1,60-1,88(m, 2H, $CH_2$-c-Pr); 2,15(s, 3H, $CH_3$); 2,30(s, 3H, $CH_3$); 4,60(d, J=8,6Hz, 1H, NH); 4,70-4,85(m, 1H, CH); 7,07-7,12(m, 2H, Ph); 7,23-7,30(m, 4H, Ph); 7,50(d, J=2Hz, 1H, Ph). |
| *VIII.24* | 2-Cl<br>4-$OCH_3$<br>5-$CH_3$ | 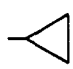 | 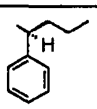 | 0,85-0,97(m, 3H, $CH_3$ et 2H, c-Pr); 1,0-1,08(m, 2H, c-Pr); 1,25-1,53(m, 1H, c-Pr et 2H, $CH_2$); 1,62-1,90(m, 2H, $CH_2$); 2,15 (s, 3H, $CH_3$); 3,83(s, 3H, $OCH_3$); 4,34(d, J=8,6Hz, 1H, NH); 4,60-4,72(m, 1H, CH); 6,87(s, 1H, Ph); 7,13(s, 1H, Ph); 7,17-7,34(m, 5H, Ph). |
| *VIII.25* | 2-Cl<br>4-$OCH_3$<br>5-$CH_3$ | -$CH_3$ | 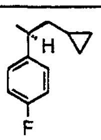 | 0,00-0,10(m, 2H, c-Pr); 0,35-0,46(m, 2H, c-Pr); 0,53-0,70(m, 1H, c-Pr); 1,55-1,80(m, 2H, $CH_2$-c-Pr); 2,14(s, 6H, $CH_3$); 3,83(s, 3H, $OCH_3$); 4,60(d, J=8,4Hz, 1H, NH); 4,73-4,85(m, 1H, CH); 6,85(s, 1H, Ph); 6,90-7,05(m, 2H, Ph); 7,12(s; 1H, Ph); 7,24-7,35(m, 2H, Ph). |
| *VIII.26* | 2-Cl<br>4-$CF_3$<br>H | -$CH_3$ | 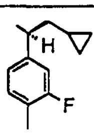 | 0,05-0,15(m, 2H, c-Pr); 0,42-0,48(m, 2H, c-Pr); 0,62-0,75(m, 1H, c-Pr); 1,65-1,85(m, 2H, $CH_2$-c-Pr); 2,26(d, J=1,8 Hz, 3H, $CH_3$); 2,30(s, 3H, $CH_3$); 4,72-4,79(m, 1H, CH); 5,0-5,15(m, 1H, NH); 7,00-7,17(m, 3H, Ph); 7,51(d, J=8,4Hz; 1H, Ph); 7,66(d, J=8,4Hz; 1H, Ph); 7,82(s, 1H, Ph). |
| *VIII.27* | 2-Cl<br>4-$OCH_3$<br>5-$CH_3$ | -$CH_3$ | 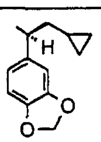 | 0,05-0,15(m, 2H, c-Pr); 0,40-0,48(m, 2H, c-Pr); 0,60-0,72(m, 1H, c-Pr); 1,65-1,87(m, 2H, $CH_2$-c-Pr); 2,19(s, 6H, $CH_3$); 3,80(s, 3H, $OCH_3$); 4,62(d, J=8,4Hz, 1H, NH); 4,72-4,80(m, 1H, CH); 5,94(s, 2H, $OCH_2O$); 6,75(d, J=8,1Hz; 1H, Ph); 6,81-6,90(m, 3H, Ph); 7,12(s, 1H, Ph). |

| *Composés* N° | $R_1,R_2,R_3$ | $R_4$ | $R_6$ | $^1$H RMN ($CDCl_3$, δ ppm) |
|---|---|---|---|---|
| *VIII.28* | 2-Cl<br>4-Cl<br>H | $-CH_3$ | 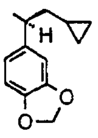 | 0,08-0,16(m, 2H, c-Pr); 0,42-0,50(m, 2H, c-Pr); 0,62-0,77(m, 1H, c-Pr); 1,62-1,87(m, 2H, $CH_2$-c-Pr); 2,23(s, 3H, $CH_3$); 4,62-4,80(m, 2H, CH et NH); 5,95(s, 2H, $OCH_2O$); 6,77(d, J=8Hz; 1H, Ph); 6,86-6,91(m, 2H, Ph); 7,30-7,41(m, 2H, Ph); 7,56(d, J=1,9Hz, 1H, Ph). |
| *VII.29* | 2-Cl<br>4-$OCH_3$<br>5-$CH_3$ | $-CH_3$ | 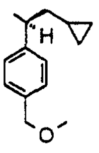 | 0,07-0,15(m, 2H, c-Pr); 0,40-0,48(m, 2H, c-Pr); 0,60-0,75(m, 1H, c-Pr); 1,65-1,95(m, 2H, $CH_2$-c-Pr); 2,20(s, 3H, $CH_3$); 2,21(s, 3H, $CH_3$); 3,41(s, 3H, $OCH_3$); 3,89(s, 3H, $OCH_3$); 4,46(s, 2H, $CH_2O$); 4,82-4,92(m, 2H, CH et NH); 6,91(s, 1H, Ph); 7,12(s, 1H, Ph); 7,28-7,32(m, 2H, Ph) 7,38-7,42(m, 2H, Ph). |
| *VIII.30* | 2-Cl<br>4-$OCH_3$<br>H | $-CH_3$ | 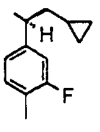 | 0,07-0,14(m, 2H, c-Pr); 0,40-0,50(m, 2H, c-Pr); 0,62-0,77(m, 1H, c-Pr); 1,67-1,90(m, 2H, $CH_2$-c-Pr); 2,21(s, 3H, $CH_3$); 2,27(d, J=2Hz, 3H, $CH_3$); 3,87(s, 3H, $OCH_3$); 4,70(d, J=8,2Hz, 1H, NH); 4,78-4,83(m, 1H, CH); 6,88(dd $J_1$=2,7Hz, $J_2$=8,6Hz, 1H, Ph); 7,05(d, J=2.7Hz, 1H, Ph); 7,07-7,17(m, 3H, Ph); 7,28(d, J=8,6Hz, 1H, Ph). |
| *VIII.31* | 2-Cl<br>4-$OCH_3$<br>5-$CH_3$ | $-CH_3$ | 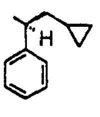 | 0,06-0,13(m, 2H, c-Pr); 0,40-0,48(m, 2H, c-Pr); 0,60-0,72(m, 1H, c-Pr); 1,63-1,90(m, 2H, $CH_2$-c-Pr); 2,19(s, 6H, $CH_3$); 3,87(s, 3H, $OCH_3$); 4,71(d, J=8,3Hz, 1H, NH); 4,82-4,90(m, 1H, CH); 6,89(s, 1H, Ph); 7,10(s, 1H, Ph); 7,22-7,41(m, 5H, Ph). |
| *VIII.32* | 2-Cl<br>4-Cl<br>H | $-CH_3$ | 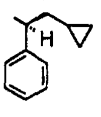 | 0,07-0,17(m, 2H, c-Pr); 0,40-0,50(m, 2H, c-Pr); 0,63-0,78(m, 1H, c-Pr); 1,65-1,95(m, 2H, $CH_2$-c-Pr); 2,22(s, 3H, $CH_3$); 4,69(d, J=8,2Hz, 1H, NH); 4,80-4,90(m, 1H, CH); 7,22-7,42(m, 7H, Ph); 7,55(d, J=2,2Hz, 1H, Ph). |
| *VIII.33* | 2-Cl<br>4-$OCH_3$<br>5-$CH_3$ | 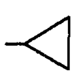 | 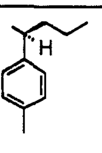 | 0,86-0,97(m, 3H, $CH_3$ et 2H, c-Pr); 1,04-1,12(m, 2H, c-Pr); 1,25-1,53(m, 1H, c-Pr et 2H, $CH_2$); 1,68-1,95(m, 2H, $CH_2$); 2,21 (s, 3H, $CH_3$); 2,35 (s, 3H, $CH_3$); 3,89(s, 3H, $OCH_3$); 4,38(d, J=8,6Hz, 1H, NH); 4,63-4,73(m, 1H, CH); 6,93(s, 1H, Ph); 7,11-7,30(m, 5H, Ph). |
| *VIII.34* | 2-Cl<br>4-Cl<br>H | 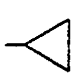 | 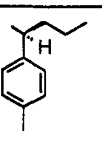 | 0,89-1,0(m, 3H, $CH_3$ et 2H, c-Pr); 1,07-1,13(m, 2H, c-Pr); 1,25-1,53(m, 1H, c-Pr et 2H, $CH_2$); 1,68-1,98(m, 2H, $CH_2$); 2,35 (s, 3H, $CH_3$); 4,42(d, J=8,5Hz, 1H, NH); 4,60-4,69(m, 1H, CH); 7,13(d, J=8Hz, 2H, Ph); 7,25(d, J=8Hz, 2H, Ph); 7,38(s, 2H, Ph); 7,56(s, 1H, Ph). |

| *Composés* N° | $R_1$,$R_2$,$R_3$ | $R_4$ | $R_6$ | $^1H$ RMN ($CDCl_3$, δ ppm) |
|---|---|---|---|---|
| *VIII.35* | 2-Cl<br>4-$OCH_3$<br>H | -$CH_3$ | 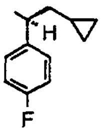 | 0,06-0,15(m, 2H, c-Pr); 0,40-0,50(m, 2H, c-Pr); 0,60-0,74(m, 1H, c-Pr); 1,65-1,90(m, 2H, $CH_2$-c-Pr); 2,21(s, 3H, $CH_3$); 3,87(s, 3H, $OCH_3$); 4,71(d, J=8,2Hz, 1H, NH); 4,78-4,85(m, 1H, CH); 6,89(dd $J_1$=2,7Hz, $J_2$=8,6Hz, 1H, Ph); 6,98-7,06(m, 3H, Ph); 7,28-7,40(m, 3H, Ph). |

**•1-[2-Chloro-4-(méthylsulfanyl)phényl]-*N*-[2-cyclopropyl-1-(3-fluoro-4-méthylphényl)éthyl]-5-méthyl-1*H*-1,2,4-triazol-3-amine *Composé VIII.36***

Une solution de 600 mg (1,9 mmol) de *N*-acétyl-*N'*-[2-cyclopropyl-1-(3-fluoro-4-méthylphényl)éthyl]-*S*-méthyl-isothiourée dans 10 ml de diméthylformamide est additionnée de 470 mg (2,2 mmol) de chlorhydrate de 2,4-dichlorophénylhydrazine puis de 800 mg (2,5 mmol) de carbonate de césium et de tamis moléculaire 4Å. Le mélange réactionnel est agité pendant 4 heures à 140°C, puis est refroidi. Le tamis moléculaire est éliminé par filtration et le filtrat est dilué par 100 ml d'acétate d'éthyle. La phase organique est lavée par l'acide chlorhydrique 1N, par de l'eau, par une solution aqueuse saturée en hydrogénocarbonate de sodium, par de l'eau, puis de l'eau saturée en chlorure de sodium. Elle est séchée sur sulfate de sodium puis les solvants sont évaporés sous pression réduite. Le résidu brut est purifié par chromatographie sur une colonne de gel de silice (éluant cyclohexane/acétate d'éthyle 3/1 (v/v)). On obtient 385 mg de *N*-[2-cyclopropyl-1-(3-fluoro-4-méthylphényl)éthyl]-1-(2,4-dichlorophényl)-5-méthyl-1*H*-1,2,4-triazol-3-amine et 115 mg de *composé VIII.36*. Rendement 13 %.
$^1H$ RMN ($CDCl_3$, δ ppm) : 0,02-0,13(m, 2H, c-Pr); 0,40-0,48(m, 2H, c-Pr); 0,60-0,72(m, 1H, c-Pr); 1,60-1,88(m, 2H, $CH_2$-c-Pr); 2,20(s, 3H, $CH_3$); 2,24(d, J=1,8Hz, 3H, $CH_3$); 2,52(s, 3H, $SCH_3$); 4,75-4,88(m, 2H, CH et NH); 7,04-7,14 (m, 3H, Ph); 7,18(dd, $J_1$=2Hz, $J_2$=8,3Hz 1H, Ph); 7,25(d, J=8,3Hz, 1H, Ph); 7,33(d, J=2Hz, 1H, Ph).

**4) Préparation des aminotriazoles de formule (I) par la voie B2**

**EXEMPLE 11**

**•[1-(2-Chloro-4-méthoxy-5-méthylphényl)-*N*-[2-cyclopropyl-1-(4-fluorophényl)éthyl]-5-méthyl-*N*-propyl-1*H*-1,2,4-triazol-3-amine**

240 mg (6 mmol) d'hydrure de potassium (obtenu à partir d'une suspension huileuse par lavage au pentane puis séchage sous argon) sont mis en suspension dans 2 ml de benzène anhydre. Le mélange est agité à 5-10°C et additionné successivement de 780 mg (1,85 mmol) de *composé VIII.1* dissout dans 6 ml de benzène et de 750 mg (2 mmol) de 2,3,11,12-cyclohexano-1,4,7,10,13,16-hexaoxacyclooctadécane.
Après agitation pendant une heure trente minutes à température ambiante, 0,6 ml (6 mmol) de iodopropane sont ajoutés et le mélange est agité pendant trois heures à température ambiante. Le mélange réactionnel est refroidi au bain de glace et additionné de 1 ml d'éthanol, puis 1 ml d'eau, puis il est dilué par 100 ml d'acétate d'éthyle. La phase organique est lavée à l'eau, puis à l'eau saturée en chlorure de sodium, séchée sur sulfate de sodium puis les solvants sont évaporés sous pression réduite. Le résidu est purifié par chromatographie sur une colonne de gel de silice (éluant : cyclohexane/acétate d'éthyle 4/1 (v/v)). On obtient 590 mg de produit gommeux. Rendement 75 %.
$^1H$ RMN ($CDCl_3$, δ ppm) : 0,1-0,17(m, 2H, c-Pr); 0,39-0,45(m, 2H, c-Pr); 0,69-0,76(m, 4H, $CH_3$ et CH, c-Pr); 1,30-1,45 et 1,50-1,65(2m, 2H, $CH_2$); 1,88-1,98(m, 2H, $CH_2$) 2,21 (s, 3H, $CH_3$); 2,22(s, 3H, $CH_3$); 2,94-3,02 et 3,10-3,20(2m, 2H, $NCH_2$); 3,87(s, 3H, $CH_3$), 5,47(t, 1H, CH); 6,92(s, 1H, Ph); 6,94-7,02(m, 2H, Ph); 7,19(s, 1H, Ph); 7,38-7,44(m, 2H, Ph).
Ce produit est salifié en chlorhydrate ; F = 138°C(HCl)

**EXEMPLE 12**

**•[1-(2,4-dichlorophényl)-*N*-(2-méthoxy-5-méthylphényl)-5-méthyl-*N*-(2-propynyl)-1*H*-1,2,4-triazol-3-amine**

240 mg (6 mmol) d'hydrure de potassium (obtenu à partir d'une suspension huileuse par lavage au pentane

puis séchage sous argon) sont mis en suspension dans 2 ml de benzène anhydre. Le mélange est agité à 10°C et additionné de 730 mg (2 mmol) de *composé VIII.3* dissout dans 8 ml de benzène. On rajoute 75 mg (0,2 mmol) de 2,3,11,12-dicyclohexano-1,4,7,10,13,16-hexaoxacyclooctadécane et le mélange est agité pendant deux heures à température ambiante. 0,66 ml (6 mmol) d'une solution de bromure de propargyle à 80 % dans le toluène est ensuite ajoutée et le mélange est agité une heure à température ambiante. Le mélange réactionnel est refroidi au bain de glace puis additionné successivement de 1 ml d'éthanol et 1 ml d'eau puis dilué par 100 ml d'acétate d'éthyle. La phase organique est lavée à l'eau puis à l'eau saturée en chlorure de sodium, séchée sur sulfate de sodium puis les solvants sont évaporés sous pression réduite. L'extrait brut est purifié par chromatographie sur une colonne de gel de silice (éluant : cyclohexane/acétate d'éthyle 3/1 (v/v)). On obtient 600 mg de produit. Rendement 75 %.
$^1$H RMN ($CDCl_3$, $\delta$ ppm) : 2,15(t, J=2,2Hz, 1H, CH); 2,21 (s, 3H, $CH_3$); 2,28(s, 3H, $CH_3$); 3,78(s, 3H, $OCH_3$); 4,52(d, J=2,2Hz, 2H, $CH_2$); 6,84(d, J=8,3Hz, 1H, Ph); 7,01(dd, $J_1$=2Hz, $J_2$=8,3Hz, 1H, Ph);7,23(d, J=2Hz, 1H, Ph); 7,29-7,39 (m, 2H, Ph) 7,52(d, J=2Hz, 1H, Ph).
F=116°C

Par la même méthode (le benzène pouvant être remplacé par le tétrahydrofurane) sont synthétisés les EXEMPLES 13 à 53 du TABLEAU 6 suivant :

## TABLEAU 6 : Composés de formule I synthétisés par la voie B2

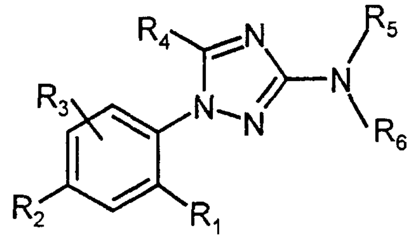

(I)

| Exemples | $R_1,R_2,R_3$ | $R_4$ | $R_5$ | $R_6$ | Sel ; F (°C) |
|---|---|---|---|---|---|
| **13** | 2-Cl<br>4-$OCH_3$<br>5-$CH_3$ | -$CH_3$ | -$CH_2$—C≡CH | 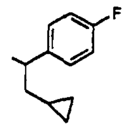 | HCl ; 72 |
| **14** | 2-Cl<br>4-$OCH_3$<br>5-$CH_3$ | 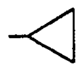 | -$(CH_2)_2CH_3$ | 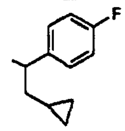 | HCl ;121 |
| **15** | 2-Cl<br>4-$OCH_3$<br>5-$CH_3$ | 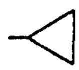 | -$CH_2$—C≡CH | 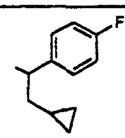 | HCl ; 139 |
| **16** | 2-Cl<br>4-Cl<br>H | -$CH_3$ | -$(CH_2)_2CH_3$ | 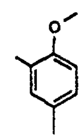 | HCl ; 149 |
| **17** | 2-Cl<br>4-$OCH_3$<br>5-$CH_3$ | 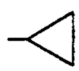 | -$(CH_2)_3CH_3$ | 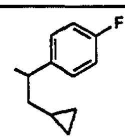 | HCl ; 91 |
| **18** | 2-Cl<br>4-$CF_3$<br>6-Cl | 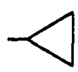 | -$(CH_2)_2CH_3$ | 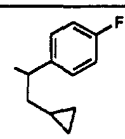 | HBr ; 90 |
| **19** | 2-Cl<br>4-Cl<br>H | 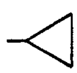 | -$(CH_2)_2CH_3$ | 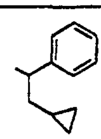 | HCl ; 132 |
| **20** | 2-Cl<br>4-$OCH_3$<br>5-$CH_3$ | 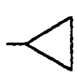 | -$(CH_2)_2CH_3$ | 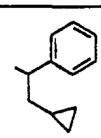 | 0,75 HCl ; 98 |

| Exemples | $R_1,R_2,R_3$ | $R_4$ | $R_5$ | $R_6$ | Sel ; F (°C)<br>$\alpha_D$ |
|---|---|---|---|---|---|
| **21** | 2-Cl<br>4-$OCH_3$<br>5-$CH_3$ | 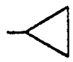 | $-(CH_2)_2CH_3$ | 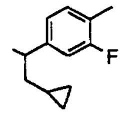 | 0,75 HCl ; 88 |
| **22** | 2-Cl<br>4-$OCH_3$<br>5-$CH_3$ | 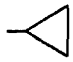 | $-(CH_2)_2CH_3$ | 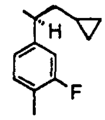 | 0,75 HCl ; 90<br>$[\alpha]^{20}_D = -105°$<br>(c = 0,52 MeOH) |
| **23** | 2-Cl<br>4-$OCH_3$<br>5-$CH_3$ | 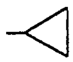 | $-CH_2-C\equiv CH$ | 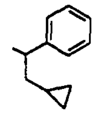 | HCl ;129 |
| **24** | 2-Cl<br>4-$OCH_3$<br>5-$CH_3$ | 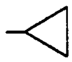 | $-(CH_2)_2CH_3$ | 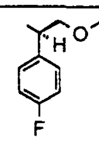 | HCl ; 132<br>$[\alpha]^{20}_D = -71°$<br>(c = 0,52 MeOH) |
| **25** | 2-Cl<br>4-$OCH_3$<br>5-$CH_3$ | 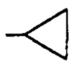 | $-CH_2-C\equiv CH$ | 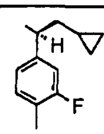 | HBr ; 99<br>$[\alpha]^{20}_D = -94°$<br>(c = 0,25 $CH_2Cl_2$) |
| **26** | 2-Cl<br>4-Cl<br>H | 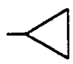 | $-(CH_2)_2CH_3$ | 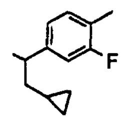 | HCl ; 139 |
| **27** | 2-Cl<br>4-Cl<br>H | 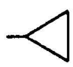 | $-(CH_2)_2CH_3$ | 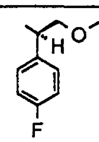 | HCl ; 119<br>$[\alpha]^{20}_D = -75°$<br>(c = 0,73 MeOH) |
| **28** | 2- $CH_3$<br>4- $CH_3$<br>H | 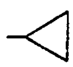 | $-(CH_2)_2CH_3$ | 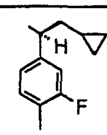 | HCl ; 77<br>$[\alpha]^{20}_D = -105°$<br>(c = 0,55 MeOH) |
| **29** | 2-Cl<br>4-Cl<br>H | $-CH_2CH_3$ | $-(CH_2)_2CH_3$ | 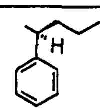 | HCl ; 130<br>$[\alpha]^{20}_D = -116°$<br>(c = 0,7 $CH_2Cl_2$) |
| **30** | 2-Cl<br>4-$OCH_3$<br>5-$CH_3$ | $-CH_3$ | $-(CH_2)_2CH_3$ | 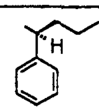 | HCl ; 87<br>$[\alpha]^{20}_D = -114°$<br>(c = 0,55 $CH_2Cl_2$) |
| **31** | 2-Cl<br>4-$OCH_3$<br>5-$CH_3$ | $-CH_3$ | $-(CH_2)_2CH_3$ | 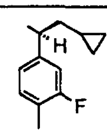 | HCl ; 114<br>$[\alpha]^{20}_D = -103°$<br>(c = 0,71 $CH_2Cl_2$) |
| **32** | 2-Cl<br>4-Cl<br>H | $-CH_3$ | $-(CH_2)_2CH_3$ | 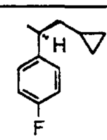 | HCl ; 82<br>$[\alpha]^{20}_D = -89°$<br>(c = 0,5 $CH_2Cl_2$) |

| Exemples | $R_1$,$R_2$,$R_3$ | $R_4$ | $R_5$ | $R_6$ | Sel ; F (°C) $\alpha_D$ |
|---|---|---|---|---|---|
| **33** | 2-Cl<br>4-$OCH_3$<br>5-$CH_3$ | $-CH_2CH_3$ | $-(CH_2)_2CH_3$ | 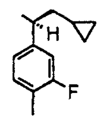 | HCl ; 93<br>$[\alpha]^{20}_D$ = -96°<br>(c = 0,5 $CH_2Cl_2$) |
| **34** | 2-Cl<br>4-Cl<br>H | $-CH_2CH_3$ | $-(CH_2)_2CH_3$ | 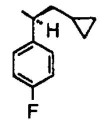 | HCl ; 139<br>$[\alpha]^{20}_D$ = -88°<br>(c = 0,5 $CH_2Cl_2$) |
| **35** | 2-Cl<br>4-$OCH_3$<br>5-$CH_3$ | $-CH_3$ | $-CH_2-C\equiv CH$ | 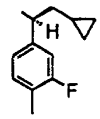 | HCl ; 89<br>$[\alpha]^{20}_D$ = -98°<br>(c = 0,47 $CH_2Cl_2$) |
| **36** | 2-Cl<br>4-Cl<br>H | 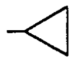 | $-(CH_2)_2CH_3$ | 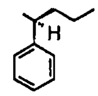 | HCl ; 98<br>$[\alpha]^{20}_D$ = -72°<br>(c = 0,52 $CH_2Cl_2$) |
| **37** | 2-Cl<br>4-$OCH_3$<br>5-$CH_3$ | $-CH_2CH_3$ | $-(CH_2)_2CH_3$ | 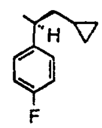 | HCl ; 79<br>$[\alpha]^{20}_D$ = -73°<br>(c = 0,9 $CH_2Cl_2$) |
| **38** | 2-Cl<br>4-Cl<br>H | $-CH_3$ | $-(CH_2)_2CH_3$ | 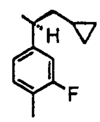 | HCl ; 93<br>$[\alpha]^{20}_D$ = -99°<br>(c = 0,52 $CH_2Cl_2$) |
| **39** | 2-Cl<br>4-$OCH_3$<br>5-$CH_3$ | $-CH_3$ | $-(CH_2)_2CH_3$ | 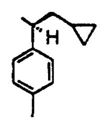 | HCl ; 139<br>$[\alpha]^{20}_D$ = -103°<br>(c = 0,56 $CH_2Cl_2$) |
| **40** | 2-Cl<br>4-Cl<br>H | $-CH_3$ | $-(CH_2)_2CH_3$ | 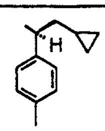 | HCl ; 94<br>$[\alpha]^{20}_D$ = -99°<br>(c = 0,5 $CH_2Cl_2$) |
| **41** | 2-Cl<br>4-$OCH_3$<br>5-$CH_3$ | 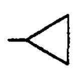 | $-(CH_2)_2CH_3$ | 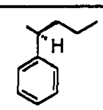 | HCl ; 65<br>$[\alpha]^{20}_D$ = -116°<br>(c = 0,7 $CH_2Cl_2$) |
| **42** | 2-Cl<br>4-$OCH_3$<br>5-$CH_3$ | $-CH_3$ | $-(CH_2)_2CH_3$ | 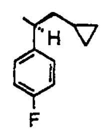 | HCl ; 120<br>$[\alpha]^{20}_D$ = -138°<br>(c = 0,33 $CH_2Cl_2$) |
| **43** | 2-Cl<br>4-$CF_3$<br>H | $-CH_3$ | $-(CH_2)_2CH_3$ | 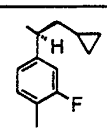 | HCl ; 138<br>$[\alpha]^{20}_D$ = -92°<br>(c = 0,62 $CH_2Cl_2$) |
| **44** | 2-Cl<br>4-$OCH_3$<br>5-$CH_3$ | $-CH_3$ | $-(CH_2)_2CH_3$ | 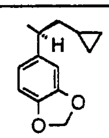 | HCl ; 98<br>$[\alpha]^{20}_D$ = -102°<br>(c = 0,64 $CH_2Cl_2$) |

| Exemples | $R_1$,$R_2$,$R_3$ | $R_4$ | $R_5$ | $R_6$ | Sel ; F (°C) $\alpha_D$ |
|---|---|---|---|---|---|
| **45** | 2-Cl<br>4-Cl<br>H | $-CH_3$ | $-(CH_2)_2CH_3$ | 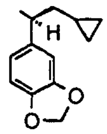 | HCl ; 76<br>$[\alpha]^{20}_D = -131°$<br>(c = 0,35 $CH_2Cl_2$) |
| **46** | 2-Cl<br>$4-OCH_3$<br>$5-CH_3$ | $-CH_3$ | $-(CH_2)_2CH_3$ | 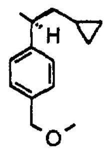 | HCl ; 143<br>$[\alpha]^{20}_D = -97°$<br>(c = 0,28 $CH_2Cl_2$) |
| **47** | 2-Cl<br>$4-OCH_3$<br>H | $-CH_3$ | $-(CH_2)_2CH_3$ | 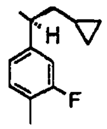 | HCl ; 118<br>$[\alpha]^{20}_D = -100°$<br>(c = 0,37 $CH_2Cl_2$) |
| **48** | 2-Cl<br>$4-OCH_3$<br>$5-CH_3$ | $-CH_3$ | $-(CH_2)_2CH_3$ | 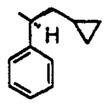 | HCl ; 89<br>$[\alpha]^{20}_D = -95°$<br>(c = 0,38 $CH_2Cl_2$) |
| **49** | 2-Cl<br>4-Cl<br>H | $-CH_3$ | $-(CH_2)_2CH_3$ | 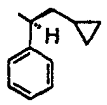 | HCl ; 113<br>$[\alpha]^{20}_D = -107°$<br>(c = 0,36 $CH_2Cl_2$) |
| **50** | 2-Cl<br>$4-OCH_3$<br>$5-CH_3$ | 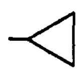 | $-(CH_2)_2CH_3$ | 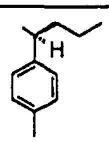 | HCl ; 78<br>$[\alpha]^{20}_D = -123°$<br>(c = 0,52 $CH_2Cl_2$) |
| **51** | 2-Cl<br>4-Cl<br>H | 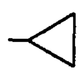 | $-(CH_2)_2CH_3$ | 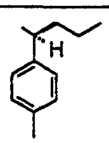 | HCl ; 85<br>$[\alpha]^{20}_D = -122°$<br>(c = 0,32 $CH_2Cl_2$) |
| **52** | 2-Cl<br>4-SMe<br>H | $-CH_3$ | $-(CH_2)_2CH_3$ | 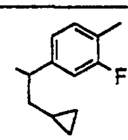 | HCl ; 73 |
| **53** | 2-Cl<br>$4-OCH_3$<br>H | $-CH_3$ | $-(CH_2)_2CH_3$ | 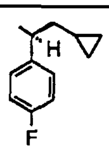 | HCl ; 83<br>$[\alpha]^{20}_D = -101°$<br>(c = 0,32 $CH_2Cl_2$) |

**Revendications**

**1.** Composés de formule:

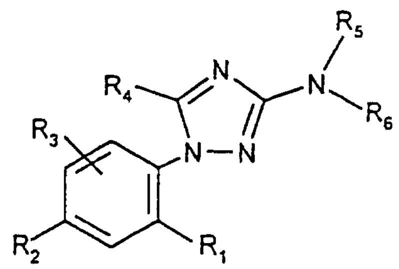

(I)

dans laquelle :

- $R_1$ et $R_2$ représentent, chacun indépendamment l'un de l'autre, un atome d'halogène ; un $(C_1-C_5)$alkyle ; un $(C_1-C_5)$alcoxy ; un groupe nitro, trifluorométhyle ou cyano ; un groupe amino $NR_aR_b$ dans lequel $R_a$ et $R_b$ représentent chacun indépendamment l'un de l'autre un hydrogène, un $(C_1-C_3)$alkyle , un $CO(C_1-C_3)$alkyle, ou bien dans lequel $R_a$ et $R_b$ constituent avec l'atome d'azote auquel ils sont liés un hétérocycle de 5 à 7 chaînons ; un groupe S-R dans lequel R représente un atome d'hydrogène ou un $(C_1-C_5)$alkyle, l'atome de soufre pouvant être monooxydé ou dioxydé ;
- $R_3$ représente l'hydrogène ou est tel que défini ci-dessus pour $R_1$ ;
- $R_4$ représente l'hydrogène; un halogène ; un $(C_1-C_5)$alkyle ; un $(C_3-C_5)$cycloalkyle ; un $(C_3-C_5)$cycloalkyl $(C_1-C_2)$alkyle ; un groupe $R_c$-X-$(C_1-C_2)$alkyle dans lequel $R_c$ représente l'hydrogène ou un $(C_1-C_3)$alkyle et X représente O, S, SO, $SO_2$ ;
- $R_5$ représente un $(C_1-C_5)$alkyle, un alcynyle de 3 à 5 atomes de carbone, un alcényle de 3 à 5 atomes de carbone ; un $(C_3-C_5)$cycloalkyl$(C_1-C_3)$alkyle ; un $(C_1-C_3)$alkyl-X$(C_0-C_3)$alkyle dans lequel X représente O, S, SO, $SO_2$ ;
- $R_6$ représente un groupe phényle substitué par un ou plusieurs radicaux Z dont l'un au moins est en position 2 et Z représente un halogène, un groupe nitro, trifluorométhyle ou cyano ; un $(C_1-C_5)$alkyle ; un $(C_1-C_5)$alkyl-X- ou $(C_1-C_3)$alkyl-X-$(C_1-C_2)$alkyle dans lesquels X représente O, S, SO ou $SO_2$ ; un hydroxy$(C_1-C_3)$alkyle ; un $COR_d$ ou $COOR_d$ où $R_d$ représente un $(C_1-C_3)$alkyle ou un $(C_3-C_5)$cycloalkyle ;
- ou bien $R_6$ représente $-CHR_7R_8$ dans lequel
- $R_7$ représente un $(C_3-C_5)$cycloalkyle ; $R_7$ représente un groupe phényle pouvant être substitué en position 3, 4 et 5 par un ou plusieurs radicaux Z', avec Z' représentant un halogène, un groupe nitro, trifluorométhyle ou cyano ; un $(C_1-C_5)$alkyle ; un $(C_1-C_5)$alkyl-X- ou $(C_1-C_3)$alkyl-X-$(C_1-C_2)$alkyle où X représente O, S, SO ou $SO_2$ ; un hydroxy$(C_1-C_3)$alkyle ; un $COR_d$ ou $COOR_d$ dans lesquels $R_d$ est tel que défini ci-dessus ; un méthylènedioxy, un éthylènedioxy ; ou bien $R_7$ représente un groupe pyridyle éventuellement substitué par un groupe amino $NR_aR_b$ tel que défini précédemment ou par un radical Z' tel que défini ci-dessus ;
- $R_8$ représente un $(C_1-C_6)$alkyle ; $(C_3-C_5)$cycloalkyle ; un $(C_3-C_5)$cycloalkyl$(C_1-C_3)$alkyle ; $(C_1-C_3)$alkyl-X-$(C_1-C_3)$alkyle où X représente O, S, SO ou $SO_2$ ; un $(C_3-C_5)$cycloalkyl$(C_1-C_2)$alkyl-X-$(C_1-C_3)$alkyle où X représente O, S, SO, $SO_2$ ;

ainsi que leurs sels d'addition pharmaceutiquement acceptables, leurs hydrates et/ou solvats.

**2.** Composés selon la revendication 1 dans lesquels :

- $R_1$ et $R_2$ représentent, chacun indépendamment l'un de l'autre, un atome d'halogène ; un $(C_1-C_5)$alkyle ; un $(C_1-C_5)$alcoxy ; un trifluorométhyle ou un groupe S-R dans lequel R représente un $(C_1-C_5)$alkyle ;
- $R_3$ représente l'hydrogène ou un $(C_1-C_5)$alkyle ;
- $R_4$ représente un $(C_1-C_5)$alkyle ; un $(C_3-C_5)$cycloalkyl ou un groupe $R_a$-X-$(C_1-C_2)$alkyle dans lequel $R_a$ représente un $(C_1-C_3)$alkyle et X représente O ;
- $R_5$ représente un $(C_1-C_5)$alkyle ou un alcynyle de 3 à 5 atomes de carbone ;
- $R_6$ représente $-CHR_7R_8$ dans lequel
- $R_7$ représente un groupe phényle pouvant être substitué en position 3, 4 et 5 par un ou plusieurs radicaux Z', avec Z' représentant un halogène ; un $(C_1-C_5)$alkyle ; un $(C_1-C_5)$alkyl-X- ou $(C_1-C_3)$alkyl-X-$(C_1-C_2)$alkyle où X représente O ; ou un groupe méthylènedioxy ;
- $R_8$ représente un $(C_1-C_6)$alkyle ; un $(C_3-C_5)$cycloalkyl$(C_1-C_3)$alkyle ; $(C_1-C_3)$alkyl-X-$(C_1-C_3)$alkyle où X représente O ;

ainsi que leurs sels d'addition pharmaceutiquement acceptables, leurs hydrates et/ou solvats.

**3.** Composés selon l'une des revendications 1 ou 2 dans lesquels $R_5$ représente un groupe propyle ou propargyle.

**4.** Composés selon l'une quelconque des revendications 1 à 3 sous forme d'énantiomère.

**5.** Composés selon l'une quelconque des revendications 1 à 4 choisis parmi :

- Chlorhydrate de 5-cyclopropyl-*N*-[2-cyclopropyl-1-(4-fluorophényl)éthyl]-1-(2,4-dichlorophényl)-*N*-propyl-1*H*-1,2,4-triazol-3-amine
- Chlorhydrate de *N*-[2-cyclopropyl-1-(4-fluorophényl)éthyl]-1-(2,4-dichlorophényl)-5-(méthoxyméthyl)-*N*-propyl-1*H*-1,2,4-triazol-3-amine
- Chlorhydrate de *N*-[2-cyclopropyl-1-(4-fluorophényl)éthyl]-1-[2,6-dichloro-4-(trifluorométhyl)phényl]-5-méthyl-*N*-propyl-1*H*-1,2,4-triazol-3-amine
- Chlorhydrate de 1-(2-chloro-4-méthoxy-5-méthylphényl)-*N*-[2-cyclopropyl-1-(4-fluorophényl)éthyl]-5-méthyl-*N*-(2-propynyl)-1*H*-1,2,4-triazol-3-amine
- Chlorhydrate de 1-(2-chloro-4-méthoxy-5-méthylphényl)-5-cyclopropyl-*N*-[2-cyclopropyl-1-(4-fluorophényl) éthyl]-*N*-propyl-1*H*-1,2,4-triazol-3-amine
- Chlorhydrate de 1-(2-chloro-4-méthoxy-5-méthylphényl)-5-cyclopropyl-*N*-[2-cyclopropyl-1-(4-fluorophényl) éthyl]-*N*-(2-propynyl)-1*H*-1,2,4-triazol-3-amine
- Bromhydrate de 5-cyclopropyl-*N*-[2-cyclopropyl-1-(4-fluorophényl)éthyl]-1-[2,6-dichloro-4-(trifluorométhyl) phényl]-*N*-propyl-1*H*-1,2,4-triazol-3-amine
- Chlorhydrate de 5-cyclopropyl-*N*-(2-cyclopropyl-1-phényléthyl)-1-(2,4-dichlorophényl)-*N*-propyl-1*H*-1,2,4-triazol-3-amine
- Chlorhydrate de 1-(2-chloro-4-méthoxy-5-méthylphényl)-5-cyclopropyl-*N*-(2-cyclopropyl-1-phényléthyl)-*N*-propyl-1*H*-1,2,4-triazol-3-amine
- Chlorhydrate de 1-(2-chloro-4-méthoxy-5-méthylphényl)-5-cyclopropyl-*N*-[(1*S*)-2-cyclopropyl-1-(3-fluoro-4-méthylphényl)éthyl]-*N*-propyl-1*H*-1,2,4-triazol-3-amine
- Chlorhydrate de 1-(2-chloro-4-méthoxy-5-méthylphényl)-5-cyclopropyl-*N*-(2-cyclopropyl-1-phényléthyl)-*N*-(2-propynyl)-1*H*-1,2,4-triazol-3-amine
- Chlorhydrate de 1-(2-chloro-4-méthoxy-5-méthylphényl)-5-cyclopropyl-*N*-[(1*R*)-1-(4-fluorophényl)-2-méthoxyéthyl]-*N*-propyl-1*H*-1,2,4-triazol-3-amine
- Bromhydrate de 1-(2-chloro-4-méthoxy-5-méthylphényl)-5-cyclopropyl-*N*-[(1*S*)-2-cyclopropyl-1-(3-fluoro-4-méthylphényl)éthyl]-*N*-(2-propynyl)-1*H*-1,2,4-triazol-3-amine
- Chlorhydrate de 5-cyclopropyl-*N*-[2-cyclopropyl-1-(3-fluoro-4-méthylphényl)éthyl]-1-(2,4-dichlorophényl)-*N*-propyl-1*H*-1,2,4-triazol-3-amine
- Chlorhydrate de 5-cyclopropyl-1-(2,4-dichlorophényl)-*N*-[(1*R*)-1-(4-fluorophényl)-2-méthoxyéthyl]-*N*-propyl-1*H*-1,2,4-triazol-3-amine
- Chlorhydrate de 5-cyclopropyl-*N*-[(1*S*)-2-cyclopropyl-1-(3-fluoro-4-méthylphényl) éthyl]-1-(2,4-diméthylphényl)-*N*-propyl-1*H*-1,2,4-triazol-3-amine
- Chlorhydrate de 1-(2,4-dichlorophényl)-5-éthyl-*N*-[(1*S*)-1-phénylbutyl]-*N*-propyl-1*H*-1,2,4-triazol-3-amine
- Chlorhydrate de 1-(2-chloro-4-méthoxy-5-méthylphényl)-5-méthyl-*N*-[(1*S*)-1-phénylbutyl]-*N*-propyl-1*H*-1,2,4-triazol-3-amine
- Chlorhydrate de 1-(2-chloro-4-méthoxy-5-méthylphényl)-*N*-[(1*S*)-2-cyclopropyl-1-(3-fluoro-4-méthylphényl) éthyl]-5-méthyl-*N*-propyl-1*H*-1,2,4-triazol-3-amine
- Chlorhydrate de *N*-[(1*S*)-2-cyclopropyl-1-(4-fluorophényl)éthyl]-1-(2,4-dichlorophényl)-5-méthyl-*N*-propyl-1*H*-1,2,4-triazol-3-amine
- Chlorhydrate de 1-(2-chloro-4-méthoxy-5-méthylphényl)-*N*-[(1*S*)-2-cyclopropyl-1-(3-fluoro-4-méthylphényl) éthyl]-5-éthyl-*N*-propyl-1*H*-1,2,4-triazol-3-amine
- Chlorhydrate de *N*-[(1*S*)-2-cyclopropyl-1-(4-fluorophényl)éthyl]-1-(2,4-dichlorophényl)-5-éthyl-*N*-propyl-1*H*-1,2,4-triazol-3-amine
- Chlorhydrate de 1-(2-chloro-4-méthoxy-5-méthylphényl)-*N*-[(1*S*)-2-cyclopropyl-1-(3-fluoro-4-méthylphényl) éthyl]-5-méthyl-*N*-(2-propynyl)-1*H*-1,2,4-triazol-3-amine
- Chlorhydrate de 5-cyclopropyl-1-(2,4-dichlorophényl)-*N*-[(1*S*)-1-phénylbutyl]-*N*-propyl-1*H*-1,2,4-triazol-3-amine
- Chlorhydrate de 1-(2-chloro-4-méthoxy-5-méthylphényl)-*N*-[(1*S*)-2-cyclopropyl-1-(4-fluorophényl)éthyl]-5-éthyl-*N*-propyl-1*H*-1,2,4-triazol-3-amine
- Chlorhydrate de *N*-[(1*S*)-2-cyclopropyl-1-(3-fluoro-4-méthylphényl)éthyl]-1-(2,4-dichlorophényl)-5-méthyl-*N*-propyl-1*H*-1,2,4-triazol-3-amine

- Chlorhydrate de 1-(2-chloro-4-méthoxy-5-méthylphényl)-*N*-[(1*S*)-2-cyclopropyl-1-(4-méthylphényl)éthyl]-5-méthyl-*N*-propyl-1*H*-1,2,4-triazol-3-amine
- Chlorhydrate de *N*-[(1*S*)-2-cyclopropyl-1-(4-méthylphényl)éthyl]-1-(2,4-dichlorophényl)-5-méthyl-*N*-propyl-1*H*-1,2,4-triazol-3-amine
- Chlorhydrate de 1-(2-chloro-4-méthoxy-5-méthylphényl)-5-cyclopropyl-*N*-[(1*S*)-1-phénylbutyl]-*N*-propyl-1*H*-1,2,4-triazol-3-amine
- Chlorhydrate de 1-(2-chloro-4-méthoxy-5-méthylphényl)-*N*-[(1*S*)-2-cyclopropyl-1-(4-fluorophényl)éthyl]-5-méthyl-*N*-propyl-1*H*-1,2,4-triazol-3-amine
- Chlorhydrate de 1-[2-chloro-4-(trifluorométhyl)phényl]-*N*-[(1*S*)-2-cyclopropyl-1-(3-fluoro-4-méthylphényl) éthyl]-5-méthyl-*N*-propyl-1*H*-1,2,4-triazol-3-amine
- Chlorhydrate de *N*-[(1*S*)-1-(1,3-benzodioxol-5-yl)-2-cyclopropyléthyl]-1-(2-chloro-4-méthoxy-5-méthylphényl)-5-méthyl-*N*-propyl-1*H*-1,2,4-triazol-3-amine
- Chlorhydrate de *N*-[(1*S*)-1-(1,3-benzodioxol-5-yl)-2-cyclopropyléthyl]-1-(2,4-dichlorophényl)-5-méthyl-*N*-propyl-1*H*-1,2,4-triazol-3-amine
- Chlorhydrate de 1-(2-chloro-4-méthoxy-5-méthylphényl)-*N*-{(1*S*)-2-cyclopropyl-1-[(4-méthoxyméthyl)phényl] éthyl}-5-méthyl-*N*-propyl-1*H*-1,2,4-triazol-3-amine
- Chlorhydrate de 1-(2-chloro-4-méthoxyphényl)-*N*-[(1*S*)-2-cyclopropyl-1-(3-fluoro-4-méthylphényl)éthyl]-5-méthyl-*N*-propyl-1*H*-1,2,4-triazol-3-amine
- Chlorhydrate de 1-(2-chloro-4-méthoxy-5-méthylphényl)-*N*-[(1*S*)-2-cyclopropyl-1-phényléthyl]-5-méthyl-*N*-propyl-1*H*-1,2,4-triazol-3-amine
- Chlorhydrate de *N*-[(1*S*)-2-cyclopropyl-1-phényléthyl]-1-(2,4-dichlorophényl)-5-méthyl-*N*-propyl-1*H*-1,2,4-triazol-3-amine
- Chlorhydrate de 1-(2-chloro-4-méthoxy-5-méthylphényl)-5-cyclopropyl-*N*-[(1*S*)-1-(4-méthylphényl)butyl]-*N*-propyl-1*H*-1,2,4-triazol-3-amine
- Chlorhydrate de 5-cyclopropyl-1-(2,4-dichlorophényl)-*N*-[(1*S*)-1-(4-méthylphényl) butyl]-*N*-propyl-1*H*-1,2,4-triazol-3-amine
- Chlorhydrate de 1-[2-chloro-4-(méthylsulfanyl)phényl]-*N*-[2-cyclopropyl-1-(3-fluoro-4-méthylphényl)éthyl]-5-méthyl-*N*-propyl-1*H*-1,2,4-triazol-3-amine
- Chlorhydrate de 1-(2-chloro-4-méthoxyphényl)-*N*-[(1*S*)-2-cyclopropyl-1-(4-fluorophényl)éthyl]-5-méthyl-*N*-propyl-1*H*-1,2,4-triazol-3-amine

ainsi que les bases correspondantes, les autres sels d'addition pharmaceutiquement acceptables, leurs solvats et/ou hydrates.

**6.** Procédé pour la préparation des composés de formule (I) selon la revendication 1 **caractérisé en ce que** l'on fait réagir un composé de formule :

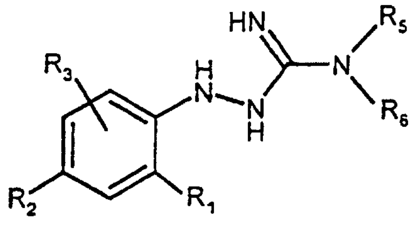

(II)

dans laquelle $R_1$, $R_2$, $R_3$, $R_5$ et $R_6$ sont tels que définis pour (I) avec un composé de formule $R_4COX$ (X représentant un halogène) pour fournir le composé (I).

**7.** Procédé pour la préparation des composés de formule (I) selon la revendication 1 **caractérisé en ce que** l'on soumet un composé de formule (VIII) :

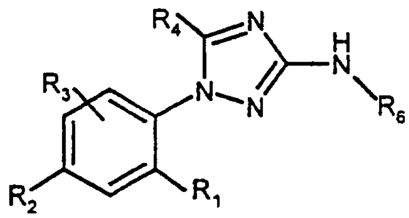

(VIII)

dans laquelle $R_1$, $R_2$, $R_3$, $R_4$ et $R_6$ sont tels que définis pour (I) à une réaction d'alkylation avec un composé de formule $R_5X$ (X représentant l'halogène) pour obtenir un composé (I).

8. Procédé pour la préparation des composés de formule (I) selon la revendication 1 et des composés de formule (VIII) **caractérisé en ce que** l'on fait réagir des composés de formule (VI) ou (IX) :

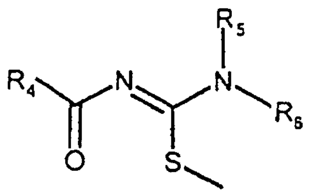

(VI)

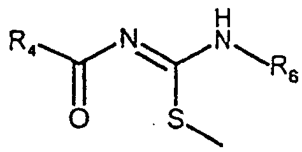

(IX)

dans lesquelles $R_4$, $R_5$ et $R_6$ sont tels que définis pour (I) avec un composé de formule (III) :

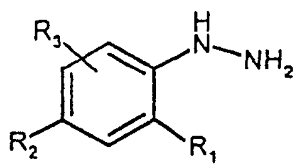

(III)

dans laquelle $R_1$, $R_2$ et $R_3$ sont tels que définis pour (I) pour fournir respectivement les composés de formule (I) ou de formule (VIII).

9. Composition pharmaceutique **caractérisée en ce qu'**elle contient en tant que principe actif un composé selon l'une quelconque des revendications 1 à 5 en association avec un ou plusieurs excipients appropriés.

10. Utilisation d'un composé selon l'une quelconque des revendications 1 à 5 pour la préparation de médicaments destinés à prévenir et/ou traiter les maladies CRF dépendantes.

11. Médicament **caractérisé en ce qu'**il contient un composé selon l'une quelconque des revendications 1 à 5.

## Patentansprüche

1. Verbindungen der Formel:

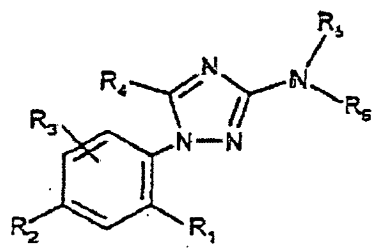

(I)

in der:

- $R_1$ und $R_2$ jeweils unabhängig voneinander ein Halogenatom; eine $(C_1-C_5)$-Alkylgruppe; eine $(C_1-C_5)$-Alkoxygruppe; eine Nitrogruppe, Trifluormethylgruppe oder Cyanogruppe; eine Aminogruppe $NR_aR_b$, in der $R_a$ und $R_b$ jeweils unabhängig voneinander ein Wasserstoffatom, eine $(C_1-C_3)$-Alkylgruppe, eine $CO-(C_1-C_3)$-Alkylgruppe darstellen oder worin $R_a$ und $R_b$ zusammen mit dem Stickstoffatom, an das sie gebunden sind, einen Heterocyclus mit 5 bis 7 Kettengliedern bilden; eine Gruppe S-R, in der R ein Wasserstoffatom oder eine $(C_1-C_5)$-Alkylgruppe darstellt, wobei das Schwefelatom monooxidiert oder dioxidiert sein kann; bedeuten;
- $R_3$ Wasserstoff bedeutet oder die oben für $R_1$ angegebenen Bedeutungen besitzt;
- $R_4$ Wasserstoff; ein Halogen; eine $(C_1-C_5)$-Alkylgruppe; eine $(C_3-C_5)$-Cycloalkylgruppe; eine $(C_3-C_5)$-Cycloalkyl-$(C_1-C_2)$-alkylgruppe; eine $R_c$-X-$(C_1-C_2)$-Alkylgruppe, in der $R_c$ Wasserstoff oder eine $(C_1-C_3)$-Alkylgruppe und X O, S, SO oder $SO_2$ darstellen; bedeutet;
- $R_5$ eine $(C_1-C_5)$-Alkylgruppe, eine Alkinylgruppe mit 3 bis 5 Kohlenstoffatomen, eine Alkenylgruppe mit 3 bis 5 Kohlenstoffatomen; eine $(C_3-C_5)$-Cycloalkyl-$(C_1-C_3)$-alkylgruppe; eine $(C_1-C_3)$-Alkyl-X-$(C_0-C_3)$-alkylgruppe, in der X O, S, SO oder $SO_2$ darstellt; bedeutet;
- $R_6$ eine Phenylgruppe, die durch eine oder mehrere Gruppen Z substituiert ist, von denen mindestens eine in der 2-Stellung steht, und Z ein Halogen, eine Nitrogruppe, eine Trifluormethylgruppe oder eine Cyanogruppe bedeutet; eine $(C_1-C_5)$-Alkylgruppe; eine $(C_1-C_5)$-Alkyl-X-Gruppe oder eine $(C_1-C_3)$-Alkyl-X-$(C_1-C_2)$-alkylgruppe, in denen X O, S, SO oder $SO_2$ darstellt; eine Hydroxy-$(C_1-C_3)$-alkylgruppe; eine Gruppe $COR_d$ oder $COOR_d$, worin $R_d$ eine $(C_1-C_3)$-Alkylgruppe oder eine $(C_3-C_5)$-Cycloalkylgruppe darstellt; bedeutet;
- oder $R_6$ $-CHR_7R_8$ bedeutet, worin
- $R_7$ eine $(C_3-C_5)$-Cycloalkylgruppe bedeutet; $R_7$ eine Phenylgruppe bedeutet, die in der 3-, 4- und 5-Stellung durch eine oder mehrere Gruppen Z' substituiert sein kann, worin Z' ein Halogen, eine Nitrogruppe, Trifluormethylgruppe oder Cyanogruppe darstellt; eine $(C_1-C_5)$-Alkylgruppe; eine $(C_1-C_5)$-Alkyl-X- oder $(C_1-C_3)$-Alkyl-X-$(C_1-C_2)$-alkylgruppe, worin X O, S, SO oder $SO_2$ darstellt; eine Hydroxy-$(C_1-C_3)$-alkylgruppe; eine Gruppe $COR_d$ oder $COOR_d$, worin $R_d$ die oben angegebenen Bedeutungen besitzt; eine Methylendioxy- oder eine Ethylendioxy-gruppe; oder $R_7$ eine Pyridylgruppe, die gegebenenfalls durch eine Aminogruppe $NR_aR_b$, wie sie oben definiert worden ist, oder durch eine Gruppe Z', wie sie oben definiert worden ist, substituiert ist; bedeutet;
- $R_8$ eine $(C_1-C_6)$-Alkylgruppe; eine $(C_3-C_5)$-Cycloalkylgruppe; eine $(C_3-C_5)$-Cycloalkyl-$(C_1-C_3)$-alkylgruppe; eine $(C_1-C_3)$-Alkyl-X-$(C_1-C_3)$-alkylgruppe, worin X O, S, SO oder $SO_2$ darstellt; eine $(C_3-C_5)$-Cycloalkyl-$(C_1-C_2)$-alkyl-X-$(C_1-C_3)$-alkylgruppe, worin X O, S, SO oder $SO_2$ darstellt; bedeutet;

sowie deren pharmazeutisch annehmbare Additionssalze, deren Hydrate und/oder Solvate.

**2.** Verbindungen nach Anspruch 1, worin:

- $R_1$ und $R_2$ jeweils unabhängig voneinander ein Halogenatom; eine $(C_1-C_5)$-Alkylgruppe; eine $(C_1-C_5)$-Alkoxygruppe; eine Trifluormethylgruppe oder eine Gruppe S-R, worin R eine $(C_1-C_5)$-Alkylgruppe darstellt; bedeuten;
- $R_3$ Wasserstoff oder eine $(C_1-C_5)$-Alkylgruppe bedeutet;
- $R_4$ eine $(C_1-C_5)$-Alkylgruppe; eine $(C_3-C_5)$-Cycloalkylgruppe oder eine $R_a$-X-$(C_1-C_2)$-Alkylgruppe, in der $R_a$ eine $(C_1-C_3)$-Alkylgruppe und X O darstellen; bedeutet;
- $R_5$ eine $(C_1-C_5)$-Alkylgruppe oder eine Alkinylgruppe mit 3 bis 5 Kohlenstoffatomen bedeutet;
- $R_6$ $-CHR_7R_8$ darstellt, worin
- $R_7$ eine Phenylgruppe, die in der 3-, 4- und 5-Stellung durch eine oder mehrere Gruppen Z' substituiert sein kann, wobei Z' ein Halogen bedeutet; eine $(C_1-C_5)$-Alkylgruppe; eine $(C_1-C_5)$-Alkyl-X- oder $(C_1-C_3)$-Alkyl-X-$(C_1-C_2)$-alkylgruppe, worin X O darstellt; oder eine Methylendioxygruppe; bedeutet;

- $R_8$ eine ($C_1$-$C_6$)-Alkylgruppe; eine ($C_3$-$C_5$)-Cycloalkyl-($C_1$-$C_3$)-alkylgruppe; oder eine ($C_1$-$C_3$)-Alkyl-X-($C_1$-$C_3$)-alkylgruppe, worin X O darstellt; bedeutet;

sowie deren pharmazeutisch annehmbare Additionssalze, deren Hydrate und/oder Solvate.

**3.** Verbindungen nach einem der Ansprüche 1 oder 2, worin $R_5$ eine Propyloder Propargylgruppe bedeutet.

**4.** Verbindungen nach einem der Ansprüche 1 bis 3 in Form des Enantiomeren.

**5.** Verbindungen nach einem der Ansprüche 1 bis 4 ausgewählt aus:

- 5-Cyclopropyl-*N*-[2-cyclopropyl-1-(4-fluorphenyl)-ethyl]-1-(2,4-dichlorphenyl)-*N*-propyl-1*H*-1,2,4-triazol-3-amin-Hydrochlorid,
- *N*-[2-Cyclopropyl-1-(4-fluorphenyl)-ethyl]-1-(2,4-dichlorphenyl)-5-(methoxymethyl)-*N*-propyl-1*H*-1,2,4-triazol-3-amin-Hydrochlorid,
- *N*-[2-Cyclopropyl-1-(4-fluorphenyl)-ethyl]-1-[2,6-dichlor-4-(trifluormethyl)-phenyl]-5-methyl-*N*-propyl-1*H*-1,2,4-triazol-3-amin-Hydrochlorid,
- 1-(2-Chlor-4-methoxy-5-methylphenyl)-*N*-[2-cyclopropyl-1-(4-fluorphenyl)-ethyl]-5-methyl-*N*-(2-propinyl)-1*H*-1,2,4-triazol-3-amin-Hydrochlorid.
- 1-(2-Chlor-4-methoxy-5-methylphenyl)-5-cyclopropyl-*N*-[2-cyclopropyl-1-(4-fluorphenyl)-ethyl]-*N*-propyl-1*H*-1,2,4-triazol-3-amin-Hydrochlorid,
- 1-(2-Chlor-4-methoxy-5-methylphenyl)-5-cyclopropyl-*N*-[2-cyclopropyl-1-(4-fluorphenyl)-ethyl]-*N*-(2-propinyl)-1*H*-1,2,4-triazol-3-amin-Hydrochlorid,
- 5-Cyclopropyl-*N*-[2-cyclopropyl-1-(4-fluorphenyl)-ethyl]-1-[2,6-dichlor-4-(trifluormethyl)-phenyl]-*N*-propyl-1H-1,2,4-triazol-3-amin-Hydrobromid.
- 5-Cyclopropyl-*N*-(2-cyclopropyl-1-phenylethyl)-1-(2,4-dichlorphenyl)-*N*-propyl-1*H*-1,2,4-triazol-3-amin-Hydrochlorid,
- 1-(2-Chlor-4-methoxy-5-methylphenyl)-5-cyclopropyl-*N*-(2-cyclopropyl-1-phenylethyl)-*N*-propyl-1*H*-1,2,4-triazol-3-amin-Hydrochlorid,
- 1-(2-Chlor-4-methoxy-5-methylphenyl)-5-cyclopropyl-*N*-[(1*S)*-2-cyclopropyl-1-(3-fluor-4methylphenyl)-ethyl] -*N*-propyl-1*H*-1,2,4-triazol-3-amin-Hydrochlorid,
- 1-(2-Chlor-4-methoxy-5-methylphenyl)-5-cyclopropyl-*N*-(2-cyclopropyl-1-phenylethyl)-*N*-(2-propinyl)-1*H*-1,2,4-triazol-3-amin-Hydrochlorid,
- 1-(2-Chlor-4-methoxy-5-methylphenyl)-5-cyclopropyl-*N*-[(1R)-1-(4-fluorphenyl)-2-methoxyethyl]-*N*-propyl-1*H*-1,2,4-triazol-3-amin-Hydrochlorid,
- 1-(2-Chlor-4-methoxy-5-methylphenyl)-5-cyclopropyl-*N*-[(1*S)*-2-cyclopropyl-1-(3-fluor-4-methylphenyl)-ethyl] -*N*-(2-propinyl)-1*H*-1,2,4-triazol-3-amin-Hydrobromid,
- 5-Cyclopropyl-*N*-[2-cyclopropyl-1-(3-fluor-4-methylphenyl)-ethyl]-1-(2,4-dichlorphenyl)-*N*-propyl-1*H*-1,2,4-triazol-3-amin-Hydrochlorid,
- 5-Cyclopropyl-1-(2,4-dichlorphenyl)-*N*-[(1R)-1-(4-fluorphenyl)-2-methoxyethyl]-*N*-propyl-1*H*-1,2,4-triazol-3-amin-Hydrochlorid,
- 5-Cyclopropyl-*N*-[(1*S*)-2-cyclopropyl-1-(3-fluor-4-methylphenyl)-ethyl]-1-(2,4-dimethylphenyl)-*N*-propyl-1*H*-1,2,4-triazol-3-amin-Hydrochlorid,
- 1-(2,4-Dichlorphenyl)-5-ethyl-*N*-[(1*S)*-1-phenylbutyl]-*N*-propyl-1*H*-1,2,4-triazol-3-amin-Hydrochlorid,
- 1-(2-Chlor-4-methoxy-5-methylphenyl)-5-methyl-*N*-[(1*S)*-1-phenylbutyl]-*N*-propyl-1*H*-1,2,4-triazol-3-amin-Hydrochlorid,
- 1-(2-Chlor-4-methoxy-5-methylphenyl)-N-[(1*S)*-2-cyclopropyl-1-(3-fluor-4-methylphenyl)-ethyl]-5-methyl-*N*-propyl-1*H*-1,2,4-triazol-3-amin-Hydrochlorid,
- *N*-[(1*S)*-2-Cyclopropyl-1-(4-fluorphenyl)-ethyl]-1-(2,4-dichlorphenyl)-5-methyl-*N*-propyl-1*H*-1,2,4-triazol-3-amin-Hydrochlorid,
- 1-(2-Chlor-4-methoxy-5-methylphenyl)-*N*-[(1*S*)-2-cyclopropyl-1-(3-fluor-4-methylphenyl)-ethyl]-5-ethyl-*N*-propyl-1*H*-1,2,4-triazol-3-amin-Hydrochlorid,
- *N*-[(1*S*)-2-Cyclopropyl-1-(4-fluorphenyl)-ethyl]-1-(2,4-dichlorphenyl)-5-ethyl-*N*-propyl-1*H*-1,2,4-triazol-3-amin-Hydrochlorid,
- 1-(2-Chlor-4-methoxy-5-methylphenyl)-*N*-[(1*S)*-2-cyclopropyl-1-(3-fluor-4-methylphenyl)-ethyl]-5-methyl-*N*-(2-propinyl)-1*H*-1,2,4-triazol-3-amin-Hydrochlorid,
- 5-Cyclopropyl-1-(2,4-dichlorphenyl)-*N*-[(1*S)*-1-phenylbutyl]-*N*-propyl-1*H*-1,2,4-triazol-3-amin-Hydrochlorid,
- 1-(2-Chlor-4-methoxy-5-methylphenyl)-*N*-[(1*S)*-2-cyclopropyl-1-(4-fluorphenyl)-ethyl]-5-ethyl-*N*-propyl-1*H*-

1,2,4-triazol-3-amin-Hydrochlorid,

- *N*-[(1*S*)-2-Cyclopropyl-1-(3-fluor-4-methylphenyl)-ethyl]-1-(2,4-dichlorphenyl)-5-methyl-*N*-propyl-1*H*-1,2,4-triazol-3-amin-Hydrochlorid,
- 1-(2-Chlor-4-methoxy-5-methylphenyl)-*N*-[(1*S*)-2-cyclopropyl-1-(4-methylphenyl)-ethyl]-5-methyl-*N*-propyl-1*H*-1,2,4-triazol-3-amin-Hydrochlorid,
- *N*-[(1*S*)-Cyclopropyl-1-(4-methylphenyl)-ethyl]-1-(2,4-dichlorphenyl)-5-methyl-*N*-propyl-1*H*-1,2,4-triazol-3-amin-Hydrochlorid,
- 1-(2-Chlor-4-methoxy-5-methylphenyl)-5-cyclopropyl-*N*-[(1*S*)-1-phenylbutyl)-*N*-propyl-1*H*-1,2,4-triazol-3-amin-Hydrochlorid,
- 1-(2-Chlor-4-methoxy-5-methylphenyl)-*N*-[(1*S*)-2-cyclopropyl-1-(4-fluorphenyl)-ethyl]-5-methyl-*N*-propyl-1*H*-1,2,4-triazol-3-amin-Hydrochlorid,
- 1-[2-Chlor-4-(trifluormethyl)-phenyl]-*N*-[(1*S*)-2-cyclopropyl-1-(3-fluor-4-methylphenyl)-ethyl]-5-methyl-*N*-propyl-1*H*-1,2,4-triazol-3-amin-Hydrochlorid,
- *N*-[(1*S*)-1-(1,3-Benzodioxol-5-yl)-2-cyclopropylethyl]-1-(2-chlor-4-methoxy-5-methylphenyl)-5-methyl-*N*-propyl-1*H*-1,2,4-triazol-3-amin-Hydrochlorid,
- *N*-[(1*S*)-1-(1,3-Benzodioxol-5-yl)-2-cyclopropylethyl]-1-(2,4-dichlorphenyl)-5-methyl-*N*-propyl-1*H*-1,2,4-triazol-3-amin-Hydrochlorid,
- 1-(2-Chlor-4-methoxy-5-methylphenyl)-*N*-{(1*S*)-2-cyclopropyl-1-[(4-methoxymethyl)-phenyl]-ethyl}-5-methyl-N-propyl-1*H*-1,2,4-triazol-3-amin-Hydrochlorid,
- 1-(2-Chlor-4-methoxyphenyl)-*N*-[(1*S*)-2-cyclopropyl-1-(3-fluor-4-methylphenyl)-ethyl]-5-methyl-*N*-propyl-1*H*-1,2,4-triazol-3-amin-Hydrochlorid,
- 1-(2-Chlor-4-methoxy-5-methylphenyl)-*N*-[(1*S*)-2-cyclopropyl-1-phenyl-ethyl]-5-methyl-*N*-propyl-1*H*-1,2,4-triazol-3-amin-Hydrochlorid,
- *N*-[(1*S*)-2-Cyclopropyl-1-phenylethyl]-1-(2,4-dichlorphenyl)-5-methyl-*N*-propyl-1*H*-1,2,4-triazol-3-amin-Hydrochlorid,
- 1-(2-Chlor-4-methoxy-5-methylphenyl)-5-cyclopropyl-*N*-[(1*S*)-1-(4-methylphenyl)-butyl]-*N*-propyl-1*H*-1,2,4-triazol-3-amin-Hydrochlorid.
- 5-Cyclopropyl-1-(2,4-dichlorphenyl)-*N*-[(1*S*)-1-(4-methylphenyl)-butyl]-*N*-propyl-1*H*-1,2,4-triazol-3-amin-Hydrochlorid.
- 1-[2-Chlor-4-(methylsulfanyl)-phenyl]-*N*-[2-cyclopropyl-1-(3-fluor-4-methylphenyl)-ethyl]-5-methyl-*N*-propyl-1*H*-1,2,4-triazol-3-amin-Hydrochlorid,
- 1-(2-Chlor-4-methoxyphenyl)-*N*-[(1*S*)-2-cyclopropyl-1-(4-fluorphenyl)-ethyl]-5-methyl-*N*-propyl-1H-1,2,4-triazol-3-amin-Hydrochlorid,

sowie deren entsprechende Basen, deren pharmazeutisch annehmbare Additionssalze, deren Solvate und/oder Hydrate.

**6.** Verfahren zur Herstellung der Verbindungen der Formel (I) nach Anspruch 1, **dadurch gekennzeichnet, daß** man eine Verbindung der Formel:

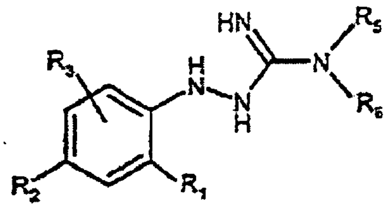

(II)

in der $R_1$, $R_2$, $R_3$, $R_5$ und $R_6$ die bezüglich (I) angegebenen Bedeutungen besitzen, mit einer Verbindung der Formel $R_4COX$ (worin X ein Halogen) darstellt, umsetzt zur Bildung der Verbindung (I).

**7.** Verfahren zur Herstellung der Verbindungen der Formel (I) nach Anspruch 1, **dadurch gekennzeichnet, daß** man eine Verbindung der Formel (VIII):

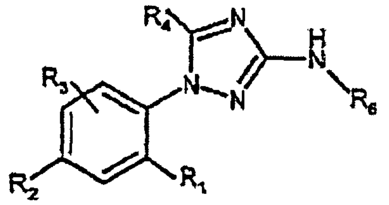

(VIII)

in der $R_1$, $R_2$, $R_3$, $R_4$ und $R_6$ die bezülgich (I) angegebenen Bedeutungen besitzen, einer Alkylierungsreaktion mit einer Verbindung der Formel $R_5X$ (worin X ein Halogen darstellt) unterwirft zur Bildung einer Verbindung (I).

**8.** Verfahren zur Herstellung der Verbindungen der Formel (I) nach Anspruch 1 und der Verbindungen der Formel (VIII), **dadurch gekennzeichnet, daß** man die Verbindungen der Formel (VI) oder (IX):

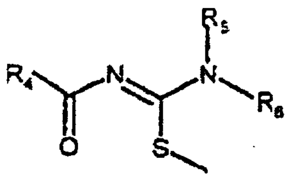

(VI)

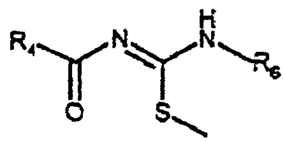

(IX)

in denen $R_4$, $R_5$ und $R_6$ die bezüglich (I) angegebenen Bedeutungen besitzen, mit einer Verbindung der Formel (III):

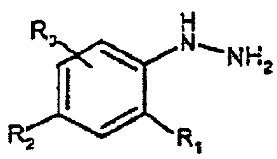

(III)

in der $R_1$, $R_2$ und $R_3$ die bezüglich (I) angegebenen Bedeutungen besitzen, umsetzt zur Bildung der Verbindungen der Formel (I) bzw. der Formel (VIII).

**9.** Pharmazeutische Zubereitung, **dadurch gekennzeichnet, daß** sie als Wirkstoff eine Verbindung nach einem der Ansprüche 1 bis 5 in Kombination mit einem oder mehreren geeigneten Trägermaterialien enthält.

**10.** Verwendung einer Verbindung nach einem der Ansprüche 1 bis 5 zur Herstellung von Arzneimitteln zur Vorbeugung und/oder Behandlung von CRF-abhängigen Erkrankungen.

**11.** Arzneimittel, **dadurch gekennzeichnet, daß** es eine Verbindung nach einem der Ansprüche 1 bis 5 enthält.

## Claims

**1.** Compounds of formula:

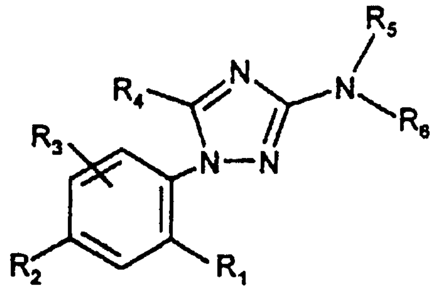

(I)

in which:

- $R_1$ and $R_2$ represent, each independently of the other, a halogen atom; a $(C_1\text{-}C_5)$alkyl; a $(C_1\text{-}C_5)$alkoxy; a nitro, trifluoromethyl or cyano group; an $NR_aR_b$ amino group in which $R_a$ and $R_b$ represent, each independently of the other, a hydrogen, a $(C_1\text{-}C_3)$alkyl or a $CO(C_1\text{-}C_3)$alkyl or else in which $R_a$ and $R_b$ constitute, with the nitrogen atom to which they are bonded, a 5- to 7-membered heterocycle; or an S-R group in which R represents a hydrogen atom or a $(C_1\text{-}C_5)$alkyl, it being possible for the sulphur atom to be monooxidized or dioxidized;
- $R_3$ represents hydrogen or is as defined above for $R_1$;
- $R_4$ represents hydrogen; a halogen; a $(C_1\text{-}C_5)$alkyl; a $(C_3\text{-}C_5)$cycloalkyl; a $(C_3\text{-}C_5)$ cycloalkyl$(C_1\text{-}C_2)$alkyl; or an $R_c\text{-}X\text{-}(C_1\text{-}C_2)$alkyl group in which $R_c$ represents hydrogen or a $(C_1\text{-}C_3)$alkyl and X represents O, S, SO or $SO_2$;
- $R_5$ represents a $(C_1\text{-}C_5)$alkyl, an alkynyl with 3 to 5 carbon atoms or an alkenyl with 3 to 5 carbon atoms; a $(C_3\text{-}C_5)$cycloalkyl$(C_1\text{-}C_3)$alkyl; or a $(C_1\text{-}C_3)$alkyl-X-$(C_0\text{-}C_3)$alkyl in which X represents O, S, SO or $SO_2$;
- $R_6$ represents a phenyl group substituted by one or more Z radicals, at least one of which is in the 2-position, and Z represents a halogen; a nitro, trifluoromethyl or cyano group; a $(C_1\text{-}C_5)$alkyl; a $(C_1\text{-}C_5)$alkyl-X- or $(C_1\text{-}C_3)$alkyl-X-$(C_1\text{-}C_2)$alkyl in which X represents O, S, SO or $SO_2$; a hydroxy$(C_1\text{-}C_3)$alkyl; or a $COR_d$ or $COOR_d$ where $R_d$ represents a $(C_1\text{-}C_3)$alkyl or a $(C_3\text{-}C_5)$cycloalkyl;
- or else $R_6$ represents $-CHR_7R_8$, in which
- $R_7$ represents a $(C_3\text{-}C_5)$cycloalkyl; $R_7$ represents a phenyl group which can be substituted in the 3-, 4- and 5-positions by one or more Z' radicals, with Z' representing a halogen; a nitro, trifluoromethyl or cyano group; a $(C_1\text{-}C_5)$alkyl; a $(C_1\text{-}C_5)$alkyl-X- or $(C_1\text{-}C_3)$alkyl-X-$(C_1\text{-}C_2)$alkyl where X represents O, S, SO or $SO_2$; a hydroxy $(C_1\text{-}C_3)$alkyl; a $COR_d$ or $COOR_d$ in which $R_d$ is as defined above; a methylenedioxy or an ethylenedioxy; or else $R_7$ represents a pyridyl group optionally substituted by an $NR_aR_b$ amino group as defined above or by a Z' radical as defined above;
- $R_8$ represents a $(C_1\text{-}C_6)$alkyl; a $(C_3\text{-}C_5)$cycloalkyl; a $(C_3\text{-}C_5)$cycloalkyl$(C_1\text{-}C_3)$alkyl; a $(C_1\text{-}C_3)$alkyl-X-$(C_1\text{-}C_3)$ alkyl where X represents O, S, SO or $SO_2$; or a $(C_3\text{-}C_5)$cycloalkyl$(C_1\text{-}C_2)$alkyl-X-$(C_1\text{-}C_3)$alkyl where X represents O, S, SO or $SO_2$;

and their pharmaceutically acceptable addition salts, their hydrates and/or their solvates.

**2.** Compounds according to Claim 1, in which:

- $R_1$ and $R_2$ represent, each independently of the other, a halogen atom; a $(C_1\text{-}C_5)$alkyl; a $(C_1\text{-}C_5)$alkoxy; a trifluoromethyl or an S-R group in which R represents a $(C_1\text{-}C_5)$alkyl;
- $R_3$ represents hydrogen or a $(C_1\text{-}C_5)$alkyl;
- $R_4$ represents a $(C_1\text{-}C_5)$alkyl; a $(C_3\text{-}C_5)$cycloalkyl or an $R_a\text{-}X\text{-}(C_1\text{-}C_2)$alkyl group in which $R_a$ represents a $(C_1\text{-}C_3)$alkyl and X represents O;
- $R_5$ represents a $(C_1\text{-}C_5)$alkyl or an alkynyl with 3 to 5 carbon atoms;
- $R_6$ represents $-CHR_7R_8$, in which
- $R_7$ represents a phenyl group which can be substituted in the 3-, 4- and 5-positions by one or more Z' radicals, with Z' representing a halogen; a $(C_1\text{-}C_5)$alkyl; a $(C_1\text{-}C_5)$alkyl-X- or $(C_1\text{-}C_3)$alkyl-X-$(C_1\text{-}C_2)$alkyl where X represents O; or a methylenedioxy group;
- $R_8$ represents a $(C_1\text{-}C_6)$alkyl; a $(C_3\text{-}C_5)$cycloalkyl$(C_1\text{-}C_3)$alkyl; or a $(C_1\text{-}C_3)$alkyl-X-$(C_1\text{-}C_3)$alkyl where X represents O;

and their pharmaceutically acceptable addition salts, their hydrates and/or their solvates.

3. Compounds according to either of Claims 1 and 2, in which $R_5$ represents a propyl or propargyl group.

4. Compounds according to any one of Claims 1 to 3, in the enantiomeric form.

5. Compounds according to any one of Claims 1 to 4, chosen from:

- 5-Cyclopropyl-*N*-[2-cyclopropyl-1-(4-fluorophenyl)-ethyl]-1-(2,4-dichlorophenyl)-*N*-propyl-1*H*-1,2,4-triazol-3-amine hydrochloride
- *N*-[2-Cyclopropyl-1-(4-fluorophenyl)ethyl]-1-(2,4-dichlorophenyl)-5-(methoxymethyl)-*N*-propyl-1*H*-1,2,4-triazol-3-amine hydrochloride
- *N*-[2-Cyclopropyl-1-(4-fluorophenyl)ethyl]-1-[2,6-dichloro-4-(trifluoromethyl)phenyl]-5-methyl-*N*-propyl-1*H*-1,2,4-triazol-3-amine hydrochloride
- 1-(2-Chloro-4-methoxy-5-methylphenyl)-*N*-[2-cyclopropyl-1-(4-fluorophenyl)ethyl]-5-methyl-*N*-(2-propynyl)-1*H*-1,2,4-triazol-3-amine hydrochloride
- 1-(2-Chloro-4-methoxy-5-methylphenyl)-5-cyclopropyl-*N*-[2-cyclopropyl-1-(4-fluorophenyl)ethyl]-*N*-propyl-1*H*-1,2,4-triazol-3-amine hydrochloride
- 1-(2-Chloro-4-methoxy-5-methylphenyl)-5-cyclopropyl-*N*-[2-cyclopropyl-1-(4-fluorophenyl)ethyl]-*N*-(2-propynyl)-1*H*-1,2,4-triazol-3-amine hydrochloride
- 5-Cyclopropyl-*N*-[2-cyclopropyl-1-(4-fluorophenyl)-ethyl]-1-[2,6-dichloro-4-(trifluoromethyl)phenyl]-*N*-propyl-1*H*-1,2,4-triazol-3-amine hydrobromide
- 5-Cyclopropyl-*N*-(2-cyclopropyl-1-phenylethyl)-1-(2,4-dichlorophenyl)-*N*-propyl-1*H*-1,2,4-triazol-3-amine hydrochloride
- 1-(2-Chloro-4-methoxy-5-methylphenyl)-5-cyclopropyl-*N*-(2-cyclopropyl-1-phenylethyl)-*N*-propyl-1*H*-1,2,4-triazol-3-amine hydrochloride
- 1-(2-Chloro-4-methoxy-5-methylphenyl)-5-cyclopropyl-*N*-[(1*S*)-2-cyclopropyl-1-(3-fluoro-4-methylphenyl)-ethyl]-*N*-propyl-1*H*-1,2,4-triazol-3-amine hydrochloride
- 1-(2-Chloro-4-methoxy-5-methylphenyl)-5-cyclopropyl-*N*-(2-cyclopropyl-1-phenylethyl)-*N*-(2-propynyl)-1*H*-1,2,4-triazol-3-amine hydrochloride
- 1-(2-Chloro-4-methoxy-5-methylphenyl)-5-cyclopropyl-*N*-[(1*R*)-1-(4-fluorophenyl)-2-methoxyethyl]-*N*-propyl-1*H*-1,2,4-triazol-3-amine hydrochloride
- 1-(2-Chloro-4-methoxy-5-methylphenyl)-5-cyclopropyl-*N*-[(1*S*)-2-cyclopropyl-1-(3-fluoro-4-methylphenyl)-ethyl]-*N*-(2-propynyl)-1*H*-1,2,4-triazol-3-amine hydrobromide
- 5-Cyclopropyl-*N*-[2-cyclopropyl-1-(3-fluoro-4-methylphenyl)ethyl]-1-(2,4-dichlorophenyl)-*N*-propyl-1*H*-1,2,4-triazol-3-amine hydrochloride
- 5-Cyclopropyl-1-(2,4-dichlorophenyl)-*N*-[(1*R*)-1-(4-fluorophenyl)-2-methoxyethyl]-*N*-propyl-1*H*-1,2,4-triazol-3-amine hydrochloride
- 5-Cyclopropyl-*N*-[(1*S*)-2-cyclopropyl-1-(3-fluoro-4-methylphenyl)ethyl]-1-(2,4-dimethylphenyl)-*N*-propyl-1*H*-1,2,4-triazol-3-amine hydrochloride
- 1-(2,4-Dichlorophenyl)-5-ethyl-*N*-[(1*S*)-1-phenylbutyl]-*N*-propyl-1*H*-1,2,4-triazol-3-amine hydrochloride
- 1-(2-Chloro-4-methoxy-5-methylphenyl)-5-methyl-*N*-[(1*S*)-1-phenylbutyl]-*N*-propyl-1*H*-1,2,4-triazol-3-amine hydrochloride
- 1-(2-Chloro-4-methoxy-5-methylphenyl)-*N*-[(1*S*)-2-cyclopropyl-1-(3-fluoro-4-methylphenyl)ethyl]-5-methyl-*N*-propyl-1*H*-1,2,4-triazol-3-amine hydrochloride
- *N*-[(1*S*)-2-Cyclopropyl-1-(4-fluorophenyl)ethyl]-1-(2,4-dichlorophenyl)-5-methyl-*N*-propyl-1*H*-1,2,4-triazol-3-amine hydrochloride
- 1-(2-Chloro-4-methoxy-5-methylphenyl)-*N*-[(1*S*)-2-cyclopropyl-1-(3-fluoro-4-methylphenyl)ethyl]-5-ethyl-*N*-propyl-1*H*-1,2,4-triazol-3-amine hydrochloride
- *N*-[(1*S*)-2-Cyclopropyl-1-(4-fluorophenyl)ethyl]-1-(2,4-dichlorophenyl)-5-ethyl-*N*-propyl-1*H*-1,2,4-triazol-3-amine hydrochloride
- 1-(2-Chloro-4-methoxy-5-methylphenyl)-*N*-[(1*S*)-2-cyclopropyl-1-(3-fluoro-4-methylphenyl)ethyl]-5-methyl-*N*-(2-propynyl)-1*H*-1,2,4-triazol-3-amine hydrochloride
- 5-Cyclopropyl-1-(2,4-dichlorophenyl)-*N*-[(1*S*)-1-phenylbutyl]-*N*-propyl-1*H*-1,2,4-triazol-3-amine hydrochloride
- 1-(2-Chloro-4-methoxy-5-methylphenyl)-*N*-[(1*S*)-2-cyclopropyl-1-(4-fluorophenyl)ethyl]-5-ethyl-*N*-propyl-1*H*-1,2,4-triazol-3-amine hydrochloride
- *N*-[(1*S*)-2-Cyclopropyl-2-(3-fluoro-4-methylphenyl)-ethyl]-1-(2,4-dichlorophenyl)-5-methyl-*N*-propyl-1*H*-

1,2,4-triazol-3-amine hydrochloride

- 1-(2-Chloro-4-methoxy-5-methylphenyl)-*N*-[(1*S*)-2-cyclopropyl-1-(4-methylphenyl)ethyl]-5-methyl-*N*-propyl-1*H*-1,2,4-triazol-3-amine hydrochloride
- *N*-[(1*S*)-2-Cyclopropyl-1-(4-methylphenyl)ethyl]-1-(2,4-dichlorophenyl)-5-methyl-*N*-propyl-1*H*-1,2,4-triazol-3-amine hydrochloride
- 1-(2-Chloro-4-methoxy-5-methylphenyl)-5-cyclopropyl-*N*-[(1*S*)-1-phenylbutyl]-*N*-propyl-1*H*-1,2,4-triazol-3-amine hydrochloride
- 1-(2-Chloro-4-methoxy-5-methylphenyl)-*N*-[(1*S*)-2-cyclopropyl-1-(4-fluorophenyl)ethyl]-5-methyl-*N*-propyl-1*H*-1,2,4-triazol-3-amine hydrochloride
- 1-[2-Chloro-4-(trifluoromethyl)phenyl]-*N*-[(1*S*)-2-cyclopropyl-1-(3-fluoro-4-methylphenyl)ethyl]-5-methyl-*N*-propyl-1*H*-1,2,4-triazol-3-amine hydrochloride
- *N*-[(1*S*)-1-(1,3-Benzodioxol-5-yl)-2-cyclopropylethyl]-1-(2-chloro-4-methoxy-5-methylphenyl)-5-methyl-*N*-propyl-1*H*-1,2,4-triazol-3-amine hydrochloride
- *N*-[(1*S*)-1-(1,3-Benzodioxol-5-yl)-2-cyclopropylethyl]-1-(2,4-dichlorophenyl)-5-methyl-*N*-propyl-1*H*-1,2,4-triazol-3-amine hydrochloride
- 1-(2-Chloro-4-methoxy-5-methylphenyl)-*N*-{(1*S*)-2-cyclopropyl-2-[(4-methoxymethyl)phenyl]ethyl}-5-methyl-*N*-propyl-1*H*-1,2,4-triazol-3-amine hydrochloride
- 1-(2-Chloro-4-methoxyphenyl)-*N*-[(1*S*)-2-cyclopropyl-1-(3-fluoro-4-methylphenyl)ethyl]-5-methyl-*N*-propyl-1*H*-1,2,4-triazol-3-amine hydrochloride
- 1-(2-Chloro-4-methoxy-5-methylphenyl)-*N*-[(1*S*)-2-cyclopropyl-1-phenylethyl]-5-methyl-*N*-propyl-1*H*-1,2,4-triazol-3-amine hydrochloride
- *N*-[(1*S*)-2-Cyclopropyl-1-phenylethyl]-1-(2,4-dichlorophenyl)-5-methyl-*N*-propyl-1*H*-1,2,4-triazol-3-amine hydrochloride
- 1-(2-Chloro-4-methoxy-5-methylphenyl)-5-cyclopropyl-*N*-[(1*S*)-1-(4-methylphenyl)butyl]-*N*-propyl-1*H*-1,2,4-triazol-3-amine hydrochloride
- 5-Cyclopropyl-1-(2,4-dichlorophenyl)-*N*-[(1*S*)-1-(4-methylphenyl)butyl]-*N*-propyl-1*H*-1,2,4-triazol-3-amine hydrochloride
- 1-[2-Chloro-4-(methylsulphanyl)phenyl]-*N*-[2-cyclopropyl-1-(3-fluoro-4-methylphenyl)ethyl]-5-methyl-*N*-propyl-1*H*-1,2,4-triazol-3-amine hydrochloride
- 1-(2-Chloro-4-methoxyphenyl)-*N*-[(1*S*)-2-cyclopropyl-1-(4-fluorophenyl)ethyl]-5-methyl-*N*-propyl-1*H*-1,2,4-triazol-3-amine hydrochloride

and the corresponding bases, the other pharmaceutically acceptable addition salts, their solvates and/or their hydrates.

**6.** Process for the preparation of the compounds of formula (I) according to Claim 1, **characterized in that** a compound of formula:

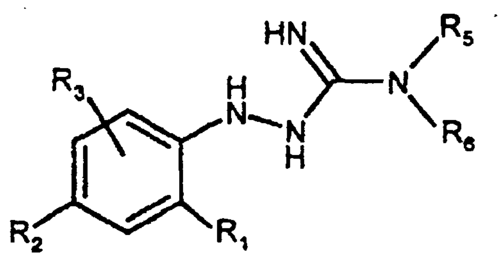

(II)

in which $R_1$, $R_2$, $R_3$, $R_5$ and $R_6$ are as defined for (I), is reacted with a compound of formula $R_4COX$ (X representing a halogen) to provide the compound (I).

**7.** Process for the preparation of the compounds of formula (I) according to Claim 1, **characterized in that** a compound of formula (VIII):

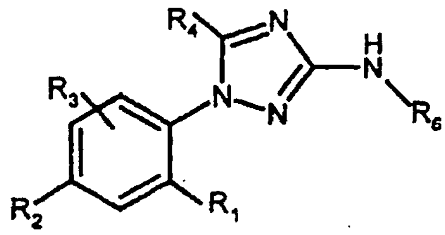

(VIII)

in which $R_1$, $R_2$, $R_3$, $R_4$ and $R_6$ are as defined for (I), is subjected to an alkylation reaction with a compound of formula $R_5X$ (X representing halogen) in order to obtain a compound (I).

**8.** Process for the preparation of the compounds of formula (I) according to Claim 1 and of the compounds of formula (VIII), **characterized in that** compounds of formula (VI) or (IX):

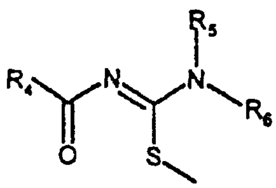

(VI)

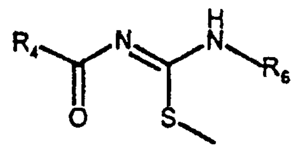

(IX)

in which $R_4$, $R_5$ and $R_6$ are as defined for (I), are reacted with a compound of formula (III):

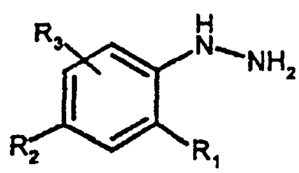

(III)

in which $R_1$, $R_2$ and $R_3$ are as defined for (I), to provide respectively the compounds of formula (I) or of formula (VIII).

**9.** Pharmaceutical composition, **characterized in that** it comprises, as active principle, a compound according to any one of Claims 1 to 5 in combination with one or more appropriate excipients.

**10.** Use of a compound according to any one of Claims 1 to 5 in the preparation of medicaments intended to prevent and/or treat CRF-dependent diseases.

**11.** Medicament, **characterized in that** it comprises a compound according to any one of Claims 1 to 5.